# EUROPEAN PATENT APPLICATION

(11) **EP 4 729 632 A1**
(43) Date of publication of application: **22.04.2026**
(21) Application number: 24383146.8
(22) Date of filing: 18.10.2024
(51) Int. Cl.: C12Q 1/6844

(54) **METHOD OF SYNTHESIZING DNA**

(71) Applicant: AskBio Inc., Research Triangle Park, NC 27709 (US)
(72) Inventor: ORANGE-BROMEHEAD PERRAMON, Katherine, E-20011 San Sebastián (ES); ECEIZA-TAPIA, Maite, E-20008 San Sebastián (ES); PEREZ-LOPEZ, Irene, E-31621 Sarriguren (ES); AGUNDEZ-CORTES, Leticia, E-20009 San Sebastián (ES); MARTIN PARRON, Sergio, E-20009 San Sebastián (ES); BASTIDA-CORCUERA, Félix D., E-20006 San Sebastián (ES)
(74) Representative: HGF

(57) **Abstract**

The technology described herein relates to methods for preparing DNA, e.g., enzymatically synthesizing DNA in a cell free method for from a template. The method comprises amplification of a DNA template with an initial amount of nucleotides and an initial amount of primers and adding additional nucleotides and primers continuously or at regular or irregular intervals after the initial amplification reaction has started.

## Description

### TECHNICAL FIELD

The technology described herein relates generally to methods for preparing DNA, e.g., enzymatically synthesizing a DNA such as in a cell-free process.

### BACKGROUND

When DNA is amplified mutations can occur during the amplification reaction due to various factors, such as the sequence being amplified (e.g., homopolymer regions, repeated regions, regions with secondary structures), which can introduce insertions, deletions, or point mutations. When amplification cycles are high, these errors can accumulate over repeated rounds of replication, potentially creating new variants in the amplified DNA sequence.

Thus, there is need in the art for methods of enzymatically synthesizing DNA, e.g., by amplification that reduces or prevents mutations in the synthesized DNA. The present disclosure addresses this need.

### SUMMARY

In one aspect, provided herein is a method for synthesizing a DNA, e.g., enzymatically synthesizing a DNA such as in a cell-free process. The method comprises amplification of a DNA template. In the amplification reaction, the DNA template is contacted with a DNA polymerase in the presence of an initial amount or portion of nucleotides and an initial amount or portion of one or more primers to make an initial amplification reaction, and at least one, e.g. at least two further additions (e.g., subsequent portions) of the primers and at least one, e.g. at least two further additions (e.g., subsequent portions) of nucleotides are made to the initial amplification reaction continuously or at regular or irregular intervals after the start of the amplification reaction, at least one, e.g. at least two further additions (e.g., subsequent portions) of the primers and at least one, e.g. at least two further additions (e.g., subsequent portions) of nucleotides are made to the initial amplification reaction at least after about 30 minutes after the start of the amplification reaction. Preferably, the amplification is isothermal amplification such as rolling circle amplification (RCA).

It is noted that the subsequent portions of the primers and nucleotides can be added at the same time or different times. Thus, in some embodiments, the further primers and nucleotides are added at the same time. In some other embodiments, the further primers and nucleotides are added at different times. Generally, a plurality of subsequent portions of the primers and the nucleotides are added to the reaction after the start of the amplification reaction. For example, at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or more subsequent portions of the primers can be added. Similarly, and independently, at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or more subsequent portions of the nucleotides can be added.

Without wishing to be bound by a theory, the method unexpectedly and surprisingly reduces/prevents mutations from occurring, e.g., insertion of a nucleotide in a homopolymer region of the DNA template during amplification. For example, less than about 5% (e.g., about 4.5%, about 4%, about 3.5%, about 3%, about 2.5%, about 2%, about 1.5%, about 1%, about 0.5%, about 0.4%, about 0.3%, about 0.2%, about 0.1% or less), e.g., substantially none) of the amplified product produced using the method described herein comprises a mutation, e.g., a nucleotide insertion in the sequence, such as a homopolymer region of the DNA template. In some embodiments, substantially none of the amplified product produced using the method described herein comprise a mutation e.g., a nucleotide insertion in the sequence, such as a homopolymer region of the DNA template. Accordingly, in some embodiments, the DNA template comprises a repeat region. Generally, the repeat region is at least 6 repeat units in length. For example, the repeat region is at least 7, or at least 8, or at least 9, or at least 11, or at least 11, or at least 12, or at least 13, or at least 14, or at least 15 repeat units in length. In some embodiments, the repeat region comprises nucleotides that are the same, i.e., the repeat unit is a nucleotide. In other words, the repeat region is a homopolymer, e.g., the repeat region comprises the nucleotide sequence (C)ₙ, (G)ₙ, (T)ₙ, or (A)ₙ, where n is an integer from 5 to 20. In some other embodiments, the repeat region comprises a dinucleotide repeat, i.e., the repeat unit is a dinucleotide. For example, the repeat region comprises a GC or CG dinucleotide repeat. In yet some other embodiments, the repeat region comprises a trinucleotide repeat, i.e., the repeat unit is a trinucleotide.

The DNA polymerase used can be a polymerase having strand displacement activity. In other words, the DNA polymerase can be a strand displacing polymerase. Some exemplary DNA polymerases for use in the method described herein include, but are not limited to, phi29 polymerase, Bst Polymerase, or a mutant or variant thereof, preferably the DNA polymerase is phi29.

In some embodiments, the method comprises amplification (e.g., RCA) of a DNA template, where the DNA template is contacted with a DNA polymerase (e.g., Phi29) in the presence of an initial amount of nucleotides and an initial amount of one or more primers, and at least one, e.g. at least two subsequent portions of both primers and nucleotides are added to the initial amplification reaction continuously starting at time zero or at regular or irregular intervals after, e.g., at least 45 minutes after the start of the amplification reaction.

The subsequent portions of the primers can be added to the amplification reaction continuously or at regular or irregular intervals. For example, the subsequent portions of the primers are added to the amplification reaction continuously or at intervals of at least about 30 minutes. In some embodiments, the subsequent portions of the primers are added to the amplification reaction continuously or at intervals of from about 45 minutes to about 75 minutes. For example, the subsequent portions of the primers are added to the amplification reaction at intervals of about 60 minutes.

The subsequent portions of the nucleotides can be added to the amplification reaction at regular or irregular intervals. For example, the subsequent portions of the nucleotides are added to the amplification reaction continuously or at intervals of at least about 30 minutes. In some embodiments, the subsequent portions of the nucleotides are added to the amplification reaction continuously or at intervals of from about 1 hours to about 3 hours. For example, the subsequent portions of the nucleotides can be added to the amplification reaction at intervals of from about 1 hours to about 2 hours.

In some embodiments, after the amplification reaction is initiated, subsequent portions of nucleotides are added at about every 30 mins, at about every 45 mins, at about every hour, at about every 1 hour, at about every 1.5 hours, at about every 2 hours, at about every 3 hours, at about every 4 hours, at about every 5 hours or more. Similarly, and independently of nucleotide addition, in some embodiments, after the amplification reaction is initiated, subsequent portions of primers are added at about every 30 mins, at about every 45 mins, at about every hour, at about every 1.5 hours, at about every 2 hours, at about every 3 hours, at about every 4 hours, at about every 5 hours or more. Primers and nucleotides e.g., mixture of nucleotides can be added at same intervals after the amplification reaction is initiated. Alternatively, primers and nucleotides e.g., mixture of nucleotides are added at different intervals after the amplification reaction is initiated.

In some embodiments, each subsequent portion of the primers independently comprises an amount that is equal or less than the amount of the primers in the initial amplification reaction. For example, each subsequent portion of the primers independently comprises equal or less than about 100% (weight or mol) (e.g., about 100%, about 95%, about 90%, about 80%, about 85%, about 72%, about 70%, about 65%, about 60%, about 55%, about 50%, about 45%, about 40%, about 35%, about 30%, about 25%, about 20%, about 15%, about 10%, about 5% or lower) of the total amount of the primers in the initial amplification reaction. In some embodiments, each subsequent portion of the primers independently comprises from about 5% to about 20%, or from about 7.5% to about 30%, or from about 10% to about 25%, or from about 10% to about 15% (weight or mol) of the total amount of the primers added to the initial amplification reaction. For example, each subsequent portion of the primers independently comprises from about 12.5% (weight or mol) of the total amount of the primers added to the initial amplification reaction.

In some embodiments, each subsequent portion of the primers is independently at a concentration of from about 1 µM to about 10 µM. For example, each subsequent portion of the primers is independently at a concentration of about 1.0 µM, about 1.5 µM, or about 2.0 µM, or about 2.5 µM, or about 3.0 µM, or about 3.5 µM, or about 4.0 µM, or about 5.0 µM, or about 5.5 µM, or about 6 µM, or about 6.5 µM, or about 7 µM, or about 7.5 µM, or about 8 µM, or about 8.5 µM, or about 9 µM, or about 9.5 µM, In some preferred embodiments, each subsequent portion of the primers is about 7 µM.

In some embodiments, the initial amplification reaction comprises less than about 50% (weight or mol) of the total amount of the primers to be added to the initial amplification reaction. For example, the initial amplification reaction comprises about 49%, about 48%, about 47%, about 46%, about 45%, about 44%, about 43%, about 42%, about 41%, about 40%, 39%, about 38%, about 37%, about 36%, about 35%, about 34%, about 33%, about 32%, about 31%, about 30%, 29%, about 28%, about 27%, about 26%, about 25%, about 24%, about 23%, about 22%, about 21%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2% or less) of the total amount of the primers to be added to the initial amplification reaction. In some preferred embodiments, the initial amplification reaction comprises, about 12.5% of the total amount of the primers to be added to the initial amplification reaction.

Generally, a total concentration of the primers added to the amplification reaction is from about 5 µM to about 70 µM. For example, the total concentration of the primers added to the amplification reaction is about 5 µM, about 6 µM, about 7 µM, about 8 µM, about 9 µM, about 10 µM, about 15 µM, about 20 µM, about 25 µM, about 30 µM, about 35 µM, about 40 µM, about 45 µM, about 50 µM, about 55 µM, about 60 µM, about 65 µM, or about 70 µM, In some preferred embodiments, the total concentration of the primers added to the amplification reaction is about 56.4 µM.

In some embodiments, each subsequent portion of the nucleotides independently comprises an amount that is equal or less than the amount of the nucleotides in the initial amplification reaction. For example, each subsequent portion of the nucleotides independently comprises less than about 75% (weight or mol) of the total amount of the nucleotides in the initial amplification reaction. In some embodiments, each subsequent portion of the nucleotides independently comprises less than about 70%, about 65%, about 60%, about 55%, about 50%, about 45%, about 40%, about 35%, about 30%, about 25%, or lower (weight or mol) of the total amount of the nucleotides in the initial amplification reaction.

In some embodiments, each subsequent portion of the nucleotides independently comprises from about 3% to about 30% (weight or mol) of the total amount of the nucleotides added to the initial amplification reaction. For example, each subsequent portion of the nucleotides independently comprises about 10% to about 25% or from about 15% to about 20% of the total amount of the nucleotides added to the initial amplification reaction. For example, each subsequent portion of the nucleotides independently comprises about 10%, about 11%, about 12%, about 13%, about 14%, about 15%, about 16%, about 17%, about 18%, about 19%, about 20%, about 21%, about 22%, about 23%, about 24%, or about 25% (mol% or weight%) of the total amount of the nucleotides added to the initial amplification reaction.

In some embodiments, each subsequent portion of the nucleotides is independently at a concentration of from about 0.001 mM to about 0.3 mM. For example, each subsequent portion of the nucleotides is independently at a concentration of from about 0.005 mM to about 0.125 mM, from about 0.01 mM to 0.1 mM, or from about 0.05 to about 0.09 mM. In some embodiments, each subsequent portion of the nucleotides is independently at a concentration of about 0.01 mM, about 0.02 mM, about 0.03 mM, about 0.04 mM, about 0.05 mM, about 0.06 mM, about 0.07 mM, about 0.08 mM, about 0.09 mM, about 0.1 mM, about 0.11 mM, about 0.12 mM, about 0.13 mM, about 0.14 mM, about 0.15 mM, about 0.16 mM, about 0.17 mM, about 0.18 mM, about 0.19 mM, about 0.20 mM, about 0.21 mM, about 0.22 mM, about 0.23 mM, about 0.24 mM, about 0.25 mM, about 0.26 mM, about 0.27 mM, about 0.28 mM, about 0.29 mM, or about 0.3 mM), In some preferred embodiments, each subsequent portion of the nucleotides is independently at a concentration of about 0.13 mM or about 0.25 mM.

Generally, the initial amplification reaction comprises less than about 30% (mol%) of the total amount of the nucleotides added to the initial amplification reaction. For example, the initial amplification reaction comprises about 29%, about 28%, about 27%, about 26%, about 25%, about 24%, about 23%, about 22%, about 21%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1% or less of the total amount of the nucleotides added to the initial amplification reaction.

In some embodiments, the total concentration of the nucleotides added to the amplification reaction is from about 0.1 mM to about 6 mM. For example, the total concentration of the nucleotides added to the amplification reaction is from about 0.25 mM to about 5.5 mM, from about 0.3 mM to about 5 mM or from about 0.25 mM to about 4.5 mM. In some embodiments, the total concentration of the nucleotides added to the amplification reaction is about 0.1 mM, about 0.15 mM, about 0.2 mM, about 0.25 mM, about 0.3 mM, about 0.35 mM, about 0.4 mM, about 0.45 mM, about 0.5 mM, about 0.55 mM, about 0.6 mM, about 0.65 mM, about 0.7 mM, about 0.75 mM, about 0.8 mM, about 0.85 mM, about 0.9 mM, about 0.95 mM, about 1 mM, about 1.25 mM, about 1.5 mM, about 1.75 mM, or about 1.25 mM, or about 3 mM, , or about 3.25 mM, or about 3.5 mM, or about 3.75 mM, or about 4.25 mM, or about 4.5 mM, or about 4.75 mM, or about 5 mM. In some embodiments, the total concentration of the nucleotides added to the amplification reaction is about 0.5 mM, or about 0.77 mM, or about 4 mM, preferably about 4 mM.

Generally, the nucleotides comprise a mix of dATP, dATP, dGTP and dCTP. A molar ratio of A to G, A to C, T to G, and/or or T to C in the nucleotides added to the reaction mixture is about 1:1. In some embodiments, a molar ratio of A to G, A to C, T to G, or T to C in the nucleotides added to the reaction mixture is higher than 1. For example, a molar ratio of A to G, A to C, T to G, or T to C in the nucleotides added to the reaction mixture is about 1.1, about 1.2, about 1.3, about 1.4, about 1.5, about 1.6, about 1.7, about 1.8, about 1.9, about 2, about 2.1, about 2.2, about 2.3, about 2.4, about 2.5, about 2.6, about 2.7, about 2.8, about 2.9, about 3 or more.

In some embodiments, the repeat region is at least 6 nucleotides in length. For example, the repeat region is at least 7, or at least 8, or at least 9, or at least 11, or at least 11, or at least 12, or at least 13, or at least 14, or at least 15 nucleotides in length.

The template DNA can be double-stranded DNA (dsDNA) or is single-stranded DNA (ssDNA). For example, the DNA template is an open circular dsDNA, a closed circular dsDNA, an open linear dsDNA or a closed linear dsDNA. In another non-limiting example, the DNA template can be a linear ssDNA or a closed circular ssDNA. Additionally, the DNA template can be from about 500 bases to about 100 kilobase. For example, the DNA template can be from about 1 kilobases to about 50 kilobases, or from about 1.5 kilobases to about 25 kilobases, or from about 2 kilobases to about 20 kilobases.

In some embodiments, the DNA template comprises at least one processing enzyme target sequence. The processing enzyme target sequence can comprise a recombinase targeting sequence or a protelomerase targeting sequence. When the DNA template comprises at least one processing enzyme target sequence, the method can further comprise supplying a processing enzyme to the reaction mixture. If needed, the processing enzyme can be supplied at regular or irregular intervals during the amplification reaction.

The amplification reaction can further comprise a DNA polymerase stabilizing agent such as glycerol, polyols, reducing agents etc. In some embodiments, the DNA polymerase stabilizing agent is a reducing agent. Exemplary reducing agents include, but are not limited to, dithiothreitol (DTT), 1,2-propanediol, ethylene glycol, ethanol, isopropanol, glycerol, Trehalose, and acetyl cysteine. Preferably the reducing agent is DTT. Thus, in some embodiments, the amplification reaction further comprises DTT. For example, the amplification reaction comprises DTT at a concentration of from about 1 mM to about 3 mM. In some embodiments, the amplification reaction comprises DTT at a concentration of from about 1.5 mM to 2.5 mM or from about 1.75 mM to about 2.25 mM, For example, the amplification reaction comprises DTT at a concentration of about 1.5 mM, about 1.55 mM, about 1.6 mM, about 1.65 mM, about 1.7 mM, about 1.75 mM, about 1.8 mM, about 1.85 mM, about 1.9 mM, about 1.95 mM, about 2 mM, about 2.05 mM, about 2.1 mM, about 2.15 mM, about 2.2 mM, about 2.25 mM, about 2.3 mM, about 2.35 mM, about 2.4 mM, about 2.45 mM, or about 2.5 mM, preferably about 2 mMe.g., about 1.5 mM, about 1.55 mM, about 1.6 mM, about 1.65 mM, about 1.7 mM, about 1.75 mM, about 1.8 mM, about 1.85 mM, about 1.9 mM, about 1.95 mM, about 2 mM, about 2.05 mM, about 2.1 mM, about 2.15 mM, about 2.2 mM, about 2.25 mM, about 2.3 mM, about 2.35 mM, about 2.4 mM, about 2.45 mM, or about 2.5 mM, preferably about 2 mM, e.g., from about 1.5 mM to 2.5 mM or from about 1.75 mM to about 2.25 mM. For example, the amplification reaction comprises DTT at a concentration of about 1.5 mM, about 1.55 mM, about 1.6 mM, about 1.65 mM, about 1.7 mM, about 1.75 mM, about 1.8 mM, about 1.85 mM, about 1.9 mM, about 1.95 mM, about 2 mM, about 2.05 mM, about 2.1 mM, about 2.15 mM, about 2.2 mM, about 2.25 mM, about 2.3 mM, about 2.35 mM, about 2.4 mM, about 2.45 mM, or about 2.5 mM. In some preferred embodiments, the amplification reaction comprises DTT at a concentration of about 2 mM.

The DNA produced by the method described herein can be no end DNA (neDNA), closed-ended linear duplex DNA (ceDNA), close ended linear duplexed DNA (cIDNA), linear-covalently closed DNA, or hpDNA^{™} or osDNA^{™} (4BaseBio). In some embodiments, the DNA produced by the method described herein is neDNA.

### BRIEF DESCRIPTION OF THE DRAWINGS

The patent of application file contains at least one drawing executed in color. Copies of this patent or patent application with color drawings(s) will be provided by the Office upon request and payment of the necessary fees.

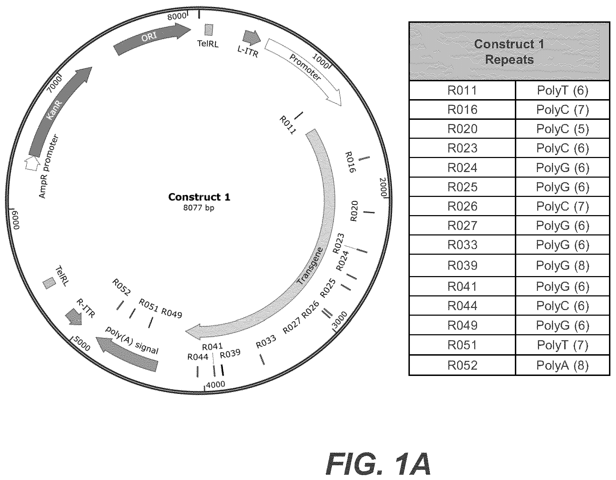

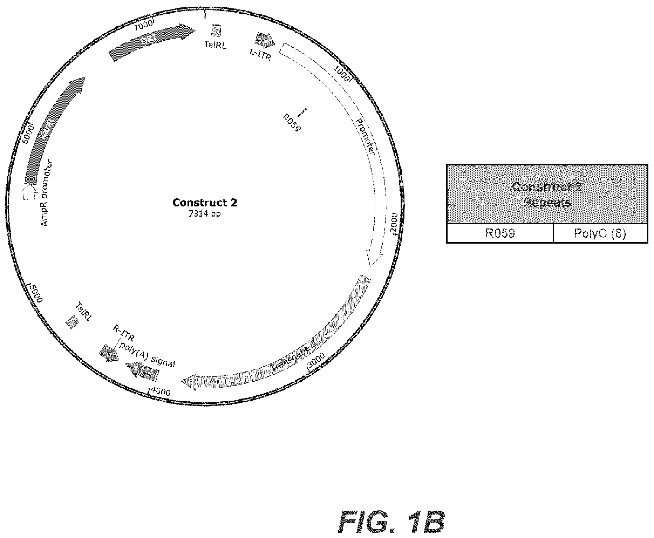

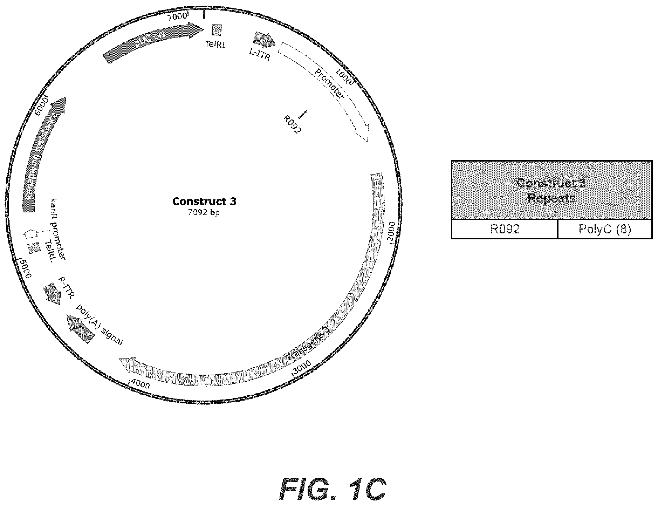

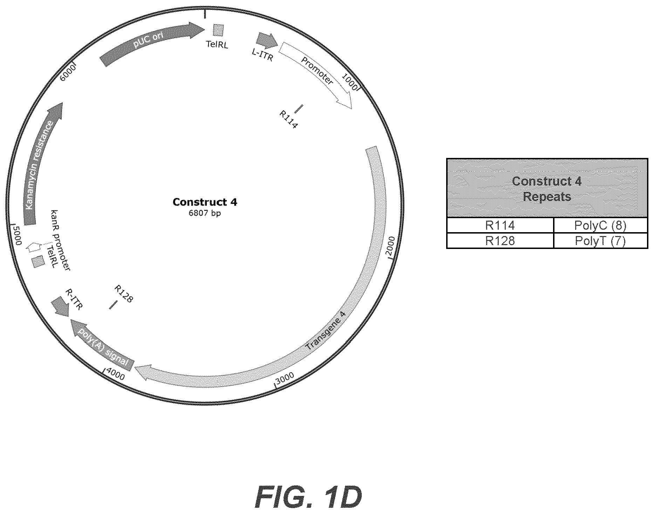

**FIGS. 1A-1D** are schematics of the neDNA^{™} precursor constructs map showing constructs 1-4 used in the experimental work featuring main genetic elements. The regions of repeated nucleotide sequences, homopolymers, were sequentially codified (R and number). The tables in the figures display only the repeats prone to mutations for each construct. TeIRL: TelN recognition site; L-ITR: left Inverted Terminal Repeat; Promoter; Transgene; R: repeat region of nucleotide sequence (C)n, (G)n, (T)-n, or (A)n, where n is an integer from 5 to 20; Poly(A) signal: polyadenylation signal, R-ITR: right Inverted Terminal Repeat; AmpR promoter: Ampicillin promoter; KanR: Kanamycin resistance gene; ORI: bacterial origin of replication.
**Fig.** 2 shows that both the 1mL scale and the 20mL scale are representative scale-down models of the large manufacturing scale in terms of insertion frequencies in homopolymer regions.
**FIGS. 3A and 3B** show lower insertions were obtained when low GC content is used, while varying AT concentration apparently does not impact insertions.
**FIG. 4** shows lower insertion frequencies were obtained when dNTPs concentration was lowered.
**FIG.5** shows that reducing dNTPs concentration or modifying dNTPs and primer feeding conditions reduced the frequency of insertions in homopolymer regions.
**FIG. 6** shows lower insertion frequencies were obtained when adding 2mM DTT.
**FIGS. 7-11** show insertion in homopolymer regions can be prevented by reducing dNTPs and primer concentration during amplification reaction (e.g., RCA), by adding both reagents in multiple feedings, and optionally including DTT in the reaction.

### DETAILED DESCRIPTION

It is to be understood that both the foregoing general description and the following detailed description are exemplary and explanatory only and are not restrictive of the invention, as claimed. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described. All documents, or portions of documents, cited in this application, including, but not limited to, patents, patent applications, articles, books, and treatises, are hereby expressly incorporated by reference in their entirety for any purpose.

In one aspect provided herein is a method for synthesizing a DNA from a DNA template. The method comprises starting the amplification of a DNA template with a polymerase in the presence of an initial amount of primers, nucleotides, and making further additions of the primers and nucleotides to the initial reaction after the amplification reaction has proceeded for a period of time. The further primers and nucleotides can be added at regular or irregular periods. The further primers and nucleotides can be supplied together or separately. It is preferred that the primers and the nucleotides are not mixed together prior to being added to the amplification reaction. The further primers and nucleotides can be added as each are required to the amplification reaction independently of each other.

Without wishing to be bound by a theory, the provision of a controlled supply of further primers and nucleotides to the amplification reaction is advantageous, since this rational control strategy can allow for reducing or preventing mutations that may be introduced during synthesis of the DNA as compared to an analogous reaction mixture where all the primers and nucleotides are supplied in the amplification reaction at the start.

The method described herein can be particularly feasible for "industrial-scale" (or "large-scale") synthesis of DNA, e.g., industrial-scale or large-scale enzymatic synthesis of DNA. As used herein, the terms "industrial-scale" or "large-scale" refer to a production scale that is larger than an experimental or laboratory use for research purposes only. For example, the industrial- or large-scale means methods wherein the volume of the amplification reaction is at least about 1 L. For example, the volume of the amplification reaction in an industrial- or large-scale can be at least about 1.5 L, at least about 2 L, at least about 2.5 L, at least about 3 L, at least about 3.5 L, at least about 4 L, at least about 4.5 L, at least about 5 L, at least about 6 L, at least about 7 L, at least about 8 L, at least about 9 L, at least about 10 L, at least about 11 L, at least about 12 L, at least about 13 L, at least about 14 L, at least about 15 L, at least about 16 L, at least about 17 L, at least about 18L, at least about 19 L, or at least about 20L. In some embodiments, the volume of the amplification reaction is about 2.5 L to about 7.5 L. For example, the volume of the amplification reaction is about from about 5 L to about 10 L. In some embodiments, the volume of the amplification reaction is about 5 L, about 5.5 L, about 6 L, about 6.5 L, about 7 L, about 7.5 L, about 8 L, about 8.5 L, about 9 L, about 9.5 L, or about 10 L.

### Template

Any polynucleotide can be used as the DNA template. The DNA template can be single-stranded or double-stranded. Further the DNA template can be linear or circular. In some embodiments, the DNA template is double-stranded, e.g., double-stranded DNA (dsDNA). For example, the DNA template is an open circular dsDNA, a closed circular dsDNA, a no end DNA (neDNA), an open linear dsDNA, a closed linear dsDNA, close ended linear duplexed DNA (clDNA), or linear-covalently closed DNA.

In some embodiments, the DNA template is single-stranded, e.g., single-stranded DNA (ssDNA). For example, the DNA template can be a linear ssDNA or a closed circular ssDNA.

In some embodiments, the DNA template is neDNA.

Closed linear dsDNA (e.g., closed circular dsDNA) or closed linear ssDNA typically comprise covalently closed ends, i.e. hairpin ends, where base pairing between complementary DNA strands is not present. The hairpin loops join the ends of complementary DNA strands. The loops may themselves contain complementary sequences, particularly if the loops comprise part of the protelomerase target sequence. The closed circular dsDNA may be a plasmid.

The DNA template can comprise any sequence, either naturally derived or artificial. For example, the DNA template can comprise an expression cassette comprising, consisting or consisting essentially of promoter, e.g., a eukaryotic promoter operably linked to a sequence enclosing a protein of interest, and optionally a transcription termination sequence, e.g., a eukaryotic transcription termination sequence. Optionally the expression cassette can be a minimal cassette, which lacks one or more bacterial or vector sequences, typically selected from the group consisting of: (i) bacterial origins of replication; (ii) bacterial selection markers (such as antibiotic resistance genes), and (iii) unmethylated CpG motifs. A "promoter" is a nucleotide sequence which initiates and regulates transcription of a polynucleotide. Promoters can include inducible promoters (where expression of a polynucleotide sequence operably linked to the promoter is induced by an analyte, co factor, regulatory protein, etc.), repressible promoters (where expression of a polynucleotide sequence operably linked to the promoter is repressed by an analyte, cofactor, regulatory protein, etc.), and constitutive promoters. It is intended that the term "promoter" or "control element" includes full-length promoter regions and functional (e.g., controls transcription or translation) segments of these regions. "Operably linked" refers to an arrangement of elements wherein the components so described are configured so as to perform their usual function. Thus, a given promoter operably linked to a nucleic acid sequence is capable of effecting the expression of that sequence when the proper enzymes are present. The promoter need not be contiguous with the sequence, so long as it functions to direct the expression thereof. Thus, for example, intervening untranslated yet transcribed sequences can be present between the promoter sequence and the nucleic acid sequence and the promoter sequence can still be considered "operably linked" to the coding sequence. Thus, the term "operably linked" is intended to encompass any spacing or orientation of the promoter element and the DNA sequence of interest which allows for initiation of transcription of the DNA sequence of interest upon recognition of the promoter element by a transcription complex.

In some embodiments, the DNA template comprises a repeat region. As used herein, the term "repeat region" means a contiguous sequence of nucleotides that are the same, a repeat of two alternating nucleotides (e.g., GC or CG), or a repeating pattern of three nucleotides. The repeat region can be at least 5, e.g., 6, 7, 8, 9, 10, 11, 12, 13, 14, 15 or more nucleotides in length.

In some embodiments, the repeat region comprises nucleotides that are the same. In other words, the repeat region is a homopolymeric region. For example, the repeat region comprises the nucleotide sequence (C)ₙ, (G)ₙ, (T)ₙ, or (A)ₙ, where n is an integer from 5 to 20. In some embodiments, the repeat region comprises the nucleotide sequence (C)ₙ, where n is 5, 6, 7, 8, 9, 10, 11, 12, 12, 14 or 15, e.g., n is 5, 6, 7, 8, 9 or 10. In some embodiments, the repeat region comprises the nucleotide sequence (G)ₙ, where n is 5, 6, 7, 8, 9, 10, 11, 12, 12, 14 or 15, e.g., n is 5, 6, 7, 8, 9 or 10. In some embodiments, the repeat region comprises the nucleotide sequence (T)ₙ, where n is 5, 6, 7, 8, 9, 10, 11, 12, 12, 14 or 15, e.g., n is 5, 6, 7, 8, 9 or 10. In some embodiments, the repeat region comprises the nucleotide sequence (A)ₙ, where n is 5, 6, 7, 8, 9, 10, 11, 12, 12, 14 or 15, e.g., n is 5, 6, 7, 8, 9 or 10.

In some embodiments, the repeat region comprises a dinucleotide repeat. For example, the repeat region comprises the nucleotide sequence (N¹N²)ₘ, where N¹ and N² are different nucleotides and m is 3, 4, 5, 6, 7, 8, 9, 10, 11, 12, 13, 14 or 15. In some embodiments, the repeat region comprises the nucleotide sequence (GC)ₘ or (CG)ₘ, where m is 3, 4, 5, 6, 7, 8, 9,10, 11, or 120, e.g. m is 4, 5, 6, 7 or 8.

In some embodiments, the repeat region comprises a trinucleotide repeat. For example, the repeat region comprises the nucleotide sequence (N¹N²N³)ₚ, where N¹ N², and N³ are different nucleotides and p is 2, 3, 4, 5, 6, 7, 8, 9, 10, 11, 12, 13, 14 or 15.

In some embodiments, the DNA template comprises a nucleotide sequence for expression. The nucleotide sequence for expression can be a nucleotide sequence for expression in a cell (e.g., in a transfected cell *in vitro* or *in vivo*), or it can be for expression in a cell free system (i.e., e.g., protein synthesis). The nucleotide sequence for expression can be for therapeutic purposes, e.g., gene therapy or vaccine. The nucleotide sequence for expression can be a gene, and said gene encodes a therapeutic protein a vaccine, and the like. The DNA sequence can comprise a sequence which is transcribed into an active RNA form, e.g., a small interfering RNA molecule (siRNA).

The DNA template can be of any suitable length. For example, the template can be up to 100 kilobases, or up to 90 kilobases, or up to 80 kilobases, or up to 75 kilobases, or up to 70 kilobases, or up to 60 kilobases, or up to 50 kilobases, or up to 40 kilobases, or up to 30 kilobases. In some embodiments, the DNA template is from about 500 bases to about 100 kilobases, e.g., from about 1 kilobases to about 50 kilobases, from about 1.5 kilobases to about 25 kilobases, or from about 2 kilobases to about 20 kilobases.

The DNA template can comprise at least one processing enzyme target sequence, such as one, two, three, four or more processing enzyme target sites. Without wishing to be bound by a theory, a processing enzyme target sequence can allow for the synthesized DNA, e.g., the enzymatically synthesized DNA to be optionally processed further following synthesis. A processing enzyme is an enzyme that recognizes its target site and processes the DNA. The processing enzyme target sequence can be a target sequence for a restriction enzyme. A restriction enzyme, i.e. a restriction endonuclease, binds to a target sequence and cleaves at a specific point. The processing enzyme target sequence may be a target for a recombinase. A recombinase directionally catalyzes a DNA exchange reactions between short (30-40 nucleotides) target site sequences that are specific to each recombinase. Examples of recombinases include the Cre recombinase (with loxP as a target sequence) and FLP recombinase (with short flippase recognition target (FRT) sites). The processing enzyme target sequence may be a target for a site-specific integrase, such as the phiC31 integrase. The processing enzyme target sequence may be a target for a protelomerase enzyme. A protelomerase target sequence is any DNA sequence whose presence in a DNA template allows for its conversion into a closed linear DNA by the enzymatic activity of protelomerase. In other words, the protelomerase target sequence is required for the cleavage and religation of double stranded DNA by protelomerase to form covalently closed linear DNA. Typically, a protelomerase target sequence comprises any palindromic sequence i.e. any double-stranded DNA sequence having two-fold rotational symmetry, also described herein as an inverted repeat. The length of the inverted repeat differs depending on the specific organism. The palindrome or inverted repeat may be perfect or imperfect. A protelomerase target sequence preferably comprises a double stranded palindromic (inverted repeat) sequence of at least 14 base pairs in length. Suitable protelomerase targeting sites are known in the art. See, for example, US Patent Publication No. US20120282283, content of which is incorporated herein by reference. The processing enzyme target sequence can be a target sequence for a RNA polymerase, such that the synthesized DNA becomes a template for polypeptide synthesis. In this instance, the processing enzyme targeting site is a promoter, preferably a eukaryotic promoter.

In some embodiments, the DNA template comprises at least one processing enzyme target sequence and the method comprises supplying a processing enzyme to the reaction mixture. It is noted that the processing enzyme can be supplied or added to the reaction at any time. For example, the processing enzyme can be supplied or added to the reaction once DNA synthesis is complete. The processing enzyme can be a RNA polymerase, a recombinase, a restriction endonuclease or a protelomerase. Preferably the processing enzyme is a protelomerase.

The processing enzyme can be supplied or added to the reaction at any appropriate time; this may be in response to a signal. One or more additions of processing enzyme can be made. For example, the processing enzyme can be supplied or added in aliquots, which are added discretely to the reaction. 1, 2, 3, 4, 5, 6, 7, 8, 9, 10 or more aliquots of processing enzyme can be added to the reaction. It can be advantageous to add the processing enzyme just before a portion of primers and/or nucleotides are added to the reaction. Alternatively, the processing enzyme can be added with or after a portion of primers and/or nucleotides are added to the reaction. The processing enzyme can be added in response to a signal that the amount of the primers and/or nucleotides have depleted to a desired point, and/or the DNA in the reaction mixture has reached a desired concentration.

In some embodiments, the DNA template comprises a protelomerase targeting sequence, and the method further comprises supplying or adding a protelomerase to the reaction. As used herein, the term "protelomerase targeting sequence" refers to a nucleotide sequence that can be recognized by a protelomerase. For example, protelomerase targeting sequence is a nucleotide sequence capable of being generated by a protelomerase that joins a first protelomerase recognition sequence (PRS) to a second PRS. Exemplary protelomerase recognition sequences include, but are not limited to *E*. *coli* phage N15, *Klebsiella* phage phi K02, *Yersinia* phage Py54, *Halomonas* phage phiHAP, *Vibrio* phage VP882, and the protelomerase recognition sequence derived from Borrelia burgdorferi IpB31.16. In some embodiments, the protelomerase targeting sequence is or is derived from *E*. *Coli* phage N15.

As used herein a protelomerase, is any polypeptide capable of cleaving and rejoining a template comprising a protelomerase target site in order to produce a covalently closed linear DNA molecule. Thus, the protelomerase has DNA cleavage and ligation functions. A typical substrate for protelomerase is circular double stranded DNA. If this DNA contains a protelomerase targeting sequence, the enzyme can cut the DNA at this site and ligate the ends to create a linear double stranded covalently closed DNA molecule. Exemplary protelomerases include, but are not limited to, those from bacteriophages such as N15 from *E*. *coli* (also known as TeIN), phiHAP-1 from *Halomonas quamarina,* PY54 from *Yersinia enterolytica* phiK02 from *Klebsiella oxytoca,* and VP882 from *Vibrio* sp., or variants of anythereof. Additional protelomerases are described in WO2012/017210, content if which is incorporated herein by reference. Enzymes having protelomerase-type activity have also been described as telomere resolvases (for example in *Borrelia burgdorferi*)*.* In one preferred embodiment, the protelomerase is TeIN.

The protelomerase can be supplied or added to the reaction at any time. For example, the protelomerase can be supplied or added to the reaction once enzymatic DNA synthesis is complete. It is noted that a single protelomerase or more than one protelomerase, such as two, three, four, five or more different protelomerases can be used. The protelomerase can be supplied or added to the reaction at any appropriate time; this may be in response to a signal. One or more additions of the protelomerase can be made. For example, the protelomerase can be supplied added in aliquots, which are added discretely to the reaction. 1, 2, 3, 4, 5, 6, 7, 8, 9, 10 or more aliquots of the protelomerase can be added to the reaction. It can be advantageous to add the protelomerase just before a portion of primers and/or nucleotides are added to the reaction. Alternatively, the protelomerase can be added with or after a portion of primers and/or nucleotides are added to the reaction. The protelomerase can be added in response to a signal that the amount of the primers and/or nucleotides have depleted to a desired point, and/or the DNA in the reaction mixture has reached a desired concentration. In some embodiments, the protelomerase is added once the DNA in the reaction mixture has reached a desired concentration

The DNA template can be prepared by any method known in the art. For example, the DNA template can be produced by PCR. Further, the whole or a selected portion of the DNA template can be amplified in the method described herein.

The amount of DNA template in the amplification reaction can be from about 0.5 µg/mL to about 10 µg/mL. For example, the amount of DNA template in the amplification reaction can be from about 0.75 µg/mL to about 9 µg/mL, from about 1 µg/mL to about 8 µg/mL, from about 1.25 µg/mL to about 6 µg/mL, from about 1.5 µg/mL to about 5.5 µg/mL, or from about 1.75 µg/mL to about 5 µg/mL. In some embodiments, the amount of the DNA template in the amplification reaction can be from about 2 µg/mL to about 4 µg/mL. For example, the amount of the DNA template in the amplification reaction can be about 2 µg/mL, about 2.1 µg/mL, about 2.2 µg/mL, about 2.3 µg/mL, about 2.4 µg/mL, about 2.5 µg/mL, about 2.6 µg/mL, about 2.7 µg/mL, about 2.8 µg/mL, about 2.9 µg/mL, about 3 µg/mL, about 3.1 µg/mL, about 3.2 µg/mL, about 3.3 µg/mL, about 3.4 µg/mL, about 3.5 µg/mL, about 3.6 µg/mL, about 3.7 µg/mL, about 3.8 µg/mL, about 3.9 µg/mL, or about 3 µg/mL.

### Primers

It is noted that one or more primers added to the amplification reaction can be the same they can be different, i.e., the primers added to the amplification reaction can have different sequences. Preferably, the primers are the same, i.e., have the same sequence.

A plurality of subsequent portions of the primers can be supplied to the amplification reaction. For example, 1-10, 10-20, 20-25, 25-30, or 35-40 portions of the primers can be added to the amplification reaction. In some embodiments, at least one, e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or more subsequent portions of the primers are added to the amplification reaction. For example, 4, 5, 6, 7, 8, 9, 10, 11, 12, 13, 14, or 15 subsequent portions of the primers are added to the amplification reaction are made after start of the amplification reaction.

It noted that each subsequent addition of the primers can be a portion of the total amount of primers required for or added to the process. The volume and/or concentration of primers in the portion or aliquot can vary depending on the requirements of the process. A portion or aliquot can comprise a small amount of primers, i.e., an amount sufficient to ensure that a particular concentration or amount of primers is present in the reaction mixture, e.g., a concentration or amount of primers sufficient for the amplification reaction. Generally, the primers are added to the amplification reaction in a total amount such that the total concentration of the primers in the amplification reaction is from about 5 µM to about 100 µM. For example, the total concentration of the primers added to the amplification reaction is from about 10 µM to about 95 µM, or from about 15 µM to about 90 µM, or from about 20 µM to about 85 µM, or from about 25 µM to about 80 µM, or from about 30 µM to about 75 µM, or from about 35 µM to about 75 µM, or from about 40 µM to about 70 µM, or from about 45 µM to about 65 µM, or from about 50 µM to about 60 µM. In some embodiment, a total concentration of the primers added to the amplification reaction is from about 50 µM to about 60 µM. For example, a total concentration of the primers required for or added to the amplification reaction is about 5 µM, or about 6 µM, or about 7 µM, or about 8 µM, or about 9 µM, or about 10 µM, or about 11 µM, or about 12 µM, or about 13 µM, or about 14 µM, or about 15 µM, or about 16 µM, or about 17 µM, or about 18 µM, or about 19 µM, or about 20 µM, or about 21 µM, or about 22 µM, or about 23 µM, or about 24 µM, or about 25 µM, or about 26 µM, or about 27 µM, or about 28 µM, or about 29 µM, or about 30 µM, or about 31 µM, or about 32 µM, or about 33 µM, or about 34 µM, or about 35 µM, or about 36 µM, or about 37 µM, or about 38 µM, or about 39 µM, or about 40 µM, or about 41 µM, or about 42 µM, or about 43 µM, or about 44 µM, or about 45 µM, or about 46 µM, or about 47 µM, or about 48 µM, or about 49 µM, or about 50 µM, or about 51 µM, or about 52 µM, or about 53 µM, or about 54 µM, or about 55 µM, or about 56 µM, or about 57 µM, or about 58 µM, or about 59 µM, or about 60 µM, or about 61 µM, or about 62 µM, or about 63 µM, or about 64 µM, or about 65 µM, or about 66 µM, or about 67 µM, or about 68 µM, or about 69 µM, or about 70 µM, or about 71 µM, or about 72 µM, or about 73 µM, or about 74 µM, or about 75 µM, or about 76 µM, or about 77 µM, or about 78 µM, or about 79 µM, or about 80 µM, or about 81 µM, or about 82 µM, or about 83 µM, or about 84 µM, or about 85 µM, or about 86 µM, or about 87 µM, or about 88 µM, or about 89 µM, or about 90 µM, or about 91 µM, or about 92 µM, or about 93 µM, or about 94 µM, or about 95 µM, or about 96 µM, or about 97 µM, or about 98 µM, or about 99 µM, about 100 µM, about 101 µM, about 102 µM, about 103 µM, about 104 µM, about 105 µM, about 106 µM, about 107 µM, about 108 µM, about 109 µM, about 110 µM, about 111 µM, about 112 µM, about 113 µM, about 114 µM, about 115 µM, about 116 µM, about 117 µM, about 118 µM, about 119 µM, or about 120 µM. In one preferred embodiment, the total concentration of the primers added to the amplification reaction is from about 45 µM to about 60 µM.

Generally, the initial amplification reaction comprises less than about 60% (mol% or weight%) of the total amount of primers required for or added to the amplification reaction. Stated in another way, the initial amplification reaction comprises less than about half of the total amount of primers required for or added to the amplification reaction. For example, the initial amplification reaction comprises about 49%, or about 48%, or about 47%, or about 46%, or about 45%, or about 44%, or about 43%, or about 42%, or about 41%, or about 40%, or about 39%, or about 38%, or about 37%, or about 36%, or about 35%, or about 34%, or about 33%, or about 32%, or about 31%, or about 30%, about 29%, or about 28%, or about 27%, or about 26%, or about 25%, or about 24%, or about 23%, or about 22%, or about 21%, or about 20%, or about 19%, or about 18%, or about 17%, or about 16%, or about 15%, or about 14%, or about 13%, or about 12%, or about 11%, or about 10%, or about 9%, or about 8%, or about 7%, or about 6%, or about 5% or less (mol% or weight%) of the total amount of primers required for or added to the amplification reaction. In some embodiments, the initial amplification reaction comprises about 12% (mol% or weight%) of the total amount of primers required for or added to the amplification reaction

In some embodiments, the initial amplification reaction comprises about 4.5 % or less (mol% or weight%) of the total amount of primers required for or added to the amplification reaction. For example, the initial amplification reaction comprises about 4%, or about 3.5%, or about 3%, or about 2.5%, or about 2%, or about 1.5%, or about 1%, or about 0.75%, or about 0.5%, or about 0.25%, or about 0.1% or less (mol% or weight%) of the total amount of primers required for or added to the amplification reaction. In some embodiments, the initial amplification reaction comprises about 3% (mol% or weight%) of the total amount of primers required for or added to the amplification reaction.

In some embodiments, the initial amplification reaction comprises about 0.75 % or less (mol% or weight%) of the total amount of primers required for or added to the amplification reaction. For example, the initial amplification reaction comprises about 0.5%, or about 0.45% or about 0.4%, or about 0.35%, or about 0.3%, or about 0.25%, or about 0.2%, or about 0.15%, or about 0.1%, or about 0.075%, or about 0.05%, or about 0.025%, or about 0.02%, or about 0.015%, or about 0.01%, or about 0.0075%, or about 0.005%, or about 0.0025%, or about 0.002%, or about 0.0015%, or about 0.001% or less (mol% or weight%) of the total amount of primers required for or added to the amplification reaction. In some embodiments, the initial amplification reaction comprises about 0.014% (mol% or weight%)

The amount of primers in the initial amplification reaction will not be sufficient to synthesize the desired quantity of DNA from the reaction mix. In some embodiments, the initial amplification reaction comprises primers at a concentration of about 0.5 µM to about 25 µM. For example, the initial amplification reaction comprises primers at a concentration of from about 0.75 µM to about 24 µM, or from about 1 µM to about 23 µM, or from about 1.5 µM to about 22 µM, or from about 2 µM to about 21 µM, or from about 2.5 µM to about 20 µM, or from about 3 µM to about 19 µM, or from about 3.5 µM) to about 18 µM, or from about 4 µM to about 17 µM, or from about 4.5 µM to about 16 µM, or from about 5 µM to about 15 µM. In some embodiment, the initial amplification reaction comprises primers at a concentration of from about 1 µM to about 15 µM, e.g., from about 5 µM to about 10 µM. For example, the initial amplification reaction comprises primers at a concentration of about 0.5 µM, or about 1 µM, or about 1.5 µM, or about 2 µM, or about 2.5 µM, or about 3 µM, or about 3.5 µM, or about 4 µM, or about 4.5 µM, or about 5 µM, or about 5.5 µM, or about 6 µM, or about 6.5 µM, or about 7 µM, or about 7.5 µM, or about 8 µM, or about 8.5 µM, or about 9 µM, or about 9.5 µM, or about, 10 µM, or about 10.5 µM, or about 11 µM, or about 11.5 µM, or about 12 µM, or about 12.5 µM, or about 13 µM, or about 13.5 µM, or about 14 µM, or about 14.5 µM, or about 15 µM, or about 15.5 µM, or about 16 µM, or about 16.5 µM, or about 17 µM, or about 17.5 µM, or about 18 µM, or about 18.5 µM, or about 19 µM, or about 19.5 µM, or about, 10 µM, or about 20.5 µM, or about 21 µM, or about 21.5 µM, or about 22 µM, or about 22.5 µM, or about 23 µM, or about 23.5 µM, or about 24 µM, or about 24.5 µM, or about 25 µM. In some embodiments, the initial amplification reaction comprises primers at a concentration of about 1 µM to about 15 µM. In some preferred embodiments, the initial amplification reaction comprises primers at a concentration of about 7 µM.

In some embodiments, the amount of each subsequent portion of the primers is independently less than the amount of the primers in the initial amplification reaction. For example, each subsequent portion of the primers independently comprises less than about 75% (mol% or weight%) of the total amount of the primers in the initial amplification reaction. Stated in another way, amount of each subsequent portion of the primers independently is less than about ¾ of the of the total amount of the primers in the initial amplification reaction. For example, the amount of each subsequent portion of the primers is independently about 70%, or about 65%, or about 60%, or about 65%, or about 60%, or about 45%, or about 40%, or about 35%, or about 30%, or about 25%, or lower (mol% or weight%) of the total amount of the primers in the initial amplification reaction.

In some embodiments, the amount of each subsequent portion of the primers is independently from about 6% to about 30% (mol% or weight%) of the total amount of the primers in the initial amplification reaction. For example, the amount of each subsequent portion of the primers is independently from about 10% to about 25% or from about 15% to about 20%, e.g., or about 15%, or about 16%, or about 17%, or about 18%, or about 19% or about 20% (mol% or weight%) of the total amount of the primers in the initial amplification reaction.

In some embodiments, the amount of each subsequent portion of the primers is independently about 5x, or about 10x, or about 20x, or about 30x, or about 40x, or about 50x, or about 60x, or about 70x, or about 80x, or about 90x, or about 100x, or about 200x, or about 300x, or about 400x, or about 500x, or about 600x, or about 700x, or about 800x, or about 900x, or about 1000x, or about 2000x, or about 3000x, or about 4000x, or about 5000x, or about 6000x, or about 7000x, or about 8000x, or about 9000x, or about 10000x or more than the total amount of the primers in the initial amplification reaction.

In some embodiments, the amount of each subsequent portion of the primers is about same as the total amount of the primers in the initial amplification reaction. in other words, each subsequent portion of the primers is similar in amount to the initial portion of primers added to start the initial amplification reaction.

Generally, each subsequent portion of the primers is independently added to a concentration, in addition to any primers already present in the amplification reaction, of from about 0.5 µM to about 25 µM. For example, each subsequent portion of the primers is independently added at a concentration, in addition to any primers already present in the amplification reaction, of from about 0.75 µM to about 24 µM, or from about 1 µM to about 23 µM, or from about 1 µM to about 15 µM, or from about 1.5 µM to about 22 µM, or from about 2 µM to about 21 µM, or from about 2.5 µM to about 20 µM, or from about 3 µM to about 19 µM, or from about 3.5 µM to about 18 µM, or from about 4 µM to about 17 µM, or from about 4.5 µM to about 16 µM, or from about 5 µM to about 15 µM. In some embodiment, each subsequent portion of the primers is independently added at a concentration, in addition to any primers already present in the amplification reaction, of from about 5 µM to about 10 µM. For example, each subsequent portion of the primers is independently added at a concentration, in addition to any primers already present in the amplification reaction, of about 0.5 µM, or about 1 µM, or about 1.5 µM, or about 2 µM, or about 2.5 µM, or about 3 µM, or about 3.5 µM, or about 4 µM, or about 4.5 µM, or about 5 µM, or about 5.5 µM, or about 6 µM, or about 6.5 µM, or about 7 µM, or about 7.5 µM, or about 8 µM, or about 8.5 µM, or about 9 µM, or about 9.5 µM, or about, 10 µM, or about 10.5 µM, or about 11 µM, or about 11.5 µM, or about 12 µM, or about 12.5 µM, or about 13 µM, or about 13.5 µM, or about 14 µM, or about 14.5 µM, or about 15 µM, or about 15.5 µM, or about 16 µM, or about 16.5 µM, or about 17 µM, or about 17.5 µM, or about 18 µM, or about 18.5 µM, or about 19 µM, or about 19.5 µM, or about, 10 µM, or about 20.5 µM, or about 21 µM, or about 21.5 µM, or about 22 µM, or about 22.5 µM, or about 23 µM, or about 23.5 µM, or about 24 µM, or about 24.5 µM, or about 25 µM. In some embodiments, each subsequent portion of the primers is independently added at a concentration, in addition to any primers already present in the amplification reaction, of from about 1 µM to about 15 µM, e.g.., from about 1.8 µM to about 14 µM, In some preferred embodiments, each subsequent portion of the primers is independently added at a concentration, in addition to any primers already present in the amplification reaction, of about 7 µM.

Stated in another way, each subsequent portion of the primers independently increases the concentration of the primers in the amplification reaction by from about 0.5 µM to about 25 µM. For example, each subsequent portion of the primers independently increases the concentration of the primers in the amplification reaction by from about 0.75 µM to about 24 µM, or from about 1 µM to about 23 µM, or from about 1.5 µM to about 22 µM, or from about 2 µM to about 21 µM, or from about 2.5 µM to about 20 µM, or from about 3 µM to about 19 µM, or from about 3.5 µM to about 18 µM, or from about 4 µM to about 17 µM, or from about 4.5 µM to about 16 µM, or from about 5 µM to about 15 µM, preferably from about 5 µM to about 10 µM. In some embodiments, each subsequent portion of the primers independently increases the concentration of the primers in the amplification reaction by about 0.5 µM, or about 1 µM, or about 1.5 µM, or about 2 µM, or about 2.5 µM, or about 3 µM, or about 3.5 µM, or about 4 µM, or about 4.5 µM, or about 5 µM, or about 5.5 µM, or about 6 µM, or about 6.5 µM, or about 7 µM, or about 7.5 µM, or about 8 µM, or about 8.5 µM, or about 9 µM, or about 9.5 µM, or about, 10 µM, or about 10.5 µM, or about 11 µM, or about 11.5 µM, or about 12 µM, or about 12.5 µM, or about 13 µM, or about 13.5 µM, or about 14 µM, or about 14.5 µM, or about 15 µM, or about 15.5 µM, or about 16 µM, or about 16.5 µM, or about 17 µM, or about 17.5 µM, or about 18 µM, or about 18.5 µM, or about 19 µM, or about 19.5 µM, or about, 10 µM, or about 20.5 µM, or about 21 µM, or about 21.5 µM, or about 22 µM, or about 22.5 µM, or about 23 µM, or about 23.5 µM, or about 24 µM, or about 24.5 µM, or about 25 µM. In some embodiments, each subsequent portion of the primers independently increases the concentration of the primers in the amplification reaction by from about 1 µM to about 15 µM, e.g.., from about 1.8 µM to about 14 µM, In some preferred embodiments, each subsequent portion of the primers independently increases the concentration of the primers in the amplification reaction by about 7 µM.

The subsequent portions of the primers can be provided to maintain a constant concentration of primers in the reaction mixture. Alternatively, or in addition, subsequent portions of the primers can be provided to replenish the concentration of primer, e.g., to maintain an amount of primers sufficient for the amplification reaction. It is noted that further primers are not added solely to replace a volume of reaction mixture taken for analysis or testing, but are supplied because the reaction requires further primer. It is preferred that the testing of the reaction mixture is non-invasive and that no material is withdrawn from the reaction mixture until the reaction is complete.

Further primers can be supplied in response to a need for more primers in the reaction mixture. This can be determined by several parameters, including, but not limited to % of primers remaining, i.e., the amount of primers not incorporated into a DNA molecule, in the reaction, the rate of DNA synthesis or the concentration of DNA synthesized or volume of the reaction mixture. The rate of DNA synthesis can be calculated from the concentration of DNA synthesized over time. Methods for calculating rate of DNA synthesis are well known in the art and available to one of skill in the art. As the rate of DNA synthesis starts to decrease and/or as the concentration of DNA increases to a threshold level, further primers can be supplied.

In some embodiments, each subsequent portion of the primers is added after at least about 40% or more of the primers already present in the amplification reaction have been used, i.e., have been incorporated into the synthesized DNA. For example, each subsequent portion of the primers is added after at least about 45%, about 60%, about 65%, about 60%, about 65%, about 70%, about 75%, about 80%, about 85%, or more of the primers in the amplification reaction have been used. In a preferred embodiment, each subsequent portion of the primers is added after at least about 60% or more of the primers already present in the amplification reaction have been used. It is noted that the % of primers used can be calculated based on the number of binding sites of the amplified molecule and the yield obtained.

The subsequent portions of the primers can be added to the amplification reaction at regular intervals. For example, subsequent portions of the primers can be added to the amplification reaction every 30 minutes, every 45 minutes, every 60 minutes, every 75 minutes, every 90 minutes, every 120 minutes, every 180 minutes or every 240 minutes or any other suitable time period. In some embodiments, the subsequent portions of the primers are added to the amplification reaction at intervals of from about 0.5 hours to about 2.5 hours. For example, the subsequent portions of the primers can be added to the amplification reaction at an interval of about 1 hour or about 2 hours.

The subsequent portions of the primers can be added to the amplification reaction at irregular intervals. For example, the time period between each addition of subsequent portion of nucleotides can be shorter initially, and then increase as the process proceeds, or vice versa

In some embodiments, the subsequent portions of the primers are supplied to the reaction mixture continuously, i.e. they are added in a continuous manner to the reaction mixture. Stated another way, the primers can be a drip-fed or constantly supplied. Each subsequent portions of primers according to this embodiment can be in sufficiently small amounts such that the supply of primers matches the demand by the polymerase for further primers. In this embodiment, the constant supply of primers maintains an about constant concentration of primers in the reaction mixture. It is preferred that the constant supply of further primers to the reaction mixture ensures that the concentration of primers in the reaction mixture is maintained within the higher and lower thresholds. For example, the subsequent portions of the primers can be supplied to the reaction mixture continuously at flow rates from about 0.01 mL/h to about 100 mL/h. It is noted that the flowrate is dependent of the scale. Thus, higher or lower flow rates can be used based on the scale and/or volume of the reaction.

The subsequent portion of primers can be physically provided to the reaction mixture, i.e. they are external to the reaction mixture. Thus, the supply can originate from an external source, such as a reservoir. This reservoir can be connected to the reaction mixture. The primers can be pumped into the reaction mixture. Thus, the primers and the reaction mixture are physically separate, and the supply of the further primers is a physical supply of nucleotides. Alternatively, the primers can be supplied via an osmotic pump.

It is noted that further primers are not added solely to replace a volume of reaction mixture taken for analysis or testing, but are supplied because the reaction requires further primers. It is preferred that the testing, if any, of the reaction mixture is non-invasive and that no material is withdrawn from the reaction mixture until the reaction is complete.

As used herein, the term "primer" refers to a short linear oligonucleotide that hybridizes to a target nucleic acid sequence (e.g., the DNA template to be amplified) to prime a nucleic acid synthesis reaction. The primer can be an RNA oligonucleotide, a DNA oligonucleotide, or a chimeric sequence. The primer can contain natural, synthetic, or modified nucleotides. Both the upper and lower limits of the length of the primer are empirically determined. The lower limit on primer length is the minimum length that is required to form a stable duplex upon hybridization with the target nucleic acid under nucleic acid amplification reaction conditions. Very short primers (usually less than 3 nucleotides long) do not form thermodynamically stable duplexes with target nucleic acid under such hybridization conditions. The upper limit is often determined by the possibility of having a duplex formation in a region other than the pre-determined nucleic acid sequence in the target nucleic acid. Generally, suitable primer lengths are in the range of about 3 nucleotides long to about 40 nucleotides long. A primer may be of 6 to 35, 8 to 30 or 10 to 30 nucleotides in length, or example 11, 12, 15, 18, 20 or 30 nucleotides in length. A preferred length for the primer is 11 nucleotides. Primer lengths/sequences can typically be selected based on temperature considerations i.e. as being able to bind to the template at the temperature used in the amplification step.

It is noted that the primers used in the method described herein can be non-specific (i.e. random in sequence, i.e., random primer) or can be specific for one or more sequences comprised within the DNA template. As used herein, the term "random primer" refers to a mixture of primer sequences, generated by randomizing a nucleotide at any given location in an oligonucleotide sequence in such a way that the given location may consist of any of the possible nucleotides or their analogues (complete randomization). Thus, the random primer is a random mixture of oligonucleotide sequences, consisting of every possible combination of nucleotides within the sequence. For example, a hexamer random primer may be represented by a sequence NNNNNN or (N)₆. A hexamer random DNA primer consists of every possible hexamer combinations of 4 nucleotides, i.e., A, C, G and T, resulting in a random mixture comprising 4⁶ (4,096) unique hexamer DNA oligonucleotide sequences. A random primer can be of 6 to 30, 8 to 30 or 12 to 30 nucleotides in length, for example 12, 15, 18, 20 or 30 nucleotides in length. Random primers can be effectively used to prime a nucleic acid synthesis reaction when the target nucleic acid's sequence is unknown or for performing a whole-genome amplification reaction. Without wishing to be bound by a theory, if the primers are of random sequence they allow for non-specific initiation at any site on the DNA template. This allows for high efficiency of amplification through multiple initiation reactions from each template strand.

In some embodiments, the primers or one or more of the primers are specific, i.e., a specific primer. As used herein, the term "specific primer" refers to a primer of a specified sequence, i.e., a primer the sequence of which is complementary to a specific nucleotide sequence in the template DNA to be amplified. In other words, specific primers have a sequence which is complementary to a sequence in the DNA template from which initiation of amplification is desired. In this embodiment, a pair of primers may be used to specifically amplify a portion of the DNA template which is internal to the two primers binding sites. Alternatively, a single specific primer can be used. For example, if the DNA template includes one or more protelomerase target sequences, it is possible to use a specific primer which binds the protelomerase target sequence. Since protelomerase target sequences are palindromic in nature, a specific primer which binds to the palindromic sequence can prime amplification on both strands of the DNA template. Such a process is described in detail in WO2012/017210.

Primers can be unlabeled, or can comprise one or more labels, for example radionuclides or fluorescent dyes. Primers can also comprise chemically modified nucleotides. For example, the primers can be capped in order to prevent initiation of DNA synthesis until the cap is removed, i.e., by chemical or physical means. The primers can also be modified, e.g., the primers can be modified with phosphorothioate bonds to prevent degradation by the exonuclease activity of the phi29.

### Nucleotides

A plurality of subsequent portions of the nucleotides can be supplied to the amplification reaction. For example, 1-10, 10-20, 20-25, 25-30, or 35-40 portions of the nucleotides can be added to the amplification reaction. In some embodiments, at least one, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or more subsequent portions of the nucleotides are added to the amplification reaction. For example, 1, 2, 3, 4, 5, 6, 7, 8, 9, or 10 subsequent portions of the nucleotides are added to the amplification reaction are made after start of the amplification reaction.

It noted that each subsequent addition of the nucleotides can be a portion of the total amount of nucleotides required for or added to the process. The volume and/or concentration of nucleotides in the portion can vary depending on the requirements of the process. A portion can comprise a small amount of nucleotides, i.e., an amount sufficient to ensure that a particular concentration or amount of nucleotides is present in the reaction mixture, e.g., a concentration or amount of nucleotides sufficient for the amplification reaction. Generally, the nucleotides are added to the amplification reaction in a total amount such that the total concentration of the nucleotides in the amplification reaction does not exceed about 6mM.

For example, the nucleotides are added to the amplification reaction such that the total concentration of the nucleotides added to the amplification reaction does not exceed about 6.0 mM, or about 5.5 mM, or about 5.0 mM, or about 4.5 mM, or about 4 mM, or about 3.5 mM, or about 3 mM, or about 2.5 mM, or about 2 mM. In some embodiment, a total concentration of the nucleotides added to the amplification reaction is from about 0.1 mM to about 6 mM. For example, a total concentration of the nucleotides required for or added to the amplification reaction is from about 0.25 mM to about 5.5 mM, or from about 0.3 mM to about 5 mM or from about 0.25 mM to about 4.5 mM. In some cases, the total concentration of the nucleotides added to the amplification reaction is about 0.1 mM, or about 0.15 mM, or about 0.2 mM, or about 0.25 mM, or about 0.3 mM, or about 0.35 mM, or about 0.4 mM, or about 0.45 mM, or about 0.5 mM, or about 0.55 mM, or about 0.6 mM, or about 0.65 mM, or about 0.7 mM, or about 0.75 mM, or about 0.8 mM, or about 0.85 mM, or about 0.9 mM, or about 0.95 mM, or about 1 mM, or about 1.25 mM, or about 1.5 mM, or about 1.75 mM, or about 1.25 mM, or about 2.5 mM, or about 2.75 mM, or about 3 mM, or about 3.25 mM, or about 3.5 mM, or about 3.75 mM, or about 4.25 mM, or about 4.5 mM, or about 4.75 mM, or about 5 mM. In some embodiments, the total concentration of the nucleotides added to the amplification reaction is from about 0.25 mM to about 1 mM, e.g., from about 0.3 mM to about 0.9 mM, or from about 0.4 mM to about 0.85 mM, or from about 0.45 mM to about 0.8 mM. In some embodiments, the total concentration of the nucleotides added to the amplification reaction is from about 0.5 mM to about 0.77 mM. In some embodiments, the total concentration of the nucleotides added to the amplification reaction is from about 2 mM to about 6 mM, e.g., from about 3 mM to about 5 mM, or from about 3.5 mM to about 4.5 mM. In some preferred embodiments, the total concentration of the nucleotides added to the amplification reaction is about 4 mM.

Generally, the initial amplification reaction comprises less than about 50% (mol% or weight%) of the total amount of nucleotides required for or added to the amplification reaction. Stated in another way, the initial amplification reaction comprises less than about half of the total amount of nucleotides required for or added to the amplification reaction. For example, the initial amplification reaction comprises from about 3% to about 50% of the total amount of nucleotides required for or added to the amplification reaction. In some embodiments, the initial amplification reaction comprises about 49%, or about 48%, or about 47%, or about 46%, or about 45%, or about 44%, or about 43%, or about 42%, or about 41%, or about 40%, or about 39%, or about 38%, or about 37%, or about 36%, or about 35%, or about 34%, or about 33%, or about 32%, or about 31%, or about 30%, about 29%, or about 28%, or about 27%, or about 26%, or about 25%, or about 24%, or about 23%, or about 22%, or about 21%, or about 20%, or about 19%, or about 18%, or about 17%, or about 16%, or about 15%, or about 14%, or about 13%, or about 12%, or about 11%, or about 10%, or about 9%, or about 8%, or about 7%, or about 6%, or about 5%, or about 4%, or about 3%, or about 2% or less (mol% or weight%) of the total amount of nucleotides required for or added to the amplification reaction. In some embodiments, the initial amplification reaction comprises from about 3% to about 13% of the total amount of nucleotides required for or added to the amplification reaction.

The amount of nucleotides in the initial amplification reaction will not be sufficient to synthesize the desired quantity of DNA from the reaction mix. In some embodiments, the, the initial amplification reaction comprises nucleotides at a concentration of from about 0.1 mM to about 1 mM, e.g., from about 0.2 mM to about 0.9 mM, or from about 0.3 mM to about 0.8 mM or from about 0.4 mM to about 0.7 mM. For example, the initial amplification reaction comprises nucleotides at a concentration of about 0.1 mM, or about 0.15 mM, or about 0.2 mM, or about 0.25 mM, or about 0.3 mM, or about 0.35 mM, or about 0.4 mM, or about 0.45 mM, or about 0.5 mM, or about 0.55 mM, or about 0.6 mM, or about 0.65 mM, or about 0.7 mM, or about 0.75 mM, or about 0.8 mM, or about 0.85 mM, or about 0.9 mM, or about 0.95 mM, or about 1 mM. In some embodiments, the initial amplification reaction comprises nucleotides at a concentration of about 0.1 mM, or about 0.11 mM, or about 0.12 mM, or about 0.13 mM, or about 0.14 mM, or about 0.15 mM, or about 0.16 mM, or about 0.17 mM, or about 0.18 mM, or about 0.19 mM, or about 0.20 mM, or about 0.21 mM, about 0.22 mM, or about 0.23 mM, or about 0.24 mM, or about 0.25 mM, or about 0.26 mM, or about 0.27 mM, or about 0.28 mM, or about 0.29 mM, or about 0.30 mM. Preferably, the initial amplification reaction comprises nucleotides at a concentration of from about 0.1 mM to about 0.3 mM. For example, the initial amplification reaction comprises nucleotides at a concentration of about 0.1 mM, or about 0.11 mM, or about 0.12 mM, or about 0.13 mM, or about 0.14 mM, or about 0.15 mM. In some preferred embodiments, the initial amplification reaction comprises nucleotides at a concentration of about 0.12 mM or 0.13 mM. In some other preferred embodiments, the initial amplification reaction comprises nucleotides at a concentration of about 0.25 mM. In yet some other preferred embodiments, the initial amplification reaction comprises nucleotides at a concentration of about 0.13 mM.

In some embodiments, the amount of each subsequent portion of the nucleotides is independently less than the amount of the nucleotides in the initial amplification reaction. For example, each subsequent portion of the nucleotides independently comprises less than about 75% (mol% or weight%) of the total amount of the nucleotides in the initial amplification reaction. Stated in another way, amount of each subsequent portion of the nucleotides independently is less than about ¾ of the of the total amount of the nucleotides in the initial amplification reaction. For example, the amount of each subsequent portion of the nucleotides is independently about 70%, or about 65%, or about 60%, or about 55%, or about 50%, or about 45%, or about 40%, or about 35%, or about 30%, or about 25%, or lower (mol% or weight%) of the total amount of the nucleotides in the initial amplification reaction.

In some embodiments, the amount of each subsequent portion of the nucleotides is about the same as the total amount of the nucleotides in the initial amplification reaction. In some embodiments, the amount of each subsequent portion of the nucleotides is independently from about 5% to about 30% (mol% or weight%) of the total amount of the nucleotides in the initial amplification reaction. For example, the amount of each subsequent portion of the nucleotides is independently from about 10% to about 25% or from about 15% to about 20%, e.g., or about 15%, or about 16%, or about 17%, or about 18%, or about 19% or about 20% (mol% or weight%) of the total amount of the nucleotides in the initial amplification reaction.

Generally, each subsequent portion of the nucleotides is independently added to a concentration, in addition to any nucleotides already present in the amplification reaction, of from about 0.001 mM to about 75 mM. For example, each subsequent portion of the nucleotides is independently added at a concentration, in addition to any nucleotides already present in the amplification reaction, of from about 0.005 mM to about 0. 7 mM, or from about 0.0075 mM to about 0.6 mM, or from about 0.01 mM to 0.5 mM, or from about 0.0125 mM to about 0.4 mM, or from about 0.025 to about 0.3 mM. In some preferred embodiments, each subsequent portion of the nucleotides is independently added at a concentration, in addition to any nucleotides already present in the amplification reaction, from about 0.05 mM to about 0.25 mM. In some embodiments, each subsequent portion of the nucleotides is independently added at a concentration, in addition to any nucleotides already present in the amplification reaction, of about 0.01 mM, or about 0.02 mM, or about 0.03 mM, or about 0.04 mM, or about 0.05 mM, or about 0.06 mM, or about 0.07 mM, or about 0.08 mM, or about 0.09 mM, or about 0.1 mM, or about 0.11 mM, or about 0.12 mM, or about 0.13 mM, or about 0.14 mM, or about 0.15 mM, or about 0.16 mM, or about 0.17 mM, or about 0.18 mM, or about 0.19 mM, or about 0.2 mM, or about 0.21 mM, or about 0.22 mM. or about 0.23 mM, or about 0.24 mM, or about 0.25 mM, or about 0.26 mM, or about 0.27 mM, or about 0.27 mM, or about 0.28 mM, or about 0.29 mM, or about 0.30 mM. For example, each subsequent portion of the nucleotides is independently added at a concentration, in addition to any nucleotides already present in the amplification reaction, of about 0.05 mM, or about 0.06 mM, or about 0.07 mM, or about 0.08 mM, or about 0.09 mM, or about 0.1 mM, or about 0.11 mM, or about 0.12 mM, or about 0.13 mM, or about 0.14 mM, or about 0.15 mM, or about 0.16 mM, or about 0.17 mM, or about 0.18 mM, or about 0.19 mM, or about 0.2 mM, or about 0.21 mM, or about 0.22 mM. or about 0.23 mM, or about 0.24 mM, or about 0.25 mM.

Stated in another way, each subsequent portion of the nucleotides independently increases the concentration of the nucleotides in the amplification reaction by from about 0.001 mM to about 0.75 mM. For example, each subsequent portion of the nucleotides independently increases the concentration of the nucleotides in the amplification reaction by from about 0.001 mM to about 0.15 mM, or from about 0.0075 mM to about 0.6 mM, or from about 0.01 mM to 0.5 mM, or from about 0.0125 mM to about 0.4 mM, or from about 0.025 to about 0.3 mM.. In some embodiments, each subsequent portion of the nucleotides independently increases the concentration of the nucleotides in the amplification reaction by from about 0.05 mM to about 0.25 mM. In some embodiments, each subsequent portion of the nucleotides independently increases the concentration of the nucleotides in the amplification reaction by from about 0.005 mM to about 0.125 mM, from about 0.01 mM to 0.1 mM, or from about 0.05 to about 0.09 mM. For example, each subsequent portion of the nucleotides independently increases the concentration of each subsequent portion of the nucleotides independently increases the concentration of the nucleotides in the amplification reaction by about 0.01 mM, or about 0.02 mM, or about 0.03 mM, or about 0.04 mM, or about 0.05 mM, or about 0.06 mM, or about 0.07 mM, or about 0.08 mM, or about 0.09 mM, or about 0.1 mM, or about 0.11 mM, or about 0.12 mM, or about 0.13 mM, or about 0.14 mM, or about 0.15 mM, or about 0.16 mM, or about 0.17 mM, or about 0.18 mM, or about 0.19 mM, about 0.2 mM, or about 0.21 mM, or about 0.22 mM. or about 0.23 mM, or about 0.24 mM, or about 0.25 mM, or about 0.26 mM, or about 0.27 mM, or about 0.27 mM, or about 0.28 mM, or about 0.29 mM, or about 0.30 mM. For example, each subsequent portion of the nucleotides independently increases the concentration of each subsequent portion of the nucleotides independently increases the concentration of the nucleotides in the amplification reaction by about 0.05 mM, or about 0.06 mM, or about 0.07 mM, or about 0.08 mM, or about 0.09 mM, or about 0.1 mM, or about 0.11 mM, or about 0.12 mM, or about 0.13 mM, or about 0.14 mM, or about 0.15 mM, or about 0.16 mM, or about 0.17 mM, or about 0.18 mM, or about 0.19 mM, about 0.2 mM, or about 0.21 mM, or about 0.22 mM. or about 0.23 mM, or about 0.24 mM, or about 0.25 mM.

Inventors have discovered that higher amounts of nucleotides in the amplification reaction can lead to mutations in the synthesized DNA. Accordingly, in some preferred embodiments, each subsequent portion of the nucleotides is independently added at a concentration, in addition to any nucleotides already present in the amplification reaction, less than about 0.3 mM. For example, each subsequent portion of the nucleotides is independently added at a concentration, in addition to any nucleotides already present in the amplification reaction, of about 0.05 mM, or about 0.06 mM, or about 0.07 mM, or about 0.08 mM, or about 0.09 mM, or about 0.1 mM, or about 0.11 mM, or about 0.12 mM, or about 0.13 mM, or about 0.14 mM, or about 0.15 mM, or about 0.16 mM, or about 0.17 mM, or about 0.18 mM, or about 0.19 mM, or about 0.2 mM, or about 0.21 mM, or about 0.22 mM. or about 0.23 mM, or about 0.24 mM, or about 0.25 mM.

The subsequent portions of the nucleotides can be provided to maintain a constant concentration of nucleotides in the reaction mixture. Alternatively, or in addition, subsequent portions of the nucleotides can be provided to replenish the concentration of nucleotides, e.g., to maintain an amount of nucleotides sufficient for the amplification reaction. It is noted that further nucleotides are not added solely to replace a volume of reaction mixture taken for analysis or testing, but are supplied because the reaction requires further nucleotides. It is preferred that the testing of the reaction mixture is non-invasive and that no material is withdrawn from the reaction mixture until the reaction is complete.

Further nucleotides can be supplied in response to a need for more nucleotides in the reaction mixture. This can be determined by several parameters, including, but not limited to % of nucleotides remaining, i.e., amount of nucleotides not incorporated into a DNA molecule, in the reaction, the rate of DNA synthesis or the concentration of DNA synthesized or volume of the reaction mixture. The rate of DNA synthesis can be calculated from the concentration of DNA synthesized over time. Methods for calculating rate of DNA synthesis are well known in the art and available to one of skill in the art. As the rate of DNA synthesis starts to decrease and/or as the concentration of DNA increases to a threshold level, further nucleotides can be supplied.

In some embodiments, each subsequent portion of the nucleotides is added after at least about 40% (weight or mol) or more of the nucleotides already present in the amplification reaction have been used, i.e., have been incorporated into the synthesized DNA. For example, each subsequent portion of the nucleotides is added after at least about 45%, about 50%, about 55%, about 60%, about 65%, about 70%, about 75%, about 80%, about 85%, or more of the nucleotides in the amplification reaction have been used. In a preferred embodiment, each subsequent portion of the nucleotides is added after at least about 50% or more of the nucleotides already present in the amplification reaction have been used. The percent of nucleotides that have been used up in the reaction can be calculated with the estimated activity of the Phi29, the half-life of the Phi29, the concentration of the dNTPs, the number of primer binding sites in the construct and the size and amount of template molecules in the reaction. For example, the amount of nucleotides consumed can be calculated by knowing the initial template molecules and their length, plus the yield obtained at a certain point of the amplification (final molecules). Stated in another way, based on the stoichiometry of the RCA reaction, new molecules molarity can be used to calculate the consumed dNTPs molarity.

It is noted that further nucleotides are not added solely to replace a volume of reaction mixture taken for analysis or testing, but are supplied because the reaction requires further nucleotides. It is preferred that the testing, if any, of the reaction mixture is non-invasive and that no material is withdrawn from the reaction mixture until the reaction is complete.

The nucleotides can be provided in a mixture of one or more suitable bases, preferably, one or more of adenine (A), guanine (G), thymine (T), cytosine (C). Two, three or preferably all four nucleotides (A, G, T, and C) are used in the method described herein. A molar ratio of different nucleotides in each portion of the nucleotides added to the amplification reaction can be 1:1 or different. Preferably, a molar ratio of A to G, A to C, T to G, and/or T to C in a portion of nucleotides added to the amplification reaction is 1:1.

In some embodiments, a molar ratio of A to G, A to C, T to G, or T to C in a portion of nucleotides added to the amplification reaction is higher than 1. For example, a molar ratio of A to G in a portion of nucleotides added to the amplification reaction is about 1.1 or higher. In some cases, a molar ratio of A to G in a portion of nucleotides added to the amplification reaction is about 1.2, or about 1.3, or about 1.4, or about 1.5, or about 1.6, or about 1.7, or about 1.8, or about 1.9, or about 2, or about 2.1, or about 2.2, or about 2.3, or about 2.4, or about 2.5, or about 2.6, or about 2.7, or about 2.8, or about 2.9, or about 3 or higher.

In some embodiments, a molar ratio of A to C in a portion of nucleotides added to the amplification reaction is about 1.1 or higher. For example, a molar ratio of A to C in a portion of nucleotides added to the amplification reaction is about 1.2, or about 1.3, or about 1.4, or about 1.5, or about 1.6, or about 1.7, or about 1.8, or about 1.9, or about 2, or about 2.1, or about 2.2, or about 2.3, or about 2.4, or about 2.5, or about 2.6, or about 2.7, or about 2.8, or about 2.9, or about 3 or higher.

In some cases, a molar ratio of A to G, and/or A to C in a portion of nucleotides added to the amplification reaction is about 70:30 (mol or weight ratio).

In some embodiments, a molar ratio of T to G in a portion of nucleotides added to the amplification reaction is about 1.1 or higher. For example, a molar ratio of T to G in a portion of nucleotides added to the amplification reaction is about 1.2, or about 1.3, or about 1.4, or about 1.5, or about 1.6, or about 1.7, or about 1.8, or about 1.9, or about 2, or about 2.1, or about 2.2, or about 2.3, or about 2.4, or about 2.5, or about 2.6, or about 2.7, or about 2.8, or about 2.9, or about 3 or higher.

In some embodiments, a molar ratio of T to C in a portion of nucleotides added to the amplification reaction is about 1.1 or higher. For example, a molar ratio of T to C in a portion of nucleotides added to the amplification reaction is about 1.2, or about 1.3, or about 1.4, or about 1.5, or about 1.6, or about 1.7, or about 1.8, or about 1.9, or about 2, or about 2.1, or about 2.2, or about 2.3, or about 2.4, or about 2.5, or about 2.6, or about 2.7, or about 2.8, or about 2.9, or about 3 or higher.

In some cases, a molar ratio of T to G, or T to C in a portion of nucleotides added to the amplification reaction is about 70:30.

In some embodiments, a molar ratio of G to A, G to T, C to A, or C to T in a portion of nucleotides added to the amplification reaction is higher than 1. For example, a molar ratio of G to A in a portion of nucleotides added to the amplification reaction is about 1.1 or higher. In some cases, a molar ratio of G to A in a portion of nucleotides added to the amplification reaction is about 1.2, or about 1.3, or about 1.4, or about 1.5, or about 1.6, or about 1.7, or about 1.8, or about 1.9, or about 2, or about 2.1, or about 2.2, or about 2.3, or about 2.4, or about 2.5, or about 2.6, or about 2.7, or about 2.8, or about 2.9, or about 3 or higher.

In some embodiments, a molar ratio of G to T in a portion of nucleotides added to the amplification reaction is about 1.1 or higher. For example, a molar ratio of G to T in a portion of nucleotides added to the amplification reaction is about 1.2, or about 1.3, or about 1.4, or about 1.5, or about 1.6, or about 1.7, or about 1.8, or about 1.9, or about 2, or about 2.1, or about 2.2, or about 2.3, or about 2.4, or about 2.5, or about 2.6, or about 2.7, or about 2.8, or about 2.9, or about 3 or higher.

In some embodiments, a molar ratio of C to A in a portion of nucleotides added to the amplification reaction is about 1.1 or higher. For example, a molar ratio of C to A in a portion of nucleotides added to the amplification reaction is about 1.2, or about 1.3, or about 1.4, or about 1.5, or about 1.6, or about 1.7, or about 1.8, or about 1.9, or about 2, or about 2.1, or about 2.2, or about 2.3, or about 2.4, or about 2.5, or about 2.6, or about 2.7, or about 2.8, or about 2.9, or about 3 or higher.

In some embodiments, a molar ratio of C to T in a portion of nucleotides added to the amplification reaction is about 1.1 or higher. For example, a molar ratio of C to T in a portion of nucleotides added to the amplification reaction is about 1.2, or about 1.3, or about 1.4, or about 1.5, or about 1.6, or about 1.7, or about 1.8, or about 1.9, or about 2, or about 2.1, or about 2.2, or about 2.3, or about 2.4, or about 2.5, or about 2.6, or about 2.7, or about 2.8, or about 2.9, or about 3 or higher.

The subsequent portions of the nucleotides can be added to the amplification reaction at regular intervals. For example, subsequent portions of the nucleotides can be added to the amplification reaction every 30 minutes, every 45 minutes, every 60 minutes, every 75 minutes, every 90 minutes, every 120 minutes, every 180 minutes or every 240 minutes or any other suitable time period. In some embodiments, the subsequent portions of the nucleotides are added to the amplification reaction at intervals of from about 1.5 hours to about 2.5 hours. For example, the subsequent portions of the nucleotides can be added to the amplification reaction at an interval of about 2 hours.

The subsequent portions of the nucleotides can be added to the amplification reaction at irregular intervals. For example, the time period between each addition of subsequent portion of nucleotides can be shorter initially, and then increase as the process proceeds, or vice versa.

In some embodiments, the subsequent portions of the nucleotides are supplied to the reaction mixture continuously, i.e. they are added in a continuous manner to the reaction mixture. Stated another way, the nucleotides can be a drip-fed or constantly supplied. Each subsequent portions of nucleotides according to this embodiment can be in sufficiently small amounts such that the supply of nucleotides matches the demand by the polymerase for further nucleotides. In this embodiment, the constant supply of nucleotides maintains an about constant concentration of nucleotides in the reaction mixture. It is preferred that the constant supply of further nucleotides to the reaction mixture ensures that the concentration of nucleotides in the reaction mixture is maintained within the higher and lower thresholds. For example, the subsequent portions of the nucleotides can be supplied to the reaction mixture continuously at flow rates from about 0.01 mL/h to about 100 mL/h. It is noted that the flowrate is dependent of the scale. Thus, higher or lower flow rates can be used based on the scale and/or volume of the reaction.

The subsequent portion of nucleotides can be physically provided to the reaction mixture, i.e. they are external to the reaction mixture. Thus, the supply can originate from an external source, such as a reservoir. This reservoir can be connected to the reaction mixture. The nucleotides can be pumped into the reaction mixture. Thus, the nucleotides and the reaction mixture are physically separate, and the supply of the further nucleotides is a physical supply of nucleotides. Alternatively, the nucleotides can be supplied via an osmotic pump.

Alternatively, subsequent portions of the nucleotides can be provided to the amplification reaction mixture by activating biologically inactivated nucleotides. In order to supply the nucleotides to the reaction mixture, the biologically inactive nucleotides are activated by suitable means. Thus, the nucleotides can comprise any suitable biologically inactive nucleotides. The biologically inactive nucleotides can be included in the reaction mixture at the start but only supplied to the reaction mixture by controlled activation. Alternatively, the biologically inactive nucleotides can be physically added to the reaction mixture in aliquots or continuously, and subsequently activated to supply the nucleotides to the reaction mixture. The activation can be physical (i.e. heat or light) or chemical, and it is the control of activation that controls supply of the nucleotide.

It is noted that any suitable nucleotide can be used in the method described herein. For example, the nucleotide can be in the form of deoxynucleoside triphosphate (dNTP). Suitable dNTPs include, but are not limited to, dATP (deoxyadenosine triphosphate), dGTP (deoxyguanosine triphosphate), dTTP (deoxythymidine triphosphate), dUTP (deoxyuridine triphosphate), dCTP (deoxycytidine triphosphate), dITP (deoxyinosine triphosphate), dXTP (deoxyxanthosine triphosphate), and derivatives and modified versions thereof. It is preferred to use a mixture of dATP, dGTP, dTTP and dCTP or modified version thereof.

The subsequent portions of the nucleotides can be in solution or provided in lyophilized form. In some preferred embodiments, subsequent portions of the nucleotides are in solution.

The nucleotides can be present as monovalent metal ion nucleotide salts, divalent metal ion nucleotide salts, or a mixture of monovalent and divalent salts. Exemplary monovalent metal ions include, but are not limited to, to lithium (Li⁺), sodium (Na⁺), ammonium (NH4+) or potassium (K⁺). Exemplary divalent metal ions include, but not limited to, magnesium (Mg²⁺), manganese (Mn²⁺), calcium (Ca²⁺), beryllium (Be²⁺), zinc (Zn²⁺) and strontium (Sr²⁺).

In some embodiments, nucleotides can be present as a nucleotide salt, and said salt comprises a monovalent cation having an ionic radius greater than the ionic radius of sodium ions. See, for example, US Patent Publication No. 20210301312, content of which is incorporated herein by reference in its entirety, describing nucleotide salts comprising a monovalent cation having an ionic radius greater than the ionic radius of sodium ions.

In some embodiments, one or more nucleotides can be present as a nucleotide complex, where the nucleotide complex comprises nucleotides that are bound to 0.2 to 2 divalent cations and 0.2 to 2.5 monovalent cations per nucleotide. See, for example, US Patent Publication No. 20230091493, content of which is incorporated herein by reference in its entirety, describing nucleotide complexes comprising nucleotides that are bound to 0.2 to 2 divalent cations and 0.2 to 2.5 monovalent cations per nucleotide.

The nucleotides can comprise modified nucleotides. The modified nucleotide can be a biologically inactive nucleotide. A biologically inactivated nucleotide can have a removable moiety protecting a group of the nucleotide, such as the 3' carbon oxygen or the terminal phosphate oxygen. A biologically inactive nucleotide can be a caged nucleotide or a blocked nucleotide. Suitable moieties include but are not limited to photoactivatable (caging) group, including a-carboxy-2-nitrobenzyl (CNB), 1- (2-nitrophenyl)ethyl (NPE), 4,5-dimethoxy-2-nitrobenzyl (DM NB), I-(4,5-dimethoxy-2-nitrophenyl)ethyl (DMNPE) and 5-carboxymethoxy-2-nitrobenzyl (CMNB) (Molecular Probes). These groups are generally attached via terminal phosphate oxygen. The moiety can be removed by any suitable means. For example, if the moiety is photolabile, flash photolysis of the moiety by ultraviolet light leads to a rapid and highly localized release of the biologically active nucleotide at the site of illumination. The moiety can be heat labile, and thus the biologically active nucleotide can be released by heat. If heat is used to biologically activate the nucleotide, the temperature requirements of the polymerase must be considered, and this is within the remit of the skilled person. For example, the terminal phosphate can be esterified with a caging or blocking moiety.

The nucleotides can all be natural nucleotides (i.e. unmodified), they can be modified nucleotides that act like natural nucleotides and are biologically active (i.e. LNA nucleotides - locked nucleic acid), they can be modified and biologically inactive or they can be a mixture of unmodified and modified nucleotides, and/or a mixture of biologically active and biologically inactive nucleotides. Each type (i.e. base) of nucleotide can be provided in one or more forms, i.e. unmodified and modified, or biologically active and biologically inactive.

### Polymerase

It is noted that a single polymerase or more than one polymerase, such as two, three, four, five or more different polymerases can be used. The polymerase can be any suitable polymerase, such that it synthesizes DNA. Any polymerase can be used, including any commercially available DNA polymerase. Two, three, four, five or more different polymerases can be used, for example one which provides a proofreading function and one or more others which do not. DNA polymerases having different mechanisms can be used e.g. strand displacement type polymerases and polymerases replicating DNA by other methods.

A polymerase can be highly stable, such that its activity is not substantially reduced by prolonged incubation under process conditions. Therefore, the polymerase preferably has a long half-life under a range of process conditions including but not limited to temperature and pH. In some embodiments, the polymerase has one or more characteristics suitable for a manufacturing process. The polymerase preferably has high fidelity, for example through having proofreading activity. Furthermore, it is preferred that a polymerase displays high processivity, high strand-displacement activity and a low Km for dNTPs and DNA. A polymerase can be capable of using circular and/or linear DNA as template. The polymerase can be capable of using dsDNA or ssDNA as a template. In some embodiments, the polymerase does not display DNA exonuclease activity that is not related to its proofreading activity.

In some embodiments, the polymerase is a DNA polymerase.

In some embodiments, the polymerase is a polymerase having strand displacement activity (a strand displacing polymerase). For example, the polymerase is a DNA polymerase having DNA strand displacement activity. "Strand displacement" describes the ability to displace downstream DNA encountered during synthesis.

A strand displacing polymerase used in the method described herein preferably has a processivity of at least about 10 kb. For example, the strand displacing polymerase has a processivity of at least about 15 kb, or at least about 20 kb, or at least about 25 kb, or at least about 30 kb, or at least about 35 kb, or at least about 40 kb, or at least about 45 kb, or at least about 50 kb, or at least about 55 kb, or at least about 60 kb, or at least about 65 kb, or at least about 70 kb, or at least about 75 kb, or greater. In some embodiments, the strand displacing polymerase has a processivity that is comparable to, or greater than the processivity of phi29 DNA polymerase.

Examples of polymerases having DNA strand displacement activity that can be used as provided herein include, without limitation, Phi29 (New England Biolabs, Inc., Ipswich, Mass., US), Deep Vent^{®} (New England Biolabs, Inc.), *Bacillus stearothermophilus* (Bst) DNA polymerase I (New England Biolabs, Inc.), Klenow fragment of DNA polymerase I (New England Biolabs, Inc.), M-MuLV reverse transcriptase (New England Biolabs, Inc.), VentR^{®}(exo-minus) DNA polymerase (New England Biolabs, Inc.), VentR^{®} DNA polymerase (New England Biolabs, Inc.), Deep Vent^{®} (exo-) DNA polymerase (New England Biolabs, Inc.), Bst DNA polymerase large fragment (New England Biolabs, Inc.) and Bacillus subtilis Pol I (Bsu) polymerase, or mutants or variants of any one of them. Other polymerases having strand displacement activity can also be used.

In some embodiments, the polymerase is phi29 DNA polymerase or a mutant or variant thereof. For example, the polymerase is phi29 DNA polymerase, phi29 DNA polymerase (PacBio), 4BB QualiPhi^{™} (4basebio), EquiPhi^{™} (ThermoFisher), phi29 DNA polymerase (MedChemExpress) or phi-29-XT (New England Bio). Preferably, the polymerase is a phi29 DNA polymerase.

### Amplification

The method described herein comprises amplification of a DNA template, i.e., a step of amplifying the DNA template. As used herein, the term "amplifying" refers to a step of submitting a nucleic acid strand to conditions sufficient to allow for amplification of a polynucleotide if all of the components of the reaction are intact. Components of an amplification reaction include, e.g., primers, a polynucleotide template, polymerase, nucleotides, and the like. The term "amplifying" typically refers to an "exponential" increase in target nucleic acid. However, "amplifying" as used herein can also refer to linear increases in the numbers of a select target sequence of nucleic acid, such as is obtained with cycle sequencing. Methods of amplifying and synthesizing nucleic acid sequences are known in the art. For example, see US Patent Nos. 7,906.282, 8,367,328, 5,518,900, 7,378,262, 5,476,774, and 6,638,722, contents of all of which are incorporated by reference herein in their entirety. Such methods include, but are not limited to, isothermal amplification, polymerase chain reaction (PCR) and variants of PCR such as Rapid amplification of cDNA ends (RACE), ligase chain reaction (LCR), multiplex RT-PCR, immuno-PCR, SSIPA, qPCR, Real Time RT-qPCR and nanofluidic digital PCR.

In some embodiments of any of the aspects, the amplification is isothermal amplification. As used herein, "isothermal amplification" refers to amplification that occurs at a single temperature. For example, the amplification process is performed at a single temperature or where the major aspect of the amplification process is performed at a single temperature. Generally, isothermal amplification relies on the ability of a polymerase to copy the template strand being amplified to form a bound duplex. Isothermal amplification permits rapid and specific amplification of a target nucleic acid at a constant temperature. In general, isothermal amplification is comprised of (i) hybridization of primers to sequences within a target nucleic acid, and (ii) subsequent amplification involving multiple rounds of primer annealing, elongation, and strand displacement (as a non-limiting example, using a strand displacing DNA polymerase). The primers used in isothermal amplification are oligonucleotides of sufficient length and appropriate sequence to provide initiation of polymerization, e.g., each primer can be specifically designed to be complementary to a strand of the template polynucleotide to be amplified.

Non-limiting examples of isothermal amplification include, but are not limited to, Rolling Circle Amplification (RCA), Loop Mediated Isothermal Amplification (LAMP), Recombinase Polymerase Amplification (RPA), Helicase-dependent isothermal DNA amplification (HDA), Nucleic acid sequence-based amplification (NASBA), strand displacement amplification (SDA), nicking enzyme amplification reaction (NEAR), and polymerase Spiral Reaction (PSR). See e.g., Yan et al., Isothermal amplified detection of DNA and RNA, March 2014, Molecular BioSystems 10(5), DOI: 10.1039/c3mb70304e, the content of which is incorporated herein by reference in its entirety.

In some preferred embodiments, the isothermal amplification reaction(s) is Rolling Circle Amplification. As used herein, the term "rolling circle amplification (RCA)" refers to a nucleic acid amplification reaction that amplifies a circular nucleic acid template (e.g., single/double stranded DNA circles) via a rolling circle mechanism. Rolling circle amplification reaction is initiated by the hybridization of a primer to a circular, often single-stranded, nucleic acid template. The nucleic acid polymerase then extends the primer that is hybridized to the circular nucleic acid template by continuously progressing around the circular nucleic acid template to replicate the sequence of the nucleic acid template over and over again (rolling circle mechanism). The rolling circle amplification typically produces concatamers comprising tandem repeat units of the circular nucleic acid template sequence. The rolling circle amplification may be a linear RCA (LRCA), exhibiting linear amplification kinetics (e.g., RCA using a single, specific primer), or may be an exponential RCA (ERCA) exhibiting exponential amplification kinetics. Rolling circle amplification may also be performed using multiple primers (multiply primed rolling circle amplification or MPRCA) leading to hyper-branched concatamers. For example, in a double-primed RCA, one primer may be complementary, as in the linear RCA, to the circular nucleic acid template, whereas the other may be complementary to the tandem repeat unit nucleic acid sequences of the RCA product. Consequently, the double-primed RCA may proceed as a chain reaction with exponential amplification kinetics featuring a cascade in series of multiple-hybridization, primer-extension, and strand-displacement events involving both the primers and both strands. This often generates a discrete set of concatemeric, double-stranded nucleic acid amplification products. The RCA may be performed *in vitro* under isothermal conditions using a suitable nucleic acid polymerase such as a polymerase possessing strand displacement DNA synthesis ability, e.g., phi29 DNA polymerase.

In some embodiments, the isothermal amplification reaction(s) is Loop Mediated Isothermal Amplification (LAMP). LAMP is a single tube technique for the amplification of DNA; LAMP uses 4-6 primers, which form loop structures to facilitate subsequent rounds of amplification.

In some embodiments, the isothermal amplification reaction(s) is Recombinase Polymerase Amplification (RPA). RPA is a low temperature DNA and RNA amplification technique. The RPA process employs three core enzymes - a recombinase, a single-stranded DNA-binding protein (SSB) and strand-displacing polymerase. Recombinases are capable of pairing oligonucleotide primers with homologous sequence in duplex DNA. SSB bind to displaced strands of DNA and prevent the primers from being displaced. Finally, the strand displacing polymerase begins DNA synthesis where the primer has bound to the target DNA. By using two opposing primers, much like PCR, if the target sequence is indeed present, an exponential DNA amplification reaction is initiated. No other sample manipulation such as thermal or chemical melting is required to initiate amplification. At optimal temperatures (e.g., 37-42 °C), the RPA reaction progresses rapidly and results in specific DNA amplification from just a few target copies to detectable levels, typically within 10 minutes, for rapid detection of the target nucleic acid. In some embodiments of any of the aspects, the single-stranded DNA-binding protein is a gp32 SSB protein. In some embodiments of any of the aspects, the recombinase is a UvsX recombinase. See e.g., US Patent 7,666,598, the content of which is incorporated herein by reference in its entirety. In some embodiments of any of the aspects, RPA can also be referred to as Recombinase Aided Amplification (RAA).

In some embodiments, the isothermal amplification reaction(s) is Helicase-dependent isothermal DNA amplification (HDA). HDA uses the double-stranded DNA unwinding activity of a helicase to separate strands for *in vitro* DNA amplification at constant temperature. In some embodiments of any of the aspects, the helicase is a thermostable helicase, which can improve the specificity and performance of HDA; as such, the isothermal amplification reaction(s) can be thermophilic helicase-dependent amplification (tHDA). As a non-limiting example, the helicase is the thermostable UvrD helicase (Tte-UvrD), which is stable and active from 45 to 65 °C.

In some embodiments, the isothermal amplification reaction(s) is Nucleic acid sequence-based amplification (NASBA), which is also known as transcription mediated amplification (TMA). NASBA is an isothermal technique predominantly used for the amplification of RNA through the cyclic formation of complimentary DNA and destruction of original RNA sequence (e.g., using RNase H). The NASBA reaction mixture contains three enzymes-reverse transcriptase (RT), RNase H, and T7 RNA polymerase-and two primers. T7 RNA Polymerase is an RNA polymerase from the T7 bacteriophage that catalyzes the formation of RNA from DNA in the 5'→ 3' direction. Primer 1 (P1) contains a 3' terminal sequence that is complementary to a sequence on the target nucleic acid and a 5' terminal (+) sense sequence of a promoter that is recognized by the T7 RNA polymerase. Primer 2 (P2) contains a sequence complementary to the P1-primed DNA strand. The NASBA enzymes and primers operate in concert to amplify a specific nucleic acid sequence exponentially. NASBA results in the amplification of the target RNA to cDNA to RNA to cDNA, etc., with alternating reverse transcription (e.g., RNA to DNA) and transcription steps (e.g., DNA to RNA), and the RNA being degraded after each transcription.

In some embodiments, the isothermal amplification reaction(s) is Strand Displacement Amplification (SDA). SDA is an isothermal, *in vitro* nucleic acid amplification technique based upon the ability of the restriction endonuclease Hincll to nick the unmodified strand of a hemiphosphorothioate form of its recognition site, and the ability of exonuclease deficient klenow (exo-klenow) DNA polymerase to extend the 3'-end at the nick and displace the downstream DNA strand. Exponential amplification results from coupling sense and antisense reactions in which strands displaced from a sense reaction serve as target for an antisense reaction and vice versa.

In some embodiments, the isothermal amplification reaction(s) is nicking enzyme amplification reaction (NEAR), which is a similar approach to SDA. In NEAR, DNA is amplified at a constant temperature (e.g., 55 °C to 59 °C) using a polymerase and nicking enzyme. The nicking site is regenerated with each polymerase displacement step, resulting in exponential amplification.

In some embodiments, the isothermal amplification reaction(s) is Polymerase Spiral Reaction (PSR). The PSR method employs a DNA polymerase (e.g., Bst) and a pair of primers. The forward and reverse primer sequences are reverse to each other at their 5' end, whereas their 3' end sequences are complementary to their respective target nucleic acid sequences. The PSR method is performed at a constant temperature 61 °C-65 °C, yielding a complicated spiral structure.

In some embodiments of any of the aspects, the isothermal amplification reaction(s) is polymerase cross-linking spiral reaction (PCLSR). PCLSR uses three primers (e.g., two outer-spiral primers and a cross-linking primer) to produce three independent prerequisite spiral products, which can be cross-linked into a final spiral amplification product.

### DTT

In some embodiments, the amplification reaction further comprises a DNA polymerase stabilizing agent. Exemplary DNA polymerase stabilizing agents include, but are not limited to, bovine serum albumin (BSA) and other stabilizing proteins, reducing agents, glycerol, TMANO, MMO, ethylene glycol, ethanol, isopropanol, and Trehalose,

The DNA polymerase stabilizing agent can be a reducing agent. Thus, in some embodiments, the amplification reaction further comprises a reducing agent. Exemplary reducing agents include, but are not limited to, dithiothreitol (DTT), β-mercaptoethanol, cysteine, dithioerythritol, glutathione.1,2-propanediol, and acetyl cysteine.

In some embodiments, the amplification reaction further comprises dithiothreitol (DTT). The amplification reaction can comprise DTT in a concentration of upto about 5 mM, or upto about 4.5 mM, or upto about 4 mM, or upto about 3.5 mM, or upto about 3 mM, or upto about 2.5 mM, or upto about 2 mM. In some embodiment, the concentration of the DTT in the amplification reaction is from about 0.1 mM to about 5 mM. For example, the concentration of the DTT in the amplification reaction is from about 0.5 mM to about 4 mM, or from about 1 mM to about 3 mM or from about 1.5 mM to about 2.5 mM. For example, the concentration of DTT in the amplification reaction is from about 1.75 mM to about 2.25 mM. In some cases, the concentration of DTT in the amplification reaction is about 0.1 mM, or about 0.2 mM, or about 0.3 mM, or about 0.4 mM, or about 0.5 mM, or about 0.6 mM, or about 0.7 mM, or about 0.8 mM, or about 0.9 mM, or about 1 mM, or about 1.1 mM, or about 1.2 mM, or about 1.3 mM, or about 1.4 mM, or about 1.5 mM, or about 1.6 mM, or about 1.7 mM, or about 1.8 mM, or about 1.9 mM, or about 2 mM, or about 2.1 mM, or about 2.2 mM, or about 2.3 mM, or about 2.4 mM, or about 2.5 mM, or about 2.6 mM, or about 2.7 mM, or about 2.8 mM, or about 2.9 mM, or about 3 mM ,or about 3.1 mM, or about 3.2 mM, or about 3.3 mM, or about 3.4 mM, or about 3.5 mM, or about 3.6 mM, or about 3.7 mM, or about 3.8 mM, or about 3.9 mM, or about 4 mM, or about 4.1 mM, or about 4.2 mM, or about 4.3 mM, or about 4.4 mM, or about 4.5 mM, or about 4.6 mM, or about 4.7 mM, or about 4.8 mM, or about 4.9 mM, or about 5 mM. In some preferred embodiments, the concentration of DTT in the amplification reaction is about 1.5 mM, or about 1.6 mM, or about 1.7 mM, or about 1.8 mM, or about 1.9 mM, or about 2 mM, or about 2.1 mM, or about 2.2 mM, or about 2.3 mM, or about 2.4 mM, or about 2.5 mM. Preferably, the concentration of DTT in the amplification reaction is about 2 mM.

In some embodiments, the amplification reaction does not comprise any DTT.

### Reaction time

Generally, the amplification reaction is carried out for a time period sufficient to synthesize the desired quantity of DNA from the reaction mix. For example, the amplification reaction is performed for at least about 6 hours or more. In some embodiments, the amplification reaction is performed for at least about 6.5 hours, or at least about 7 hours, or at least about 7.5 hours, or at least about 8 hours, or at least about 8.5 hours, or at least about 9 hours, or at least about 9.5 hours, or at least about 10 hours, or at least about 10.5 hours, or at least about 11 hours, or at least about 11.5 hours, or at least about 12 hours, or at least about 12.5 hours, or at least about 13 hours, or at least about 13.5 hours, or at least about 14 hours, or at least about 14.5 hours, or at least about 15 hours, or at least about 15.5 hours, or at least about 16 hours, or at least about 16.5 hours, or at least about 17 hours, or at least about 17.5 hours, or at least about 18 hours, or at least about 18.5 hours, or at least about 19 hours, or at least about 19.5 hours, or at least about 20 hours, or at least about 20.5 hours, or at least about 21 hours, or at least about 21.5 hours, or at least about 22 hours, or at least about 22.5 hours, or at least about 23 hours, or at least about 23.5 hours, or at least about 24 hours, or at least about 24.5 hours, or at least about 25 hours, or at least about 25.5 hours, or at least about 26 hours, or at least about 26.5 hours, or at least about 27 hours, or at least about 27.5 hours, or at least about 28 hours, or at least about 28.5 hours, or at least about 29 hours, or at least about 29.5 hours, or at least about 30 hours, or at least about 30.5 hours, or at least about 31 hours, or at least about 31.5 hours, or at least about 32 hours, or at least about 32.5 hours, or at least about 33 hours, or at least about 33.5 hours, or at least about 34 hours, or at least about 34.5 hours, or at least about 35 hours, or at least about 35.5 hours, or at least about 36 hours, or at least about 36.5 hours, or at least about 37 hours, or at least about 37.5 hours, or at least about 38 hours, or at least about 38.5 hours, or at least about 39 hours, or at least about 39.5 hours, or at least about 40 hours, or at least about 40.5 hours, or at least about 41 hours, or at least about 41.5 hours, or at least about 42 hours, or at least about 42.5 hours, or at least about 43 hours, or at least about 43.5 hours, or at least about 44 hours, or at least about 44.5 hours, or at least about 45 hours, or at least about 45.5 hours, or at least about 46 hours, or at least about 46.5 hours, or at least about 47 hours, or at least about 47.5 hours, or at least about 48 hours. In some embodiments, the amplification reaction is performed for a period of from about 8 hours to about 31 hours.

### Temperature

Generally, the amplification reaction is performed under conditions promoting amplification of the DNA template. The conditions can comprise use of any temperature allowing for amplification of DNA. Typically, an appropriate temperature is selected based on the temperature at which a specific polymerase has optimal activity. This information is commonly available and forms part of the general knowledge of the skilled person. The skilled person would routinely be able to identify a suitable temperature for efficient amplification according to the method described herein. For example, the amplification can be performed at a range of temperatures, and yields of amplified DNA could be monitored to identify an optimal temperature range for a given polymerase. The amplification can be carried out at a constant temperature.

The amplification reaction can be performed at a temperature in the range of about 20°C to about 90°C. A preferred temperature range can be from about 20°C to about 40°C, or about 25°C to about 35°C.

In some embodiments, the amplification is at a temperature of about 20°C, about 21°C, about 22°C, about 23°C, about 24°C, about 25°C, about 26°C, about 27°C, about 28°C, about 29°C, about 30°C, about 31°C, about 32°C, about 33°C, about 34°C, about 35°C, about 36°C, about 37°C, about 38°C, about 39°C, about 40°C, about 41°C, about 42°C, about 43°C, about 44°C, or about 45°C. Preferably, the amplification is at a temperature of about 25°C, about 26°C, about 27°C, about 28°C, about 29°C, about 30°C, about 31°C, about 32°C, about 33°C, about 34°C, about 35°C, about 36°C, about 37°C, about 38°C, about 39°C, or about 40°C. For example, the amplification is at a temperature of about 27°C, about 28°C, about 29°C, about 30°C, about 31°C, about 32°C, about 33°C, about 34°C, or about 35°C. In some preferred embodiments, amplification is at a temperature of about 30°C.

In some embodiments, the amplification is performed at least at least about 20°C, at least about 21°C, at least about 22°C, at least about 23°C, at least about 24°C, at least about 25°C, at least about 26°C, at least about 27°C, at least about 28°C, at least about 29°C, at least about 30°C, at least about 31°C, at least about 32°C, at least about 33°C, at least about 34°C, at least about 35°C, at least about 36°C, at least about 37°C, at least about 38°C, at least about 39°C, at least about 40°C, at least 41°C, at least about 42°C, at least about 43°C, at least 4 about 4°C, or about at least 45°C.

In some embodiments, the amplification is at at most about 20°C, at most about 21°C, at most about 22°C, at most about 23°C, at most about 24°C, at most about 25°C, at most about 26°C, at most about 27°C, at most about 28°C, at most about 29°C, at most about 30°C, at most about 31°C, at most about 32°C, at most about 33°C, at most about 34°C, at most about 35°C, at most about 36°C, at most about 37°C, at most about 38°C, at most about 39°C, at most about 40°C, at most about 41°C, at most about 42°C, at most about 43°C, at most about 44°C, or at most about 45°C.

When a phi29 DNA polymerase is used, a suitable temperature range would be about 25°C to about 35°C. For example, when a phi29 DNA polymerase is used, amplification can be a temperature of about 25°C, about 26°C, about 27°C, about 28°C, about 29°C, about 30°C, about 31 °C, about 32°C, about 33°C, about 34°C, or about 35°C, preferably about 28°C, about 29°C, about 30°C, about 31°C, about 32°C, more preferably about 30°C.

### pH

Other conditions promoting amplification of the DNA template comprise the presence of suitable buffering agents/pH and other factors which are required for enzyme performance or stability. Suitable conditions include any conditions used to provide for activity of polymerase enzymes known in the art.

Accordingly, the pH of the reaction can be from about 3 to about 10. For example, pH of the reaction can be from about 4 to about 9, or about 5 to about 8. In some embodiments, the pH of the reaction is about 4.5, or about 5, or about 5.5, or about 6, or about 6.5, or about 7, or about 7.5, or about 8, or about 8.5, or about 9. In some preferred embodiments, the pH of the reaction is about 6.5, or about 7, or about 7.5, or about 8, or about 8.5. For example, the pH of the reaction is about 7, or about 7.5, or about 8. In some embodiments, the pH of the reaction is about 7.5. In some other embodiments, the pH of the reaction is about 8.

The pH of the reaction can be maintained by use of one or more buffering agents. Such buffers include, but are not limited to, Tris-base, Tris-HCl, MES, Bis-Tris, ADA, ACES, PIPES, MOBS, MOPS, MOPSO, Bis-Tris Propane, BES, TES, HEPES, DIPSO, TAPSO, Trizma, HEPPSO, POPSO, TEA, EPPS, Tricine, Gly-Gly, Bicine, HEPBS, TAPS, AMPD, TABS, AMPSO, CHES, CAPSO, AMP, CAPS, CABS, phosphate, citric acid-sodium hydrogen phosphate, citric acid-sodium citrate, sodium acetate-acetic acid, imidazole and sodium carbonate-sodium bicarbonate. In some embodiments, the buffer is Tris-base or Tris-HCl.

### Additional components/reagents

The amplification reaction can also include additional components and reagents as needed for amplification. For example, the amplification reaction can comprise metal ions and/or salt of metal ions. The amplification reaction can comprise salts of metals such as, but not limited to, salts of divalent metal ions, including but not limited to, magnesium (Mg²⁺), manganese (Mn²⁺), calcium (Ca²⁺), beryllium (Be²⁺), zinc (Zn²⁺) and strontium (Sr²⁺), and/or salts of monovalent metal ions, including but not limited to lithium (Li⁺), sodium (Na⁺) or potassium (K⁺). The salts can include chlorides, acetates and sulphates. Other salts that can be included are ammonium salts, in particular ammonium sulphate. The skilled person would be aware that the supply of nucleotides in the form of a metal ion salt will affect the concentration of metal ions in the reaction mixture, and can take account of this additional source of metal ions if required. The total concentration of metal ions in the reaction mixture preferably includes all sources of metal ions.

In some embodiments, the amplification reaction further comprises one or more surfactants, which may be non-ionic surfactants. Exemplary surfactants include ionic surfactants, non-ionic surfactants, and combinations thereof. For example, the surfactant can be, without limitation, TRITON X-100 (chemical name Polyoxyethylene octyl phenyl ether), TWEEN 20 (also known as polysorbate 20, or its chemical name PEG(20)sorbitan monolaurate), TWEEN 80 (also known as polysorbate 80, or its chemical name polyoxyethylene sorbitan monooleate), sodium dodecylsulfate, sodium stearate, ammonium lauryl sulfate, TRITON AG 98 (Rhone-Poulenc), poloxamer 407, poloxamer 188 and the like, and combinations thereof.

The amplification reaction can also include one or more additional proteins. For example, the amplification reaction can comprise a single-stranded binding protein (SSBP). Exemplary single-stranded binding proteins include, for example, E. coli SSB, T4 gp32, T4 gene 32, ET SSB, T7 gene 2.5 SSB, phage phi29 SSB, any homologous protein or protein complex from any species, or a functional variant thereof.

The amplification reaction can also comprise a pyrophosphatase, such as Yeast Inorganic pyrophosphatase, e.g., *Saccharomyces cerevisiae* pyrophosphatase, available commercially from New England Biolabs.

The amplification reaction can also include agents that relax DNA and make template denaturation easier. Such agents include, for example, dimethyl sulfoxide (DMSO), formamide, glycerol and betaine. For example, the amplification reaction can comprise DMSO in an amount of from about 1% to about 7.5% (w/v or v/v) and/or betaine at a concentration of from about 0.5 M to about 1.5 M.

The amplification reaction can also include DNA condensing agents. Such agents include, for example, polyethylene glycol, cationic lipids and cationic polymers

It should be understood that the skilled person is able to modify and optimize amplification and incubation conditions for method described herein using these additional components and conditions on the basis of their general knowledge. Likewise, the specific concentrations of particular agents can be selected on the basis of previous examples in the art and further optimized on the basis of general knowledge. As an example, the amount of polymerase present in the reaction mixture can be optimized. This can involve making further addition of polymerase enzyme to the reaction mixture during the enzymatic DNA synthesis. As a further example, the amount of DNA template can be optimized. This can involve making further addition of DNA template to the reaction mixture during DNA synthesis. Further supply of polymerase enzyme and/or DNA template may be continuous or discontinuous, preferably discontinuous.

### Buffers

In the amplification reaction, contacting of the DNA template with the polymerase in the presence of the primers and nucleotides takes place under conditions promoting annealing of primers to the DNA template. The conditions include the presence of single-stranded DNA allowing for hybridization of the primers. The conditions also include a temperature and buffer allowing for annealing of the primers to the template. Appropriate annealing/hybridization conditions can be selected depending on the nature of the primer. An example of preferred annealing conditions includes a buffer. Exemplary buffers include, but are not limited to, the following: (i) 50 mM Tris HCl, pH 7.5, 10 mM MgCl₂, 20 mM (NH₄)₂SO₄, 5% glycerol, 0.2 mM BSA; (ii) 30 mM Tris-HCl pH 7.4, 30 mM KCI, 7.5 mM MgCl₂, 10 mM (NH₄)₂SO₄, 4 mM DTT; (iii) 30mM Tris-HCl pH 7.5, 20mM KCI, and 8mM MgCl₂; (iv) 50 mM Tris-HCl pH 7.5, 10 mM MgCl₂, 10 mM (NH₄)₂SO₄,); (v) 20 mM Tris-HCl pH 8.8, 10 mM (NH₄)₂SO₄, 10 mM KC1, 2 mM MgSO₄, 0.1% TRITON^{®} X-100; and (vi) 50 mM NaCl pH 7.9, 10 mM Tris-HCl, 10 mM MgCl₂, 1 mM DTT.

Other exemplary buffers that can be used in the methods provided herein include but are not limited to: Thermopol^{®} buffer; NEB^{®} buffers 1,2,3,4; CutSmart^{®} buffer; Isothermal Amplification Buffer; and the like. Custom buffers can be made with 0.5 to 2X PBS; 5 to 200 mM Tris-HCl; 5-200 mM Potassium Acetate; 5-200 mM Magnesium Acetate; 5-200 mM Tris-Acetate; or 5-200 mM Bis-Tris-Propane-HCl can be used with the addition one or all of these additives to modulate the enzyme activity (e.g., 1-50 mM KCI, 1-20 mM MgSO₄, 1-20 mM MgCl₂, 1-5 mM DTT, 0-500 ug/ml BSA, 1-500 NaCl, 0.01% to 0.5% Triton X-100 at pH values of 6-9.5).

In some preferred embodiments, the buffer for the amplification reaction is: (i) 7.5mM MgCl₂, 30mM KCI, 5mM (NH₄)₂SO₄, 12mM Tris-Base, and 17.6mM Tris-HCl; or (ii) 7.5mM MgCl₂, 30mM KCI, 5mM (NH₄)₂SO₄, 12mM Tris-Base, 17.6mM Tris-HCl, and 2mM DTT

### Synthesized DNA (product)

The DNA synthesized, i.e., the enzymatically synthesized DNA by the method described herein can be any DNA molecule. For example, the DNA synthesized according to the method described herein can be single stranded or double stranded. The synthesized DNA can be linear, or the synthesized DNA can be processed to form circles, such as minicircles, single stranded closed circles, double stranded closed circles, double stranded open circles, or closed linear double stranded DNA. The synthesized DNA can be allowed to form, or processed to form a particular secondary structure, such as, but not limited to hairpin loops (stem loops), imperfect hairpin loops, pseudoknots, or any one of the various types of double helix (A-DNA, B-DNA, or Z-DNA). In some embodiments, the DNA synthesized according to the method described herein is, or is allowed to form, or is processed to form no end DNA (neDNA), closed-ended linear duplex DNA (ceDNA), close ended linear duplexed DNA (c!DNA), an open circular dsDNA, a closed circular dsDNA, an open linear dsDNA, a closed linear dsDNA, or linear-covalently closed DNA.

In some embodiments, the DNA synthesized according to the methods described herein is, or is allowed to form, or is processed to form neDNA.

neDNA is a covalently-closed circular or linear DNA. Non-limiting examples of neDNA include doggy bone DNA and dumbbell-shaped DNA. Examples of doggy bone DNA or dumbbell-shaped DNA are described in U.S. Patent No. 6,451,563; U.S. Patent No. 9,109,250; U.S. Patent No. 9,499,847; U.S. Patent No. 10,501,782; PCT Publication No. WO 2018033730; PCT Publication No. WO 2019/246544; Yu et al. "Efficient production of superior dumbbell-shaped DNA minimal vectors for small hairpin RNA expression." Nucleic Acids Res. (2015), 43(18): e120; and H Kuhn, M D Frank-Kamenetskii & V V Demidov, "High-Purity Preparation of a Large DNA Dumbbell." Antisense Nucleic Acid Drug Development (2001) 11:149-153, contents of all of which are incorporated herein by reference in their entireties. neDNA also includes covalently closed circular DNA that lack bacterial sequences e.g., by formation of mini-circle DNA from plasmids as described in U.S. Patent No. 8,828,726, and U.S. Patent No. 7,897,380, contents of all which are incorporated herein by reference in their entireties.

Currently available techniques of synthesizing DNA by amplification can introduce mutations, e.g., insertion of one or more nucleotides in a homopolymer region in the synthesized DNA relative to the template DNA used. The inventors have discovered *inter alia* that the mutations, e.g., insertion of one or more nucleotides in a homopolymer region in the synthesized DNA can be reduced or prevented by initiating the amplification reaction with less than a full amount of primers and nucleotides, and adding further primers and nucleotides after the amplification reaction has started. Without wishing to be bound by a theory, less than about 5% of the synthesized DNA comprises a mutation, e.g., a nucleotide insertion relative to the sequence of the DNA template used. For example, less than about 4.5%, or less than about 4%, or less than about 3.5%, or less than about 3%, or less than about 2.5%, or less than about 2%, or about 1.5%, or less than about 1%, or less than about 0.5%, or less than about 0.4%, or less than about 0.3%, or less than about 0.2%, or less than about 0.1% or less (e.g., substantially none) of the synthesized DNA comprises a mutation, e.g., a nucleotide insertion relative to the sequence of the DNA template used. Stated in another way, a frequency of a mutation, e.g., insertion of a nucleotide in the synthesized DNA is less than about 5%. For example, a frequency of a mutation, e.g., insertion of a nucleotide in the synthesized DNA is less than about 4.5%, or less than about 4%, or less than about 3.5%, or less than about 3%, or less than about 2.5%, or less than about 2%, or about 1.5%, or less than about 1%, or less than about 0.5%, or less than about 0.4%, or less than about 0.3%, or less than about 0.2%, or less than about 0.1% or less (e.g., substantially none). In some cases, the synthesized DNA comprises substantially no mutations, e.g., one or more nucleotide insertions relative to the sequence of the DNA template used. Stated in another way the synthesized DNA comprises less than a detectable amount of a mutation, e.g., a nucleotide insertion relative to the sequence of the DNA template used.

It is noted that the DNA synthesized by the method described herein can be of any suitable length. Without wishing to be bound by a theory, the DNA synthesized by the method described herein can be up to or exceeding about 100 kilobases (kb or kbase). For example, the length of DNA synthesized can be in the order of up to about 90 kilobases, or up to about 80 kilobases, or up to about 70 kilobases, or up to about 60 kilobases, or up to about 50 kilobases, or up to about 40 kilobases, or up to about 30 kilobases, or up to about 2 kilobases.

In some embodiments, the DNA synthesized can be about 100 bases to about 100 kilobases, 200 bases to 90 kilobases, or about 200 bases to about 50 kilobases, or about 300 bases to about 25 kilobases, or about 400 bases to about 10 kilobases, about 1 kilobases to about 50 kilobases, about 1.5 kilobases to about 25 kilobases. In some embodiments, the DNA synthesized can be about 200 bases to 15 kilobases. In some preferred embodiments, the DNA synthesized according to the method described herein can be about 2 kilobases to about 15 kilobases.

Without wishing to be bound by a theory, the amount of DNA synthesized can be described as industrial or commercial quantities, on a large-scale, or mass production. The DNA produced by the processes can be uniform in quality, namely in DNA length and sequence. Accordingly, the method described herein can be suitable for large scale synthesis of DNA. For example, the amount of DNA synthesized according to the method described herein can exceed about 10g/l of the amplification reaction. The amount of DNA synthesized using the method described herein can be up to about 10 g/l of the amplification reaction. For example, the amount of DNA synthesized can be from about 0.1g/l to about 9 g/l, or about 0.2 g/l to about 8 g/l, or about 0.3 g/l to about 7 g/l, or about 0.4g/l to about 6 g/l, or about 0.5 g/l to about 5 g/l. The amount of DNA synthesized using the method described herein can be up to about 9g/l, up to about 8.5 g/l, up to about 8 g/l, up to about 7.5 g/l, up to about 7 g/l, up to about 6.5 g/l, up to about 6 g/l, up to about 5.5 g/l, up to about 5 g/l, up to about 4.5 g/l, up to about 4 g/l, up to about 3.5 g/l, up to about 3 g/l, up to about 2.5 g/l, up to about 2 g/l, up to about 1.5 g/l, up to about 1 g/l, up to about 0.9 g/l, up to about 0.8 g/l, up to about 0.7 g/l, up to about 0.6 g/l, up to about 0.5 g/l up to about 0.4 g/l, up to about 0.3 g/l, up to about 0.2 g/l, or up to about 0.1 g/l.

In some embodiments, the amount of DNA synthesized is from about 0.2 g/l to about 5 g/l of the reaction. For example, the amount of DNA synthesized according to the method described herein is from about 1 g/l to about to 5 g/l of the amplification reaction.

Some aspects of method described herein can also be defined according to any of the following numbered embodiments:
Embodiment 1: A method for synthesizing DNA, the method comprising amplification of a DNA template, wherein the DNA template is contacted with a DNA polymerase in the presence of a sufficient portion of a total amount of nucleotides to be added to the reaction, and a sufficient portion of a total amount of one or more primers to be added to form an initial amplification reaction, and wherein at least one subsequent sufficient portion of the total primers and at least one subsequent sufficient portions of the total nucleotides are added to the initial amplification reaction continuously or at regular or irregular intervals at least about 30 minutes after start of the amplification reaction, wherein the sufficient portion is an amount sufficient to carry out the amplification reaction.
Embodiment 2: The method of Embodiment 1, wherein the subsequent portion of the primers is added at the same time as the subsequent portion of the nucleotides.
Embodiment 3: The method of Embodiment 1, wherein the subsequent portion of the primers is added at different times than the subsequent portion of the nucleotides.
Embodiment 4: The method of any one of Embodiments 1-3, wherein a plurality of subsequent portions of the primers are added to the amplification reaction.
Embodiment 5: The method of any one of Embodiments 1-4, wherein a plurality of subsequent portions of the nucleotides are added to the amplification reaction.
Embodiment 6: The method of any one of Embodiments 1-5, wherein the DNA template comprises at least one repeat region.
Embodiment 7: The method of any one of Embodiments 1-6, wherein the amplification is rolling circle amplification (RCA).
Embodiment 8: The method of Embodiment 6 or 7, wherein the repeat region is at least 5 (e.g., 6, 7, 8, 9, 10 or more) nucleotides in length.
Embodiment 9: The method of any one of Embodiments 6-8, wherein the repeat region comprises nucleotides that are the same.
Embodiment 10: The method of any one of Embodiments 6-9, wherein the repeat region comprises a dinucleotide repeat.
Embodiment 11: The method of any one of Embodiments 6-9, wherein the repeat region comprises a trinucleotide repeat.
Embodiment 12: The method of any one of Embodiments 1-11, wherein the DNA polymerase is Phi29 polymerase, Bst Polymerase, or a mutant or variant thereof, preferably the DNA polymerase is Phi29.
Embodiment 13: The method of any one of Embodiments 1-12, wherein the amplification is RCA, the DNA polymerase is Phi29, a plurality of subsequent portions of primers and a plurality of subsequent portions nucleotides are added to the amplification reaction continuously starting at time zero or at regular or irregular intervals at least about 45 minutes after start of the amplification reaction, and the DNA template comprises at least one repeat region of at least 5 nucleotides.
Embodiment 14: The method of any one of Embodiments 1-13, wherein less than about 5% (e.g., about 4.5%, about 4%, about 3.5%, about 3%, about 2.5%, about 2%, about 1.5%, about 1%, about 0.5%, about 0.4%, about 0.3%, about 0.2%, about 0.1% or less e.g., substantially none) of the amplified product comprises a nucleotide insertion in the sequence of the DNA template.
Embodiment 15: The method of any one of Embodiments 1-14, wherein the subsequent portions of the primers are added to the amplification reaction at regular or irregular intervals.
Embodiment 16: The method of any one of Embodiments 1-15, wherein the subsequent portions of the primers are added to the amplification reaction continuously or at intervals of at least about 30 minutes.
Embodiment 17: The method of any one of Embodiments 1-16, wherein the subsequent portions of the primers are added to the amplification reaction at intervals of from about 45 minutes to about 75 minutes, optionally, the subsequent portions of the primers are added to the amplification reaction at intervals of about 60 minutes).
Embodiment 18: The method of any one of Embodiments 1-17, wherein at least 2 (e.g., 3, 4, 5, 6, 7, 8, 9, 10, 11, 12, 13, 14, 15, 16, 17, 18, 19, 20, 21, 22, 23, 24, 25, 26, 27, 28, 29, 30 or more) subsequent portions of the primers are added to the amplification reaction, optionally, 7 subsequent portions of the primers are added are added to the initial amplification reaction.
Embodiment 19: The method of any one of Embodiments 1-18, wherein each subsequent portion of the primers independently comprises an amount that is equal or less than the amount of the primers in the initial amplification reaction.
Embodiment 20: The method of any one of Embodiments 1-19, wherein each subsequent portion of the primers independently comprises equal or less than about 100% (weight or mol) (e.g., about 100%, about 95%, about 90%, about 80%, about 85%, about 72%, about 70%, about 65%, about 60%, about 55%, about 50%, about 45%, about 40%, about 35%, about 30%, about 25%, about 20%, about 15%, about 10%, about 5% or lower) of the total amount of the primers in the initial amplification reaction.
Embodiment 21: The method of any one of Embodiments 1-20, wherein each subsequent portion of the primers independently comprises from about 5% to about 20% (weight or mol) (e.g., from about 7.5% to about 30%, or from about 10% to about 25%, or from about 10% to about 15%, optionally about 12.5%) of the total amount of the primers added to the initial amplification reaction.
Embodiment 22: The method of any one of Embodiments 1-21, wherein each subsequent portion of the primers is independently at a concentration of from about 1 µM to about 10 µM, e.g., about 1.0 µM, about 1.5 µM, about 2.0 µM, about 2.5 µM, about 3.0 µM, about 3.5 µM, about 4.0 µM, about 5.0 µM, about 5.5 µM, about 6 µM, about 6.5 µM, about 7 µM, about 7.5 µM, about 8 µM, about 8.5 µM, about 9 µM, or about 9.5 µM, optionally each subsequent portion of the primers is about 7 µM .
Embodiment 23: The method of any one of Embodiments 1-22, wherein the initial amplification reaction comprises less than about 50% (weight or mol) (e.g., 49%, about 48%, about 47%, about 46%, about 45%, about 44%, about 43%, about 42%, about 41%, about 40%, 39%, about 38%, about 37%, about 36%, about 35%, about 34%, about 33%, about 32%, about 31%, about 30%, 29%, about 28%, about 27%, about 26%, about 25%, about 24%, about 23%, about 22%, about 21%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2% or less) of the total amount of the primers to be added to the initial amplification reaction, optionally, the initial amplification reaction comprises about 12.5% of the total amount of the primers to be added to the initial amplification reaction.
Embodiment 24: The method any one of Embodiments 1-23, wherein a total concentration of the primers added to the amplification reaction is from about 5 µM to about 70 µM, e.g., about 5 µM, about 6 µM, about 7 µM, about 8 µM, about 9 µM, about 10 µM, about 15 µM, about 20 µM, about 25 µM, about 30 µM, about 35 µM, about 40 µM, about 45 µM, about 50 µM, about 55 µM, about 60 µM, about 65 µM, or about 70 µM, optionally, the a total concentration of the primers added to the amplification reaction is about 56.4 µM.
Embodiment 25: The method of any one of Embodiments 1-24, wherein the subsequent portions of the nucleotides are added to the amplification reaction at regular or irregular intervals.
Embodiment 26: The method of any one of Embodiments 1-25, wherein the subsequent portions of the nucleotides are added to the amplification reaction continuously or at intervals of at least about 30 minutes.
Embodiment 27: The method of any one of Embodiments 1-26, wherein the subsequent portions of the nucleotides are added to the amplification reaction continuously or at intervals of from about 1 hours to about 3 hours, optionally, wherein the subsequent portions of the nucleotides are added to the amplification reaction at intervals of from about 1 hours to about 2 hours).
Embodiment 28: The method of any one of Embodiments 1-27, wherein at least 2 (e.g., 3, 4, 5, 6, 7, 8, 9, 10, 11, 12, 13, 14, 15, 16, 17, 18, 19, 20, 21, 22, 23, 24, 25, 26, 27, 28, 29, 30 or more) subsequent portions of the nucleotides are added to the amplification reaction, optionally 7 subsequent portions of the nucleotides are added to the amplification reaction.
Embodiment 29: The method of any one of Embodiments 1-28, wherein each subsequent portion of the nucleotides independently comprises an amount that is equal or less than the amount of the nucleotides in the initial amplification reaction.
Embodiment 30: The method of any one of Embodiments 1-29, wherein each subsequent portion of the nucleotides independently comprises less than about 75% (weight or mol) (e.g., about 70%, about 65%, about 60%, about 55%, about 50%, about 45%, about 40%, about 35%, about 30%, about 25%, or lower) of the total amount of the nucleotides in the initial amplification reaction.
Embodiment 31: The method of any one of Embodiments 1-31, wherein each subsequent portion of the nucleotides independently comprises from about 3% to about 30% (weight or mol) (e.g., from about 10% to about 25% or from about 15% to about 20%, e.g., about 10%, about 11%, about 12%, about 13%, about 14%, about 15%, about 16%, about 17%, about 18%, about 19%, about 20%, about 21%, about 22%, about 23%, about 24%, or about 25% (mol% or weight%) of the total amount of the nucleotides added to the initial amplification reaction.
Embodiment 32: The method of any one of Embodiments 1-32, wherein each subsequent portion of the nucleotides is independently at a concentration of from about 0.001 mM to about 0.3 mM (e.g., from about 0.005 mM to about 0.125 mM, from about 0.01 mM to 0.1 mM, or from about 0.05 to about 0.09 mM (e.g., about 0.01 mM, about 0.02 mM, about 0.03 mM, about 0.04 mM, about 0.05 mM, about 0.06 mM, about 0.07 mM, about 0.08 mM, about 0.09 mM, about 0.1 mM, about 0.11 mM, about 0.12 mM, about 0.13 mM, about 0.14 mM, about 0.15 mM, about 0.16 mM, about 0.17 mM, about 0.18 mM, about 0.19 mM, about 0.20 mM, about 0.21 mM, about 0.22 mM, about 0.23 mM, about 0.24 mM, about 0.25 mM, about 0.26 mM, about 0.27 mM, about 0.28 mM, about 0.29 mM, or about 0.3 mM), optionally about 0.13 mM or about 0.25 mM.
Embodiment 33: The method of any one of Embodiments 1-32, wherein the initial amplification reaction comprises less than about 30% (mol%) (e.g., about 29%, about 28%, about 27%, about 26%, about 25%, about 24%, about 23%, about 22%, about 21%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1% or less) of the total amount of the nucleotides added to the initial amplification reaction.
Embodiment 34: The method any one of Embodiments 1-33, wherein a total concentration of the nucleotides added to the amplification reaction is from about 0.1 mM to about 6 mM, e.g., from about 0.25 mM to about 5.5 mM, from about 0.3 mM to about 5 mM or from about 0.25 mM to about 4.5 mM, e.g., about 0.1 mM, about 0.15 mM, about 0.2 mM, about 0.25 mM, about 0.3 mM, about 0.35 mM, about 0.4 mM, about 0.45 mM, about 0.5 mM, about 0.55 mM, about 0.6 mM, about 0.65 mM, about 0.7 mM, about 0.75 mM, about 0.8 mM, about 0.85 mM, about 0.9 mM, about 0.95 mM, about 1 mM, about 1.25 mM, about 1.5 mM, about 1.75 mM, or about 1.25 mM, or about 3 mM, , or about 3.25 mM, or about 3.5 mM, or about 3.75 mM, or about 4.25 mM, or about 4.5 mM, or about 4.75 mM, or about 5 mM, optionally about 0.5 mM, or about 0.77 mM, or about 4 mM, preferably about 4 mM
Embodiment 35: The method of any one of Embodiments 1-34, wherein each subsequent portion of the nucleotides is added after at least of about 40% (e.g., about 45%, about 50%, about 55%, about 60%, about 65%, about 70%, about 75%, about 80%, about 85%, or more) of the nucleotides in the amplification reaction have been used.
Embodiment 36: The method of any one of Embodiments 1-35, wherein a molar ratio of A to G, A to C, T to G, or T to C in the nucleotides added to the reaction mixture is higher than 1 (e.g., about 1.1, about 1.2, about 1.3, about 1.4, about 1.5, about 1.6, about 1.7, about 1.8, about 1.9, about 2, about 2.1, about 2.2, about 2.3, about 2.4, about 2.5, about 2.6, about 2.7, about 2.8, about 2.9, about 3 or more).
Embodiment 37: The method of any one of Embodiments 6-36, wherein the repeat region is at least 6 repeat units in length.
Embodiment 38: The method of any one of Embodiments 6-37, wherein the repeat region is at least 7 repeat units in length.
Embodiment 39: The method of any one of Embodiments 6-38, wherein the repeat region is at least 8 repeat units in length.
Embodiment 40: The method of any one of Embodiments 6-39, wherein the repeat region is at least 9 repeat units in length.
Embodiment 41: The method of any one of Embodiments 6-40, wherein the repeat region is at least 10 repeat units in length.
Embodiment 42: The method of any one of Embodiments 6-41, wherein the repeat region is at least 11 repeat units in length.
Embodiment 43: The method of any one of Embodiments 6-42, wherein the repeat region is at least 12 repeat units in length.
Embodiment 44: The method of any one of Embodiments 6-43, wherein the repeat region is at least 13 repeat units in length.
Embodiment 45: The method of any one of Embodiments 6-44, wherein the repeat region comprises C.
Embodiment 46: The method of any one of Embodiments 6-44, wherein the repeat region comprises G.
Embodiment 47: The method of any one of Embodiments 6-44, wherein the repeat region comprises A.
Embodiment 48: The method of any one of Embodiments 6-44, wherein the repeat region comprises T.
Embodiment 49: The method of any one of Embodiments 6-44, wherein the repeat region comprises GC or CG dinucleotide.
Embodiment 50: The method of any one of Embodiments 1-49, wherein the DNA template is double-stranded DNA (dsDNA).
Embodiment 51: The method of any one of Embodiments 1-50, wherein the DNA template is an open circular dsDNA, a closed circular dsDNA, an open linear dsDNA or a closed linear dsDNA
Embodiment 52: The method of any one of Embodiments 1-51, wherein the DNA template is single-stranded DNA (ssDNA).
Embodiment 53: The method of Embodiment 52, wherein the DNA template is a linear ssDNA or a closed circular ssDNA.
Embodiment 54: The method of any one of Embodiments 1-53, wherein the DNA template is from about 500 bases to about 100 kilobases, e.g., from about 1 kilobases to about 50 kilobases, from about 1.5 kilobases to about 25 kilobases, or from about 2 kilobases to about 20 kilobases.
Embodiment 55: The method of any one of Embodiments 1-54, wherein the DNA template comprises at least one processing enzyme target sequence.
Embodiment 56: The method of Embodiment 55, wherein said at least one processing enzyme target sequence comprises a recombinase targeting sequence or a protelomerase targeting sequence.
Embodiment 57: The method of Embodiment 56, wherein the method further comprises supplying a processing enzyme to the reaction mixture.
Embodiment 58: The method of Embodiment 57, wherein the processing enzyme is supplied at regular or irregular intervals during the amplification reaction.
Embodiment 59: The method of any one of Embodiments 1-6 or 8-58, wherein the amplification is isothermal amplification.
Embodiment 60: The method of acEmbodiment 59, wherein the amplification is Rolling Circle Amplification (RCA), Loop Mediated Isothermal Amplification (LAMP), Recombinase Polymerase Amplification (RPA), Helicase-dependent isothermal DNA amplification (HDA), Nucleic acid sequence-based amplification (NASBA), strand displacement amplification (SDA), nicking enzyme amplification reaction (NEAR), polymerase Spiral Reaction (PSR), Hybridization Chain Reaction (HCR), Primer Exchange Reaction (PER), Signal Amplification by Exchange Reaction (SABER), transcription-based amplification system (TAS), Self-sustained sequence replication reaction (3SR), Single primer isothermal amplification (SPIA), or cross-priming amplification (CPA).
Embodiment 61: The method of any one of Embodiments 1-60, wherein the amplification reaction further comprises a DNA polymerase stabilizing agent..
Embodiment 62: The method of Embodiment 61, wherein the DNA polymerase stabilizing agent is a reducing agent.
Embodiment 63: The method of any one of Embodiments 1-62, wherein the amplification reaction comprises a reducing agent, optionally the reducing agent is dithiothreitol (DTT), 1,2-propanediol, ethylene glycol, ethanol, isopropanol, glycerol, Trehalose, and acetyl cysteine, preferably the reducing agent is DTT.
Embodiment 64: The method of any one of Embodiments 1-63, wherein the amplification reaction further comprises dithiothreitol (DTT).
Embodiment 65: The method of any one of Embodiments 1-64, wherein the amplification reaction comprises dithiothreitol (DTT) at a concentration from about 1 mM to about 3 mM, e.g., from about 1.5 mM to 2.5 mM or from about 1.75 mM to about 2.25 mM, e.g., about 1.5 mM, about 1.55 mM, about 1.6 mM, about 1.65 mM, about 1.7 mM, about 1.75 mM, about 1.8 mM, about 1.85 mM, about 1.9 mM, about 1.95 mM, about 2 mM, about 2.05 mM, about 2.1 mM, about 2.15 mM, about 2.2 mM, about 2.25 mM, about 2.3 mM, about 2.35 mM, about 2.4 mM, about 2.45 mM, or about 2.5 mM, preferably about 2 mM.
Embodiment 66: The method of any one of Embodiments 1-65, wherein the amplification product is no end DNA (neDNA), closed-ended linear duplex DNA (ceDNA), close ended linear duplexed DNA (clDNA), or linear-covalently closed DNA.
Embodiment 67: The method of any one of Embodiments 1-66, wherein the amplification product is neDNA.
Embodiment 68: The method of any one of Embodiments 1-67, wherein the method is a cell-free method.

### Definitions

For convenience, certain terms employed herein, in the specification, examples and appended claims are collected herein. Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments, and are not intended to limit the claimed invention, because the scope of the invention is limited only by the claims. Further, unless otherwise required by context, singular terms shall include pluralities and plural terms shall include the singular.

Unless defined otherwise, all technical and scientific terms used herein have the same meaning as those commonly understood to one of ordinary skill in the art to which this invention pertains. Although any known methods, devices, and materials may be used in the practice or testing of the invention, the methods, devices, and materials in this regard are described herein. Definitions of common terms in immunology and molecular biology can be found in The Merck Manual of Diagnosis and Therapy, 20th Edition, published by Merck Sharp & Dohme Corp., 2018 (ISBN 0911910190, 978-0911910421); Robert S. Porter et al. (eds.), The Encyclopedia of Molecular Cell Biology and Molecular Medicine, published by Blackwell Science Ltd., 1999-2012 (ISBN 9783527600908); and Robert A. Meyers (ed.), Molecular Biology and Biotechnology: a Comprehensive Desk Reference, published by VCH Publishers, Inc., 1995 (ISBN 1-56081-569-8); Immunology by Werner Luttmann, published by Elsevier, 2006; Janeway's Immunobiology, Kenneth Murphy, Allan Mowat, Casey Weaver (eds.), W. W. Norton & Company, 2016 (ISBN 0815345054, 978-0815345053); Lewin's Genes XI, published by Jones & Bartlett Publishers, 2014 (ISBN-1449659055); Michael Richard Green and Joseph Sambrook, Molecular Cloning: A Laboratory Manual, 4th ed., Cold Spring Harbor Laboratory Press, Cold Spring Harbor, N.Y., USA (2012) (ISBN 1936113414); Davis et al., Basic Methods in Molecular Biology, Elsevier Science Publishing, Inc., New York, USA (2012) (ISBN 044460149X); Laboratory Methods in Enzymology: DNA, Jon Lorsch (ed.) Elsevier, 2013 (ISBN 0124199542); Current Protocols in Molecular Biology (CPMB), Frederick M. Ausubel (ed.), John Wiley and Sons, 2014 (ISBN 047150338X, 9780471503385), Current Protocols in Protein Science (CPPS), John E. Coligan (ed.), John Wiley and Sons, Inc., 2005; and Current Protocols in Immunology (CPI) (John E. Coligan, ADA M Kruisbeek, David H Margulies, Ethan M Shevach, Warren Strobe, (eds.) John Wiley and Sons, Inc., 2003 (ISBN 0471142735, 9780471142737), the contents of which are all incorporated by reference herein in their entireties.

Further, the practice of the present invention can employ, unless otherwise indicated, conventional techniques of molecular biology (including recombinant techniques), microbiology, cell biology, biochemistry, and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Current Protocols in Molecular Biology" (F. M. Ausubel et al., eds., 1987, and periodic updates); "PCR: The Polymerase Chain Reaction", (Mullis et al., ed., 1994); "A Practical Guide to Molecular Cloning" (Perbal Bernard V., 1988); "Phage Display: A Laboratory Manual" (Barbas et al., 2001).

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Other than in the operating examples, or where otherwise indicated, all numbers expressing quantities of ingredients or reaction conditions used herein should be understood as modified in all instances by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number. For example, the term "about" when used in connection with percentages can mean ±5%, e.g., ±4.5%, ±4%, ±3.5%, ±3%, ±2.5%, ±2%, or ±1.5%, preferably ±1%.

As used herein the term "comprising" or "comprises" is used in reference to compositions, methods, and respective component(s) thereof, that are essential to the invention, yet open to the inclusion of unspecified elements, whether essential or not.

As used herein the term "consisting essentially of" refers to those elements required for a given embodiment. The term permits the presence of additional elements that do not materially affect the basic and novel or functional characteristic(s) of that embodiment of the invention.

The term "consisting of" refers to compositions, methods, and respective components thereof as described herein, which are exclusive of any element not recited in that description of the embodiment.

The singular terms "a," "an," and "the" include plural referents unless context clearly indicates otherwise. Similarly, the word "or" is intended to include "and" unless the context clearly indicates otherwise. It is further noted that the claims can be drafted to exclude any optional element. As such, this statement is intended to serve as antecedent basis for use of such exclusive terminology as "solely," "only" and the like in connection with the recitation of claim elements, or use of a "negative" limitation.

The abbreviation, "e.g." is derived from the Latin exempli gratia, and is used herein to indicate a non-limiting example. Thus, the abbreviation "e.g." is synonymous with the term "for example."

The term "statistically significant" or "significantly" refers to statistical significance and generally means a two standard deviation (2SD) or greater difference.

It should be understood that this disclosure is not limited to the particular methodology, protocols, and reagents, etc., provided herein and as such may vary. The terminology used herein is for the purpose of describing particular embodiments only, and is not intended to limit the scope of the present disclosure, which is defined solely by the claims. The invention is further illustrated by the following example, which should not be construed as further limiting.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

The description of embodiments of the disclosure is not intended to be exhaustive or to limit the disclosure to the precise form disclosed. While specific embodiments of, and examples for, the disclosure are described herein for illustrative purposes, various equivalent modifications are possible within the scope of the disclosure, as those skilled in the relevant art will recognize. For example, while method steps or functions are presented in a given order, alternative embodiments may perform functions in a different order, or functions may be performed substantially concurrently. The teachings of the disclosure provided herein can be applied to other procedures or methods as appropriate. The various embodiments described herein can be combined to provide further embodiments. Aspects of the disclosure can be modified, if necessary, to employ the compositions, functions and concepts of the above references and application to provide yet further embodiments of the disclosure. Moreover, due to biological functional equivalency considerations, some changes can be made in protein structure without affecting the biological or chemical action in kind or amount. These and other changes can be made to the disclosure in light of the detailed description. All such modifications are intended to be included within the scope of the appended claims.

Specific elements of any of the foregoing embodiments can be combined or substituted for elements in other embodiments. Furthermore, while advantages associated with certain embodiments of the disclosure have been described in the context of these embodiments, other embodiments may also exhibit such advantages, and not all embodiments need necessarily exhibit such advantages to fall within the scope of the disclosure.

### EXAMPLES

The technology described herein is further illustrated by the following examples which in no way should be construed as being further limiting.

### MATERIAL AND METHODS

The phi29 polymerase produces long linear repeats of circular template (concatemer), which can be processed with restriction enzymes. For all experiments described in this patent, reactions were incubated at 30°C during different time points indicated in each experiment, however, most of the time for over 28hours.

The creation of concatemeric DNA by phi29 polymerase leads to an increase in reaction viscosity due to the long length of the DNA chains in the RCA. Processing of the concatemeric DNA with restriction enzyme leads to a decrease in viscosity. After restriction enzyme processing, reactions were evaluated by agarose gel electrophoresis and yields were estimated using QuantiFluor^{®} dsDNA (Promega) or gel densitometry of the band(s) of interest using GeneTools program (SynGene). The digested reaction products were then purified using Invitrogen^{™} PureLink^{™} Quick Gel Extraction and PCR Purification Combo Kit (Catalog number: K220001) following manufacturer's instructions. Purified samples were sent for Next Generation Sequencing (NGS) in an Illumina based system (Secugen, Madrid, Spain). Reported data has a minimum coverage of 1000x.

**Table 1** displays the reagents used for the different examples. The experimental work shows evidence that the new variables and processes discovered to reduce or prevent the appearance of insertions in one or more of the homopolymer regions present in construct when phi29 is used for Rolling Cycle Amplification (RCA).

| **Table 1: Reagents used in the examples** | |
|---|---|
| Reagents | Supplier |
| Nuclease-free water | Merck Life Sciences |
| Plasmid template: construct 1 (see Figure 1A), construct 2 (see Figure 1B), construct 3 (see Figure 1C) and construct 4 (see Figure 1D) | Produced in-house |
| 5M Sodium hydroxide | Merck Life Sciences |
| Tris-Base | Merck Life Sciences |
| Tris-HCl | VWR International |
| Ammonium sulphate | Merck Life Sciences |
| Magnesium chloride hexahydrate | Merck Life Sciences |
| Potassium chloride | Merck Life Sciences |
| 100mM dNTPs | Thermo Fisher Scientific |
| 100mM dNTPs kit (dATP, dCTP, dGTP, dTTP) | Life Technologies |
| DNA primer | Eurogentec |
| 2000U/mL Thermostable pyrophosphatase | Qiagen Beverly |
| 250000U/mL Phi29 DNA polymerase | New England Biolabs |
| 100000U/mL BamHl | New England Biolabs |

### Example 1:

In order to study the appearance of mutations in homopolymer regions, a set of experiments were performed to develop two representative scale-down models of RCA (1mL scale and 20mL scale). The importance of scale-down models lies in their ability to provide insights into the behavior and performance of complex systems without the expense and risk of running full-scale productions. The 1mL scale model is representative of larger GMP enzymatic DNA manufacturing scales except for the temperature ramps, mixing conditions and material in contact with the product due to the use of a recipient with different shape and material. The 20mL model uses 50mL bioprocessing bags (Flexboy^{®}, Sartorius) of the same characteristics as the ones used in the larger manufacturing scales of enzymatic DNA. Thus, the mixing conditions and temperature ramps and other parameters can better match the larger scales. **Table 2 and 3** show the experimental protocols. Experiments and analyses were run in triplicate (A-C for each model).

| Table 2: RCA reaction components at 1 mL scale | | | |
|---|---|---|---|
| **RCA reaction 1mL scale** | | | |
| **Reagents** | **Stock Conc.** | **Final Conc.** | **Volume (µL)** |
| Water | - | - | 46.8 |
| Plasmid template: construct 1 | 0.9 mg/mL | 2.0µg/mL | 2.2 |
| NaOH | 5.0M | 5.0mM | 1.0 |
| Buffer: 30mM Tris, 30mM KCl, 7.5mM MgCl₂, 5mM (NH₄)₂SO₄ | - | - | 926.1 |
| dNTPs | 100mM | 2.0mM | 20.0 |
| Primer | Variable | 0.008µM | Variable |
| Pyrorophosphatase | 2000U/mL | 0.6U/mL | 0.3 |
| Phi29 polymerase | 250000U/mL | 500U/mL | 2.0 |

| **Feed** | | | |
|---|---|---|---|
| dNTP | 100mM | 2.0mM | 20.0 |
| Primer | Variable | 56.4µM | Variable |

| **Restriction enzyme** | | | |
|---|---|---|---|
| BamHI | 100000U/mL | 300U/mL | 3.0 |

| **Table 3: RCA reaction components at 20 mL scale** | | | |
|---|---|---|---|
| **RCA reaction 20mL scale** | | | |
| **Name** | **Stock Conc.** | **Final Conc.** | **Volume** |
| Water | - | - | 936.5µL |
| Plasmid template: construct 1 | 0.9 mg/mL | 2.0µg/mL | 43.5µL |
| NaOH | 5.00M | 5.0mM | 20.0µL |
| Buffer: 30mM Tris, 30mM KCl, 7.5mM MgCl₂, 5mM (NH₄)₂SO₄ | - | - | 18.5mL |
| dNTPs | 100mM | 2.0mM | 400.0µL |
| Primer | Variable | 0.008µM | Variable |
| Pyrophosphatase | 2000U/mL | 0.6U/mL | 6.0µL |
| Phi29 polymerase | 250000U/mL | 500U/mL | 40.0µL |

| **Feed** | | | |
|---|---|---|---|
| dNTPs | 100mM | 2.0mM | 400.0 |
| Primer | Variable | 56.4µM | Variable |

| **Restriction enzyme** | | | |
|---|---|---|---|
| BamHI | 100000U/mL | 300U/mL | 60.0 |

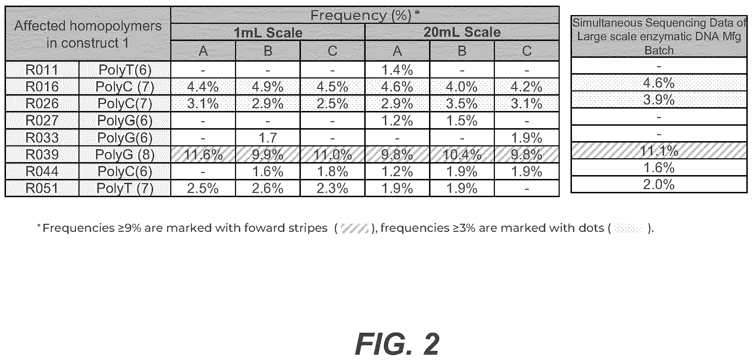

Both the 1mL scale and the 20mL scale are representative scale-down models of the large manufacturing scale in terms of insertion frequencies in homopolymer regions. See, **Figure 2****.**

### EXAMPLE 2

In order to investigate whether different proportions of dNTPs (A-T-G-C) could reduce the insertions in homopolymer regions, a set of experiments were performed following the experimental protocol showed in **¡Error! No se encuentra el origen de la referencia.** but changing the different A-T, G-C proportions as summarized in **Table 4.**

| **Table 4:** Summary of the conditions to evaluate the frequency of insertions at different dNTPs proportions | | | | |
|---|---|---|---|---|
| Condition | Reaction start (total: 2mM dNTPs) | | Feed (total: 2mM dNTPs) | |
| | A-T Conc. (%) | G-C Conc. (%) | A-T Conc. (%) | G-C Conc. (%) |
| A | 0.5mM dATP | 0.5mM dGTP | 0.5mM dATP | 0.5mM dGTP |
| | 0.5mM dTTP (50%) | 0.5mM dCTP (50%) | 0.5mM dTTP (50%) | 0.5mM dCTP (50%) |
| B | 0.7mM dATP | 0.3mM dGTP | 0.7mM dATP | 0.3mM dGTP |
| | 0.7mM dTTP (70%) | 0.3mM dCTP (30%) | 0.7mM dTTP (70%) | 0.3mM dCTP (30%) |
| C | 0.3mM dATP | 0.7mM dGTP | 0.3mM dATP | 0.7mM dGTP |
| | 0.3mM dTTP (30%) | 0.7mM dCTP (70%) | 0.3mM dTTP (30%) | 0.7mM dCTP (70%) |

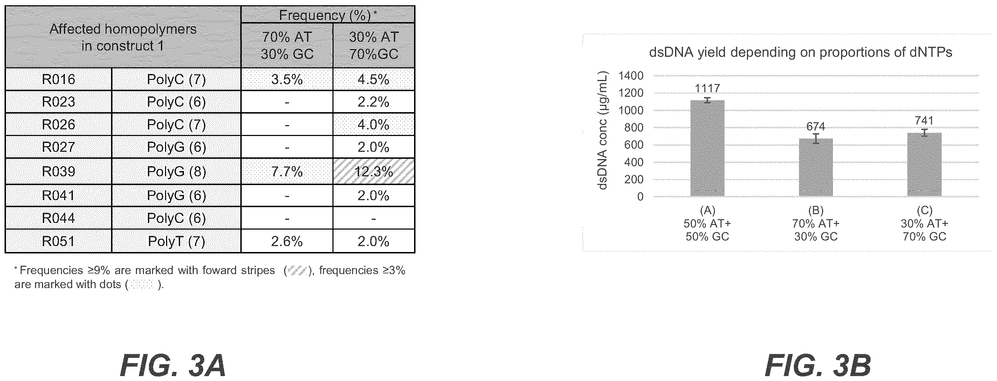

Three groups of experiments were conducted containing concentrations of 50:50 AT:GC (A), 70:30 AT:GC (B) and 30:70 AT:GC (C). DNA processing and quantification was performed as described in the introduction. Results are shown in **Figures 3A and 3B****.** As seen, lower insertions are obtained when low GC content is used, while varying AT concentration apparently does not impact insertions. However, when dNTPs are imbalanced, enzymatic DNA yield is reduced significantly.

### EXAMPLE 3

This experiment was carried out using a three-level full factorial design using the MODDE software to demonstrate the effect of different dNTPs and/or magnesium concentration on insertion frequencies.

| **Table 5:** Summary of the experiments proposed by MODDE^{®} to perform a three level Full Factorial Model to evaluate the fidelity of phi29 at different dNTPs and magnesium concentrations | | |
|---|---|---|
| Experiment number | Factors | |
| | dNTPs conc (mM) | Mg²⁺ conc (mM) |
| N7 | 2 | 10 |
| N4 | 2 | 7 |
| N1 | 2 | 4 |
| N13 | 2 | 4 |
| N15 | 2 | 4 |
| N8 | 4 | 10 |
| N5 | 4 | 7 |
| N10 | 4 | 7 |
| N11 | 4 | 7 |
| N2 | 4 | 4 |
| N9 | 6 | 10 |
| N12 | 6 | 10 |
| N14 | 6 | 10 |
| N6 | 6 | 7 |
| N3 | 6 | 7 |

The reactions were set up following **Table 2,** but changing the dNTPs and Mg²⁺ concentrations as indicated in **Table 5.**

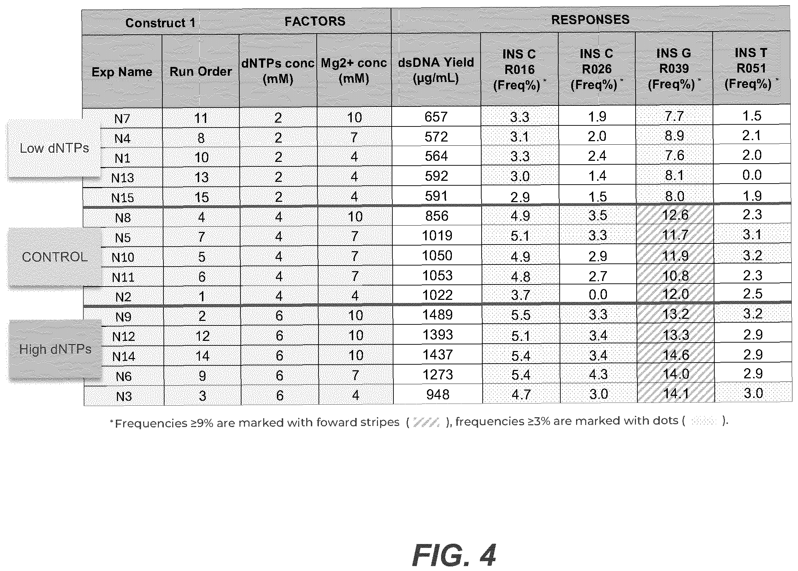

**Figure 4** shows that lower insertion frequencies are obtained when dNTPs concentration is lowered. Importantly, as expected since the dNTPs are the building blocks of the DNA chains, dsDNA yield is also reduced. No or little effect is observed in dsDNA yield or insertion frequencies when magnesium concentration is changed.

### EXAMPLE 4

In order to investigate whether lower dNTPs concentrations and/or different dNTPs and primer feeding conditions could reduce the insertions in homopolymer regions, a set of experiments were performed following the experimental protocol showed in **Table 2** but changing the dNTPs and primer conditions as summarized in **Table 6.**

| **Table 6:** Summary of dNTPs and primer conditions to evaluate the frequency of insertions | | | |
|---|---|---|---|
| Condition | Primer Addition | dNTPs Addition | Final dNTPs concentration (mM) |
| A | t=0h (0.014%) | t=0, 7h (50%) | 2 |
| | t=7h (99.99%) | | |
| B | t=0h (0.014%) | t=0, 7h (50%) | 1 |
| | t=7h (99.99%) | | |
| C | t=0h (100%) | t=0, 7h (50%) | 4 |
| D | t=0h (100%) | t=0, 7h (50%) | 2 |
| E | t=0h (100%) | t=0, 7h (50%) | 1 |
| F | t=0h (100%) | t=0,1,2,3,4,5,6,7h (12.5%) | 4 |
| G | t=0h (100%) | t=0,1,2,3,4,5,6,7h (12.5%) | 2 |
| H | t=0,1,2,3,4,5,6,7h (12.5%) | t=0,1,2,3,4,5,6,7h (12.5%) | 4 |
| I | t=0,1,2,3,4,5,6,7h (12.5%) | t=0,1,2,3,4,5,6,7h (12.5%) | 2 |

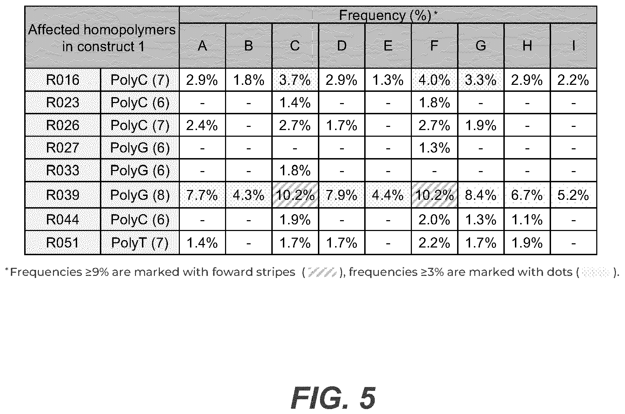

**Figure 5** shows that reducing dNTPs concentration or modifying dNTPs and primer feeding conditions can reduce the frequency of insertions in homopolymer regions.

### EXAMPLE 5

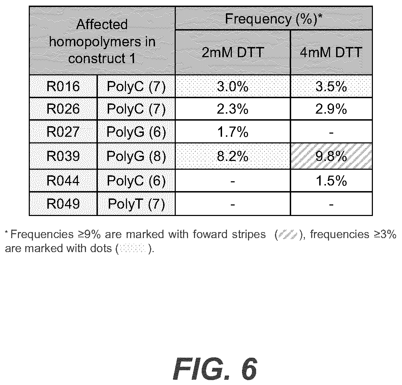

DTT is a reducing agent that in RCA can prevent the oxidation of phi29 polymerase. In order to investigate whether DTT addition could reduce the insertions in homopolymer regions, a set of experiments were performed following the experimental protocol showed in **Table 2.** Results are shown in **Figure 6****.** As seen from **Figure 6****,** lower insertion frequencies were obtained when adding 2mM DTT.

### EXAMPLE 6

In order to investigate whether the combination of lower dNTPs concentration in RCA (1mM), different dNTPs/primer feeding conditions, and DTT addition could prevent the insertions in homopolymer regions, a set of experiments were performed following the experimental protocol showed in **¡Error! No se encuentra el origen de la referencia.** but modifying some factors as specified in **Table 7.**

| **Table 7:** Summary of dNTPs, primer and DTT conditions to evaluate the frequency of insertions | | | |
|---|---|---|---|
| Condition | Primer Addition | dNTPs Addition | DTT conc (mM) |
| A | t=0h (0.014%) | t=0, 7h (50%) | 0 |
| | t=7h (99.99%) | | |
| B | t=0h (0.014%) | t=0, 7h (50%) | 2 |
| | t=7h (99.99%) | | |
| C | t=0,1,2,3,4,5,6,7h (12.5%) | t=0,1,2,3,4,5,6,7h (12.5%) | 0 |
| D | t=0,1,2,3,4,5,6,7h (12.5%) | t=0,1,2,3,4,5,6,7h (12.5%) | 2 |

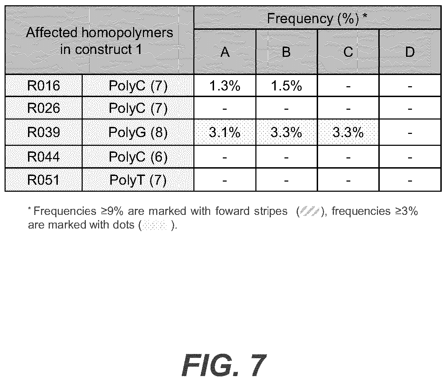

**Figure 7** shows that insertion in homopolymer regions can be prevented by reducing dNTPs and primer concentration during RCA reaction, by adding both reagents in multiple feedings, and including DTT in the reaction.

In order to confirm these results and demonstrate robustness, a new set of experiments was performed following experimental protocol showed in **Table 2** and conditions specified in **Table 8.**

| **Table 8:** . Summary of dNTPs, primer and DTT conditions to confirm insertion frequencies | | | | |
|---|---|---|---|---|
| Condition | Primer Addition | dNTPs Addition | Final dNTPs concentration (mM) | DTT conc (mM) |
| A | t=0h (0.014%) | t=0, 7h (50%) | 4 | 0 |
| | t=7h (99.99%) | | | |
| B | t=0,1,2,3,4,5,6,7h (12.5%) | t=0,1,2,3,4,5,6,7h (12.5%) | 1 | 0 |
| C | t=0,1,2,3,4,5,6,7h (12.5%) | t=0,1,2,3,4,5,6,7h (12.5%) | 1 | 2 |

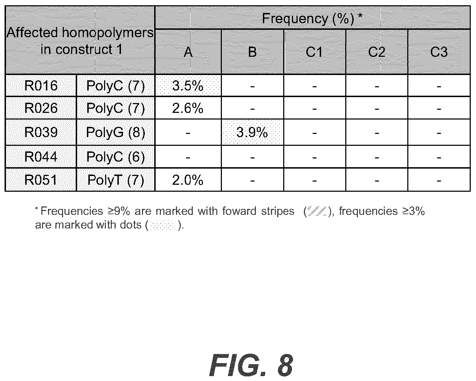

Results in **Figure 8** confirms robustness. Insertions in homopolymer regions can be prevented by reducing dNTPs and primer concentration during the RCA reaction, by adding both reagents in multiple feedings, and including DTT in the reaction.

### EXAMPLE 7

In order to investigate whether the combination of lower dNTPs concentration in RCA (1mM), different dNTPs/primer feeding conditions, and DTT addition could prevent the insertions in homopolymer regions, a set of experiments were performed following the experimental protocol showed in **Table 2** but modifying some factors as specified in **Table 9.**

| **Table 9:** Summary of dNTPs, primer, DTT and reaction time conditions to confirm insertion frequencies | | | | | |
|---|---|---|---|---|---|
| Condition | Primer Addition | dNTPs Addition | Final dNTPs concentration (mM) | DTT conc (mM) | Reaction time (h) |
| A1 | t=0,1,2,3,4,5,6,7h (12.5%) | t=0h (50%) | 1 | 0 | 8 |
| | | t=4, 8h (25%) | | | |
| A2 | t=0,1,2,3,4,5,6,7h (12.5%) | t=0h (50%) | 1 | 0 | 28 |
| | | t=4, 8h (25%) | | | |
| B1 | t=0,1,2,3,4,5,6,7h (12.5%) | t=0h (50%) | 1 | 2 | 8 |
| | | t=4, 8h (25%) | | | |
| B2 | t=0,1,2,3,4,5,6,7h (12.5%) | t=0h (50%) | 1 | 2 | 28 |
| | | t=4, 8h (25%) | | | |
| C1 | t=0,1,2,3,4,5,6,7h (12.5%) | t=0h (32%) | 0.77 | 0 | 10 |
| | | t=2.5h, 5h, 7.5,10h (17%) | | | |
| C2 | t=0,1,2,3,4,5,6,7h (12.5%) | t=0h (32%) | 0.77 | 0 | 28 |
| | | t=2.5h, 5h, 7.5,10h (17%) | | | |
| D1 | t=0,1,2,3,4,5,6,7h (12.5%) | t=0h (32%) | 0.77 | 2 | 10 |
| | | t=2.5h, 5h, 7.5,10h (17%) | | | |
| D2 | t=0,1,2,3,4,5,6,7h (12.5%) | t=0h (32%) | 0.77 | 2 | 28 |
| | | t=2.5h, 5h, 7.5,10h (17%) | | | |
| E1 | t=0,1,2,3,4,5,6,7h (12.5%) | t=0h (26%) | 0.5 | 0 | 10 |
| | | t=2, 4, 6, 8, 10h (14%) | | | |
| E2 | t=0,1,2,3,4,5,6,7h (12.5%) | t=0h (26%) | 0.5 | 0 | 28 |
| | | t=2, 4, 6, 8, 10h (14%) | | | |
| F1 | t=0,1,2,3,4,5,6,7h (12.5%) | t=0h (26%) | 0.5 | 2 | 10 |
| | | t=2, 4, 6, 8, 10h (14%) | | | |
| F2 | t=0,1,2,3,4,5,6,7h (12.5%) | t=0h (26%) | 0.5 | 2 | 28 |
| | | t=2, 4, 6, 8, 10h (14%) | | | |

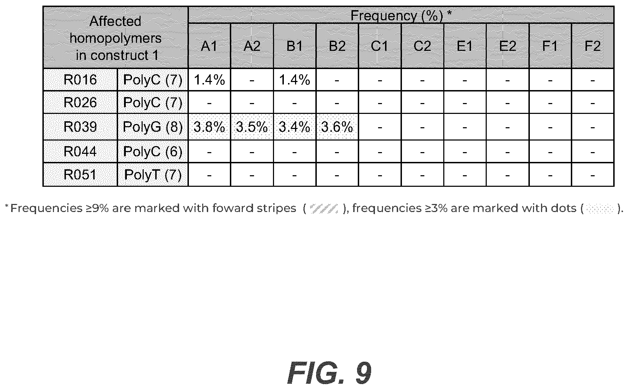

**Figure 9** confirms that insertions in homopolymer regions can be prevented by reducing dNTPs and primer concentration during the RCA reaction, by adding both reagents in multiple feedings.

### EXAMPLE 8

In order to confirm whether the combination of lower dNTPs concentration in RCA (1mM), different dNTPs/primer feeding conditions, and DTT addition prevents the insertions in homopolymer regions, independently of the construct used as plasmid template, a set of experiments were performed following the experimental protocol showed in **Table 2** and conditions specified in **Table 10.**

| **Table 10:** Summary of template, dNTPs, primer and DTT conditions to evaluate the frequency of insertions | | | | | |
|---|---|---|---|---|---|
| Condition | Template | Primer Addition | dNTPs Addition | Final dNTPs concentration (mM) | Final DTT concentration (mM) |
| A | Construct 1 | t=0h (0.014%) | t=0, 7h (50%) | 4 | 0 |
| | | t=7h (99.99%) | | | |
| B | Construct 4 | t=0h (0.014%) | t=0, 7h (50%) | 4 | 0 |
| | | t=7h (99.99%) | | | |
| C | Construct 3 | t=0h (0.014%) | t=0, 7h (50%) | 4 | 0 |
| | | t=7h (99.99%) | | | |
| D | Construct 1 | t=0,1,2,3,4,5,6,7h (12.5%) | t=0,1,2,3,4,5,6,7h (12.5%) | 1 | 2 |
| E | Construct 4 | t=0,1,2,3,4,5,6,7h (12.5%) | t=0,1,2,3,4,5,6,7h (12.5%) | 1 | 2 |
| F | Construct 3 | t=0,1,2,3,4,5,6,7h (12.5%) | t=0,1,2,3,4,5,6,7h (12.5%) | 1 | 2 |

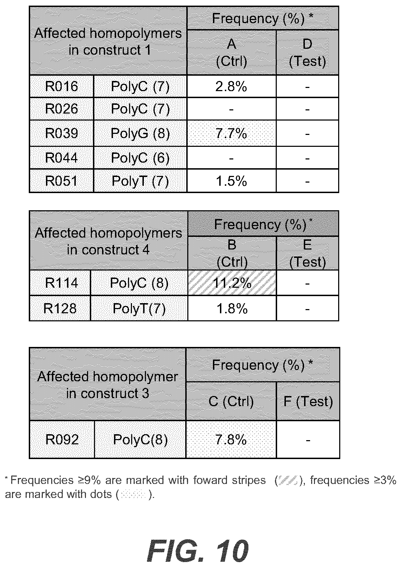

Results from the three different constructs in **Figure 10** confirm that insertions in homopolymer regions can be prevented by reducing dNTPs and primer concentration during the RCA reaction, by adding both reagents in multiple feedings, and including DTT in the reaction.

### EXAMPLE 9

In order to determine if the conditions that prevent insertions in homopolymer regions are scalable, a set of experiments were performed using two different constructs at 1mL scale and 20mL scale. Reactions were performed following the experimental protocol showed in **Tables and 3.** Conditions to prevent insertions are specified in **Table 11.**

| **Table 11:** Summary of conditions preventing insertions, in different constructs and scales | | | | | | | |
|---|---|---|---|---|---|---|---|
| Conditi on | Templ ate | RCA scale | Template concentra tion (µg/mL) | Primer Addition | dNTPs Addition | Final dNTPs concentrati on (mM) | Final DTT concentrati on (mM) |
| A | Constr uct 1 | 1mL | 2 | t=0h (0.014%) | t=0, 7h (50%) | 4 | 0 |
| | | | | t=7h (99.99%) | | | |
| B | Constr uct 1 | 1mL | 2 | t=0,1,2,3,4,5 ,6,7h (12.5%) | t=0,1,2,3,4,5, 6,7h (12.5%) | 1 | 2 |
| C | Constr uct 1 | 1mL | 4 | t=0,1,2,3,4,5 ,6,7h (12.5%) | t=0,1,2,3,4,5, 6,7h (12.5%) | 1 | 2 |
| D | Constr uct 1 | 20m L | 4 | t=0,1,2,3,4,5 ,6,7h (12.5%) | t=0,1,2,3,4,5, 6,7h (12.5%) | 1 | 2 |
| E | Constr uct 2 | 1mL | 2 | t=0h (0.014%) | t=0, 7h (50%) | 4 | 0 |
| | | | | t=7h (99.99%) | | | |
| F | Constr uct 2 | 1mL | 4 | t=0,1,2,3,4,5 ,6,7h (12.5%) | t=0,1,2,3,4,5, 6,7h (12.5%) | 1 | 2 |
| G | Constr uct 2 | 20m L | 4 | t=0,1,2,3,4,5 ,6,7h (12.5%) | t=0,1,2,3,4,5, 6,7h (12.5%) | 1 | 2 |

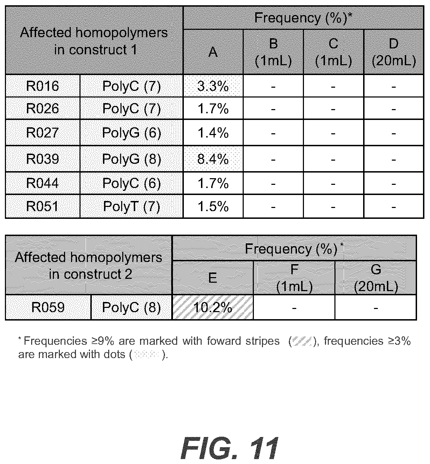

Results from different constructs in **Figure 11** confirm that insertions in homopolymer regions can be prevented by reducing dNTPs and primer concentration during the RCA reaction, by adding both reagents in multiple feedings, and including DTT in the reaction. The same result is observed at both 1mL scale and 20mL scale, demonstrating that these conditions are scalable.

All patents and other publications; including literature references, issued patents, published patent applications, and co-pending patent applications; cited throughout this application are expressly incorporated herein by reference for the purpose of describing and disclosing, for example, the methodologies described in such publications that might be used in connection with the technology described herein. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

### Clauses

1. A method for synthesizing DNA, the method comprising amplification of a DNA template, wherein the DNA template is contacted with a DNA polymerase in the presence of a sufficient portion of a total amount of nucleotides to be added to the reaction, and a sufficient portion of a total amount of one or more primers to be added to form an initial amplification reaction, and wherein at least one subsequent sufficient portion of the total primers and at least one subsequent sufficient portions of the total nucleotides are added to the initial amplification reaction continuously or at regular or irregular intervals at least about 30 minutes after start of the amplification reaction, wherein the sufficient portion is an amount sufficient to carry out the amplification reaction.
2. The method of clause 1, wherein the subsequent portion of the primers is added at the same time as the subsequent portion of the nucleotides.
3. The method of clause 1, wherein the subsequent portion of the primers is added at different times than the subsequent portion of the nucleotides.
4. The method of any one of clauses 1-3, wherein a plurality of subsequent portions of the primers are added to the amplification reaction.
5. The method of any one of clauses 1-4, wherein a plurality of subsequent portions of the nucleotides are added to the amplification reaction.
6. The method of any one of clauses 1-5, wherein the DNA template comprises at least one repeat region.
7. The method of any one of clauses 1-6, wherein the amplification is rolling circle amplification (RCA).
8. The method of clause 6 or 7, wherein the repeat region is at least 5 (e.g., 6, 7, 8, 9, 10 or more) nucleotides in length.
9. The method of any one of clauses 6-8, wherein the repeat region comprises nucleotides that are the same.
10. The method of any one of clauses 6-9, wherein the repeat region comprises a dinucleotide repeat.
11. The method of any one of clauses 6-9, wherein the repeat region comprises a trinucleotide repeat.
12. The method of any one of clauses 1-11, wherein the DNA polymerase is Phi29 polymerase, Bst Polymerase, or a mutant or variant thereof, preferably the DNA polymerase is Phi29.
13. The method of any one of clauses 1-12, wherein the amplification is RCA, the DNA polymerase is Phi29, a plurality of subsequent portions of primers and a plurality of subsequent portions nucleotides are added to the amplification reaction continuously starting at time zero or at regular or irregular intervals at least about 45 minutes after start of the amplification reaction, and the DNA template comprises at least one repeat region of at least 5 nucleotides.
14. The method of any one of clauses 1-13, wherein less than about 5% (e.g., about 4.5%, about 4%, about 3.5%, about 3%, about 2.5%, about 2%, about 1.5%, about 1%, about 0.5%, about 0.4%, about 0.3%, about 0.2%, about 0.1% or less e.g., substantially none) of the amplified product comprises a nucleotide insertion in the sequence of the DNA template.
15. The method of any one of clauses 1-14, wherein the subsequent portions of the primers are added to the amplification reaction at regular or irregular intervals.
16. The method of any one of clauses 1-15, wherein the subsequent portions of the primers are added to the amplification reaction continuously or at intervals of at least about 30 minutes.
17. The method of any one of clauses 1-16, wherein the subsequent portions of the primers are added to the amplification reaction at intervals of from about 45 minutes to about 75 minutes, optionally, the subsequent portions of the primers are added to the amplification reaction at intervals of about 60 minutes).
18. The method of any one of clauses 1-17, wherein at least 2 (e.g., 3, 4, 5, 6, 7, 8, 9, 10, 11, 12, 13, 14, 15, 16, 17, 18, 19, 20, 21, 22, 23, 24, 25, 26, 27, 28, 29, 30 or more) subsequent portions of the primers are added to the amplification reaction, optionally, 7 subsequent portions of the primers are added are added to the initial amplification reaction.
19. The method of any one of clauses 1-18, wherein each subsequent portion of the primers independently comprises an amount that is equal or less than the amount of the primers in the initial amplification reaction.
20. The method of any one of clauses 1-19, wherein each subsequent portion of the primers independently comprises equal or less than about 100% (weight or mol) (e.g., about 100%, about 95%, about 90%, about 80%, about 85%, about 72%, about 70%, about 65%, about 60%, about 55%, about 50%, about 45%, about 40%, about 35%, about 30%, about 25%, about 20%, about 15%, about 10%, about 5% or lower) of the total amount of the primers in the initial amplification reaction. The method of any one of clauses 1-20, wherein each subsequent portion of the primers independently comprises from about 5% to about 20% (weight or mol) (e.g., from about 7.5% to about 30%, or from about 10% to about 25%, or from about 10% to about 15%, optionally about 12.5%) of the total amount of the primers added to the initial amplification reaction.
21. The method of any one of clauses 1-21, wherein each subsequent portion of the primers is independently at a concentration of from about 1 µM to about 10 µM, e.g., about 1.0 µM, about 1.5 µM, about 2.0 µM, about 2.5 µM, about 3.0 µM, about 3.5 µM, about 4.0 µM, about 5.0 µM, about 5.5 µM, about 6 µM, about 6.5 µM, about 7 µM, about 7.5 µM, about 8 µM, about 8.5 µM, about 9 µM, or about 9.5 µM, optionally each subsequent portion of the primers is about 7 µM.
22. The method of any one of clauses 1-22, wherein the initial amplification reaction comprises less than about 50% (weight or mol) (e.g., 49%, about 48%, about 47%, about 46%, about 45%, about 44%, about 43%, about 42%, about 41%, about 40%, 39%, about 38%, about 37%, about 36%, about 35%, about 34%, about 33%, about 32%, about 31%, about 30%, 29%, about 28%, about 27%, about 26%, about 25%, about 24%, about 23%, about 22%, about 21%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2% or less) of the total amount of the primers to be added to the initial amplification reaction, optionally, the initial amplification reaction comprises about 12.5% of the total amount of the primers to be added to the initial amplification reaction.
23. The method any one of clauses 1-23, wherein a total concentration of the primers added to the amplification reaction is from about 5 µM to about 70 µM, e.g., about 5 µM, about 6 µM, about 7 µM, about 8 µM, about 9 µM, about 10 µM, about 15 µM, about 20 µM, about 25 µM, about 30 µM, about 35 µM, about 40 µM, about 45 µM, about 50 µM, about 55 µM, about 60 µM, about 65 µM, or about 70 µM, optionally, the a total concentration of the primers added to the amplification reaction is about 56.4 µM.
24. The method of any one of clauses 1-24, wherein the subsequent portions of the nucleotides are added to the amplification reaction at regular or irregular intervals.
25. The method of any one of clauses 1-25, wherein the subsequent portions of the nucleotides are added to the amplification reaction continuously or at intervals of at least about 30 minutes.
26. The method of any one of clauses 1-26, wherein the subsequent portions of the nucleotides are added to the amplification reaction continuously or at intervals of from about 1 hours to about 3 hours, optionally, wherein the subsequent portions of the nucleotides are added to the amplification reaction at intervals of from about 1 hours to about 2 hours).
27. The method of any one of clauses 1-27, wherein at least 2 (e.g., 3, 4, 5, 6, 7, 8, 9, 10, 11, 12, 13, 14, 15, 16, 17, 18, 19, 20, 21, 22, 23, 24, 25, 26, 27, 28, 29, 30 or more) subsequent portions of the nucleotides are added to the amplification reaction, optionally 7 subsequent portions of the nucleotides are added to the amplification reaction.
28. The method of any one of clauses 1-28, wherein each subsequent portion of the nucleotides independently comprises an amount that is equal or less than the amount of the nucleotides in the initial amplification reaction.
29. The method of any one of clauses 1-29, wherein each subsequent portion of the nucleotides independently comprises less than about 75% (weight or mol) (e.g., about 70%, about 65%, about 60%, about 55%, about 50%, about 45%, about 40%, about 35%, about 30%, about 25%, or lower) of the total amount of the nucleotides in the initial amplification reaction.
30. The method of any one of clauses 1-31, wherein each subsequent portion of the nucleotides independently comprises from about 3% to about 30% (weight or mol) (e.g., from about 10% to about 25% or from about 15% to about 20%, e.g., about 10%, about 11%, about 12%, about 13%, about 14%, about 15%, about 16%, about 17%, about 18%, about 19%, about 20%, about 21%, about 22%, about 23%, about 24%, or about 25% (mol% or weight%) of the total amount of the nucleotides added to the initial amplification reaction.
31. The method of any one of clauses 1-32, wherein each subsequent portion of the nucleotides is independently at a concentration of from about 0.001 mM to about 0.3 mM (e.g., from about 0.005 mM to about 0.125 mM, from about 0.01 mM to 0.1 mM, or from about 0.05 to about 0.09 mM (e.g., about 0.01 mM, about 0.02 mM, about 0.03 mM, about 0.04 mM, about 0.05 mM, about 0.06 mM, about 0.07 mM, about 0.08 mM, about 0.09 mM, about 0.1 mM, about 0.11 mM, about 0.12 mM, about 0.13 mM, about 0.14 mM, about 0.15 mM, about 0.16 mM, about 0.17 mM, about 0.18 mM, about 0.19 mM, about 0.20 mM, about 0.21 mM, about 0.22 mM, about 0.23 mM, about 0.24 mM, about 0.25 mM, about 0.26 mM, about 0.27 mM, about 0.28 mM, about 0.29 mM, or about 0.3 mM), optionally about 0.13 mM or about 0.25 mM.
32. The method of any one of clauses 1-32, wherein the initial amplification reaction comprises less than about 30% (mol%) (e.g., about 29%, about 28%, about 27%, about 26%, about 25%, about 24%, about 23%, about 22%, about 21%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1% or less) of the total amount of the nucleotides added to the initial amplification reaction.
33. The method any one of clauses 1-33, wherein a total concentration of the nucleotides added to the amplification reaction is from about 0.1 mM to about 6 mM, e.g., from about 0.25 mM to about 5.5 mM, from about 0.3 mM to about 5 mM or from about 0.25 mM to about 4.5 mM, e.g., about 0.1 mM, about 0.15 mM, about 0.2 mM, about 0.25 mM, about 0.3 mM, about 0.35 mM, about 0.4 mM, about 0.45 mM, about 0.5 mM, about 0.55 mM, about 0.6 mM, about 0.65 mM, about 0.7 mM, about 0.75 mM, about 0.8 mM, about 0.85 mM, about 0.9 mM, about 0.95 mM, about 1 mM, about 1.25 mM, about 1.5 mM, about 1.75 mM, or about 1.25 mM, or about 3 mM, , or about 3.25 mM, or about 3.5 mM, or about 3.75 mM, or about 4.25 mM, or about 4.5 mM, or about 4.75 mM, or about 5 mM, optionally about 0.5 mM, or about 0.77 mM, or about 4 mM, preferably about 4 mM.
34. The method of any one of clauses 1-34, wherein each subsequent portion of the nucleotides is added after at least of about 40% (e.g., about 45%, about 50%, about 55%, about 60%, about 65%, about 70%, about 75%, about 80%, about 85%, or more) of the nucleotides in the amplification reaction have been used.
35. The method of any one of clauses 1-35, wherein a molar ratio of A to G, A to C, T to G, or T to C in the nucleotides added to the reaction mixture is higher than 1 (e.g., about 1.1, about 1.2, about 1.3, about 1.4, about 1.5, about 1.6, about 1.7, about 1.8, about 1.9, about 2, about 2.1, about 2.2, about 2.3,
36. The method of any one of clauses 6-36, wherein the repeat region is at least 6 nucleotides in length.
37. The method of any one of clauses 6-37, wherein the repeat region is at least 7 nucleotides in length.
38. The method of any one of clauses 6-38, wherein the repeat region is at least 8 nucleotides in length.
39. The method of any one of clauses 6-39, wherein the repeat region is at least 9 nucleotides in length.
40. The method of any one of clauses 6-40, wherein the repeat region is at least 10 nucleotides in length.
41. The method of any one of clauses 6-41, wherein the repeat region is at least 11 nucleotides in length.
42. The method of any one of clauses 6-42, wherein the repeat region is at least 12 nucleotides in length.
43. The method of any one of clauses 6-43, wherein the repeat region is at least 13 nucleotides in length.
44. The method of any one of clauses 6-44, wherein the repeat region comprises C.
45. The method of any one of clauses 6-44, wherein the repeat region comprises G.
46. The method of any one of clauses 6-44, wherein the repeat region comprises A.
47. The method of any one of clauses 6-44, wherein the repeat region comprises T.
48. The method of any one of clauses 6-44, wherein the repeat region comprises GC or CG dinucleotide.
49. The method of any one of clauses 1-49, wherein the DNA template is double-stranded DNA (dsDNA).
50. The method of any one of clauses 1-50, wherein the DNA template is an open circular dsDNA, a closed circular dsDNA, an open linear dsDNA or a closed linear dsDNA
51. The method of any one of clauses 1-51, wherein the DNA template is single-stranded DNA (ssDNA).
52. The method of clause 52, wherein the DNA template is a linear ssDNA or a closed circular ssDNA.
53. The method of any one of clauses 1-53, wherein the DNA template is from about 500 bases to about 100 kilobases, e.g., from about 1 kilobases to about 50 kilobases, from about 1.5 kilobases to about 25 kilobases, or from about 2 kilobases to about 20 kilobases.
54. The method of any one of clauses 1-54, wherein the DNA template comprises at least one processing enzyme target sequence.
55. The method of clause 55, wherein said at least one processing enzyme target sequence comprises a recombinase targeting sequence or a protelomerase targeting sequence.
56. The method of clause 56, wherein the method further comprises supplying a processing enzyme to the reaction mixture.
57. The method of clause 57, wherein the processing enzyme is supplied at regular or irregular intervals during the amplification reaction.
58. The method of any one of clauses 1-6 or 8-58, wherein the amplification is isothermal amplification.
59. The method of clause 59, wherein the amplification is Rolling Circle Amplification (RCA), Loop Mediated Isothermal Amplification (LAMP), Recombinase Polymerase Amplification (RPA), Helicase-dependent isothermal DNA amplification (HDA), Nucleic acid sequence-based amplification (NASBA), strand displacement amplification (SDA), nicking enzyme amplification reaction (NEAR), polymerase Spiral Reaction (PSR), Hybridization Chain Reaction (HCR), Primer Exchange Reaction (PER), Signal Amplification by Exchange Reaction (SABER), transcription-based amplification system (TAS), Self-sustained sequence replication reaction (3SR), Single primer isothermal amplification (SPIA), or cross-priming amplification (CPA).
60. The method of any one of clauses 1-60, wherein the amplification reaction further comprises a DNA polymerase stabilizing agent.
61. The method of clause 61, wherein the DNA polymerase stabilizing agent is a reducing agent.
62. The method of any one of clauses 1-62, wherein the amplification reaction comprises a reducing agent, optionally the reducing agent is dithiothreitol (DTT), 1,2-propanediol, β-mercaptoethanol, cysteine, dithioerythritol, glutathione., and acetyl cysteine, preferably the reducing agent is DTT.
63. The method of any one of clauses 1-63, wherein the amplification reaction further comprises DTT.
64. The method of any one of clauses 1-64, wherein the amplification reaction comprises DTT at a concentration from about 1 mM to about 3 mM, e.g., from about 1.5 mM to 2.5 mM or from about 1.75 mM to about 2.25 mM, e.g., about 1.5 mM, about 1.55 mM, about 1.6 mM, about 1.65 mM, about 1.7 mM, about 1.75 mM, about 1.8 mM, about 1.85 mM, about 1.9 mM, about 1.95 mM, about 2 mM, about 2.05 mM, about 2.1 mM, about 2.15 mM, about 2.2 mM, about 2.25 mM, about 2.3 mM, about 2.35 mM, about 2.4 mM, about 2.45 mM, or about 2.5 mM, preferably about 2 mM.
65. The method of any one of clauses 1-65, wherein the amplification product is no end DNA (neDNA), closed-ended linear duplex DNA (ceDNA), close ended linear duplexed DNA (clDNA), or linear-covalently closed DNA.
66. The method of any one of clauses 1-66, wherein the amplification product is neDNA.
67. The method of any one of clauses 1-67, wherein the method is a cell-free method.

## Claims

1. A method for synthesizing DNA, the method comprising amplification of a DNA template, wherein the DNA template is contacted with a DNA polymerase in the presence of a sufficient portion of a total amount of nucleotides to be added to the reaction, and a sufficient portion of a total amount of one or more primers to be added to form an initial amplification reaction, and wherein at least one subsequent sufficient portion of the total primers and at least one subsequent sufficient portions of the total nucleotides are added to the initial amplification reaction continuously or at regular or irregular intervals at least about 30 minutes after start of the amplification reaction, wherein the sufficient portion is an amount sufficient to carry out the amplification reaction.

2. The method of claim 1, wherein the subsequent portion of the primers is added at the same time as the subsequent portion of the nucleotides, or
wherein the subsequent portion of the primers is added at different times than the subsequent portion of the nucleotides.

3. The method of claims 1 or 2, wherein a plurality of subsequent portions of the primers are added to the amplification reaction, and/or
wherein a plurality of subsequent portions of the nucleotides are added to the amplification reaction.

4. The method of any one of claims 1-3, wherein the DNA template comprises at least one repeat region.

5. The method of any one of claims 1-4, wherein the amplification is rolling circle amplification (RCA).

6. The method of claim 4 or 5, wherein the repeat region is at least 5 (e.g., 6, 7, 8, 9, 10 or more) nucleotides in length.

7. The method of any one of claims 4-6, wherein the repeat region comprises nucleotides that are the same, and/or
wherein the repeat region comprises a dinucleotide repeat, or
wherein the repeat region comprises a trinucleotide repeat.

8. The method of any one of claims 1-7, wherein the DNA polymerase is Phi29 polymerase, Bst Polymerase, or a mutant or variant thereof, preferably the DNA polymerase is Phi29, and/or
wherein the amplification is RCA, the DNA polymerase is Phi29, a plurality of subsequent portions of primers and a plurality of subsequent portions nucleotides are added to the amplification reaction continuously starting at time zero or at regular or irregular intervals at least about 45 minutes after start of the amplification reaction, and the DNA template comprises at least one repeat region of at least 5 nucleotides, and/or
wherein less than about 5% (e.g., about 4.5%, about 4%, about 3.5%, about 3%, about 2.5%, about 2%, about 1.5%, about 1%, about 0.5%, about 0.4%, about 0.3%, about 0.2%, about 0.1% or less e.g., substantially none) of the amplified product comprises a nucleotide insertion in the sequence of the DNA template, and/or
wherein the subsequent portions of the primers are added to the amplification reaction at regular or irregular intervals, and/or
wherein the subsequent portions of the primers are added to the amplification reaction continuously or at intervals of at least about 30 minutes, and/or
wherein the subsequent portions of the primers are added to the amplification reaction at intervals of from about 45 minutes to about 75 minutes, optionally, the subsequent portions of the primers are added to the amplification reaction at intervals of about 60 minutes), and/or
wherein at least 2 (e.g., 3, 4, 5, 6, 7, 8, 9, 10, 11, 12, 13, 14, 15, 16, 17, 18, 19, 20, 21, 22, 23, 24, 25, 26, 27, 28, 29, 30 or more) subsequent portions of the primers are added to the amplification reaction, optionally, 7 subsequent portions of the primers are added are added to the initial amplification reaction, and/or
wherein each subsequent portion of the primers independently comprises an amount that is equal or less than the amount of the primers in the initial amplification reaction, and/or
wherein each subsequent portion of the primers independently comprises equal or less than about 100% (weight or mol) (e.g., about 100%, about 95%, about 90%, about 80%, about 85%, about 72%, about 70%, about 65%, about 60%, about 55%, about 50%, about 45%, about 40%, about 35%, about 30%, about 25%, about 20%, about 15%, about 10%, about 5% or lower) of the total amount of the primers in the initial amplification reaction. The method of any one of claims 1-20, wherein each subsequent portion of the primers independently comprises from about 5% to about 20% (weight or mol) (e.g., from about 7.5% to about 30%, or from about 10% to about 25%, or from about 10% to about 15%, optionally about 12.5%) of the total amount of the primers added to the initial amplification reaction, and/or
wherein each subsequent portion of the primers is independently at a concentration of from about 1 µM to about 10 µM, e.g., about 1.0 µM, about 1.5 µM, about 2.0 µM, about 2.5 µM, about 3.0 µM, about 3.5 µM, about 4.0 µM, about 5.0 µM, about 5.5 µM, about 6 µM, about 6.5 µM, about 7 µM, about 7.5 µM, about 8 µM, about 8.5 µM, about 9 µM, or about 9.5 µM, optionally each subsequent portion of the primers is about 7 µM, and/or
wherein the initial amplification reaction comprises less than about 50% (weight or mol) (e.g., 49%, about 48%, about 47%, about 46%, about 45%, about 44%, about 43%, about 42%, about 41%, about 40%, 39%, about 38%, about 37%, about 36%, about 35%, about 34%, about 33%, about 32%, about 31%, about 30%, 29%, about 28%, about 27%, about 26%, about 25%, about 24%, about 23%, about 22%, about 21%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2% or less) of the total amount of the primers to be added to the initial amplification reaction, optionally, the initial amplification reaction comprises about 12.5% of the total amount of the primers to be added to the initial amplification reaction, and/or
wherein a total concentration of the primers added to the amplification reaction is from about 5 µM to about 70 µM, e.g., about 5 µM, about 6 µM, about 7 µM, about 8 µM, about 9 µM, about 10 µM, about 15 µM, about 20 µM, about 25 µM, about 30 µM, about 35 µM, about 40 µM, about 45 µM, about 50 µM, about 55 µM, about 60 µM, about 65 µM, or about 70 µM, optionally, the a total concentration of the primers added to the amplification reaction is about 56.4 µM, and/or
wherein the subsequent portions of the nucleotides are added to the amplification reaction at regular or irregular intervals, and/or
wherein the subsequent portions of the nucleotides are added to the amplification reaction continuously or at intervals of at least about 30 minutes, and/or
wherein the subsequent portions of the nucleotides are added to the amplification reaction continuously or at intervals of from about 1 hours to about 3 hours, optionally, wherein the subsequent portions of the nucleotides are added to the amplification reaction at intervals of from about 1 hours to about 2 hours), and/or
wherein at least 2 (e.g., 3, 4, 5, 6, 7, 8, 9, 10, 11, 12, 13, 14, 15, 16, 17, 18, 19, 20, 21, 22, 23, 24, 25, 26, 27, 28, 29, 30 or more) subsequent portions of the nucleotides are added to the amplification reaction, optionally 7 subsequent portions of the nucleotides are added to the amplification reaction, and/or
wherein each subsequent portion of the nucleotides independently comprises an amount that is equal or less than the amount of the nucleotides in the initial amplification reaction, and/or
wherein each subsequent portion of the nucleotides independently comprises less than about 75% (weight or mol) (e.g., about 70%, about 65%, about 60%, about 55%, about 50%, about 45%, about 40%, about 35%, about 30%, about 25%, or lower) of the total amount of the nucleotides in the initial amplification reaction, and/or
wherein each subsequent portion of the nucleotides independently comprises from about 3% to about 30% (weight or mol) (e.g., from about 10% to about 25% or from about 15% to about 20%, e.g., about 10%, about 11%, about 12%, about 13%, about 14%, about 15%, about 16%, about 17%, about 18%, about 19%, about 20%, about 21%, about 22%, about 23%, about 24%, or about 25% (mol% or weight%) of the total amount of the nucleotides added to the initial amplification reaction, and/or
wherein each subsequent portion of the nucleotides is independently at a concentration of from about 0.001 mM to about 0.3 mM (e.g., from about 0.005 mM to about 0.125 mM, from about 0.01 mM to 0.1 mM, or from about 0.05 to about 0.09 mM (e.g., about 0.01 mM, about 0.02 mM, about 0.03 mM, about 0.04 mM, about 0.05 mM, about 0.06 mM, about 0.07 mM, about 0.08 mM, about 0.09 mM, about 0.1 mM, about 0.11 mM, about 0.12 mM, about 0.13 mM, about 0.14 mM, about 0.15 mM, about 0.16 mM, about 0.17 mM, about 0.18 mM, about 0.19 mM, about 0.20 mM, about 0.21 mM, about 0.22 mM, about 0.23 mM, about 0.24 mM, about 0.25 mM, about 0.26 mM, about 0.27 mM, about 0.28 mM, about 0.29 mM, or about 0.3 mM), optionally about 0.13 mM or about 0.25 mM, and/or
wherein the initial amplification reaction comprises less than about 30% (mol%) (e.g., about 29%, about 28%, about 27%, about 26%, about 25%, about 24%, about 23%, about 22%, about 21%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1% or less) of the total amount of the nucleotides added to the initial amplification reaction and/or
wherein a total concentration of the nucleotides added to the amplification reaction is from about 0.1 mM to about 6 mM, e.g., from about 0.25 mM to about 5.5 mM, from about 0.3 mM to about 5 mM or from about 0.25 mM to about 4.5 mM, e.g., about 0.1 mM, about 0.15 mM, about 0.2 mM, about 0.25 mM, about 0.3 mM, about 0.35 mM, about 0.4 mM, about 0.45 mM, about 0.5 mM, about 0.55 mM, about 0.6 mM, about 0.65 mM, about 0.7 mM, about 0.75 mM, about 0.8 mM, about 0.85 mM, about 0.9 mM, about 0.95 mM, about 1 mM, about 1.25 mM, about 1.5 mM, about 1.75 mM, or about 1.25 mM, or about 3 mM, , or about 3.25 mM, or about 3.5 mM, or about 3.75 mM, or about 4.25 mM, or about 4.5 mM, or about 4.75 mM, or about 5 mM, optionally about 0.5 mM, or about 0.77 mM, or about 4 mM, preferably about 4 mM, and/or
wherein each subsequent portion of the nucleotides is added after at least of about 40% (e.g., about 45%, about 50%, about 55%, about 60%, about 65%, about 70%, about 75%, about 80%, about 85%, or more) of the nucleotides in the amplification reaction have been used.

9. The method of any one of claims 1-8, wherein a molar ratio of A to G, A to C, T to G, or T to C in the nucleotides added to the reaction mixture is higher than 1 (e.g., about 1.1, about 1.2, about 1.3, about 1.4, about 1.5, about 1.6, about 1.7, about 1.8, about 1.9, about 2, about 2.1, about 2.2, about 2.3,

10. The method of any one of claims 4-9, wherein the repeat region is at least 6, at least 7, at least 8, at least 9, at least 10, at least 11, at least 12, and/or at least 13 nucleotides in length.

11. The method of any one of claims 4-10, wherein the repeat region comprises C, or G or A or T or GC or CG dinucleotide.

12. The method of any one of claims 1-11, wherein the DNA template is double-stranded DNA (dsDNA), and/or
wherein the DNA template is an open circular dsDNA, a closed circular dsDNA, an open linear dsDNA or a closed linear dsDNA, and/or
wherein the DNA template is single-stranded DNA (ssDNA), optionally wherein the DNA template is a linear ssDNA or a closed circular ssDNA.

13. The method of any one of claims 1-12, wherein the DNA template is from about 500 bases to about 100 kilobases, e.g., from about 1 kilobases to about 50 kilobases, from about 1.5 kilobases to about 25 kilobases, or from about 2 kilobases to about 20 kilobases, and/or
wherein the DNA template comprises at least one processing enzyme target sequence, optionally
wherein said at least one processing enzyme target sequence comprises a recombinase targeting sequence or a protelomerase targeting sequence, optionally
wherein the method further comprises supplying a processing enzyme to the reaction mixture, optionally
wherein the processing enzyme is supplied at regular or irregular intervals during the amplification reaction.

14. The method of any one of claims 1-4 or 6-13, wherein the amplification is isothermal amplification, optionally
wherein the amplification is Rolling Circle Amplification (RCA), Loop Mediated Isothermal Amplification (LAMP), Recombinase Polymerase Amplification (RPA), Helicase-dependent isothermal DNA amplification (HDA), Nucleic acid sequence-based amplification (NASBA), strand displacement amplification (SDA), nicking enzyme amplification reaction (NEAR), polymerase Spiral Reaction (PSR), Hybridization Chain Reaction (HCR), Primer Exchange Reaction (PER), Signal Amplification by Exchange Reaction (SABER), transcription-based amplification system (TAS), Self-sustained sequence replication reaction (3SR), Single primer isothermal amplification (SPIA), or cross-priming amplification (CPA).

15. The method of any one of claims 1-14, wherein the amplification reaction further comprises a DNA polymerase stabilizing agent, optionally
wherein the DNA polymerase stabilizing agent is a reducing agent, and/or wherein the amplification reaction comprises a reducing agent, optionally the reducing agent is dithiothreitol (DTT), 1,2-propanediol, β-mercaptoethanol, cysteine, dithioerythritol, glutathione., and acetyl cysteine, preferably the reducing agent is DTT, and/or wherein the amplification reaction further comprises DTT, and/or
wherein the amplification reaction comprises DTT at a concentration from about 1 mM to about 3 mM, e.g., from about 1.5 mM to 2.5 mM or from about 1.75 mM to about 2.25 mM, e.g., about 1.5 mM, about 1.55 mM, about 1.6 mM, about 1.65 mM, about 1.7 mM, about 1.75 mM, about 1.8 mM, about 1.85 mM, about 1.9 mM, about 1.95 mM, about 2 mM, about 2.05 mM, about 2.1 mM, about 2.15 mM, about 2.2 mM, about 2.25 mM, about 2.3 mM, about 2.35 mM, about 2.4 mM, about 2.45 mM, or about 2.5 mM, preferably about 2 mM, and/or
wherein the amplification product is no end DNA (neDNA), closed-ended linear duplex DNA (ceDNA), close ended linear duplexed DNA (clDNA), or linear-covalently closed DNA, and/or
wherein the amplification product is neDNA, and/or wherein the method is a cell-free method.
